# EUROPÄISCHE PATENTSCHRIFT

(11) **EP 3 401 327 B1**
(45) Veröffentlichungstag und Bekanntmachung des Hinweises auf die Patenterteilung: **13.01.2021**
(21) Anmeldenummer: 18167570.3
(22) Anmeldetag: 18.05.2005
(51) Int. Cl.: C07K 14/47, C07K 16/30, A61K 39/395, A61P 35/00

(54) **DIFFERENTIELL IN TUMOREN EXPRIMIERTE GENPRODUKTE UND DEREN VERWENDUNG**
GENE PRODUCTS DIFFERENTIALLY EXPRESSED IN TUMORS AND THEIR USES
PRODUITS GÉNIQUES D'EXPRESSION DIFFÉRENTIELLE DANS LES TUMEURS ET LEUR UTILISATION

(30) Priorität: 18.05.2004 DE 102004024617
(43) Veröffentlichungstag der Anmeldung: 14.11.2018
(62) Teilanmeldung aus: 11002013.8
(73) Patentinhaber: Astellas Pharma Inc., Chuo-ku Tokyo 103-8411 (JP)
(72) Erfinder: TÜRECI, Özlem, 55116 Mainz (DE); SAHIN, Ugur, 55116 Mainz (DE); KOSLOWSKI, Michael, 65933 Frankfurt (DE); FRITZ, Stefan, 55237 Flonheim (DE); GEPPERT, Harald-Gerhard, 42697 Solingen (DE)
(74) Vertreter: Schnappauf, Georg

(56) Entgegenhaltungen:
- WO-A2-2004/047863
- NIIMI T ET AL: "CLAUDIN-18, A NOVEL DOWSTREAM TARGET GENE FOR THE T/EBP/NKX2.1 HOMEODO", MOLECULAR AND CELLULAR BIOLOGY, AMERICAN SOCIETY FOR MICROBIOLOGY, WASHINGTON, US, Bd. 21, Nr. 21, 1. Januar 2001 (2001-01-01), Seiten 7380-7390, XP002375751, ISSN: 0270-7306, DOI: 10.1128/MCB.21.21.7380-7390.2001
- BRAENDLEIN S ET AL: "PAM-1, A NATURAL HUMAN IGM ANTIBODY AS NEW TOOL FOR DETECTION OF BREAST AND PROSTATE PRECURSORS", HUMAN ANTIBO, IOS PRESS, AMSTERDAM, NL, Bd. 13, Nr. 4, 1. Januar 2004 (2004-01-01) , Seiten 97-104, XP009057297, ISSN: 1093-2607
- SAHIN UGUR ET AL: "Claudin-18 splice variant 2 is a pan-cancer target suitable for therapeutic antibody development", CLINICAL CANCER RESEARCH, THE AMERICAN ASSOCIATION FOR CANCER RESEARCH, US, Bd. 14, Nr. 23, 1. Dezember 2008 (2008-12-01), Seiten 7624-7634, XP002588324, ISSN: 1078-0432, DOI: 10.1158/1078-0432.CCR-08-1547

### SEQUENZPROTOKOLL

<110> Ganymed Pharmaceuticals GmbH
<120> Differentiell in Tumoren exprimierte Genprodukte und deren Verwendung
<130> 342-23 EPT6
<150> DE 10 2004 024 617.3
   <151> 2004-05-18
<160> 150
<170> PatentIn version 3.1
<210> 1
   <211> 1875
   <212> DNA
   <213> Homo sapiens
<400> 1
   caggccagag tcccagctgt cctggactct gctgtgggga agggctgatg caggtgtgga      60
   gtcaaatgtg ggtgcctcct gcagccgggt gccaggaggg gtggaggggc caccctgggc     120
   tttgtccggg agcctggtct tcccgtcctt gggctgacag gtgctgctgc ctctgagccc     180
   tccctgctaa gagctgtgtg ctgggtaagg ctggtggccc tttgggctcc ctgtccagga     240
   tttgtgctct ggagggtagg gcttgctggg ctggggactg gaggggaacg tggagctcct     300
   tctgcctcct ttcctgcccc atgacagcag gcagatccca ggagagaaga gctcaggaga     360
   tgggaagagg atctgtccag gggttagacc tcaagggtga cttggagttc tttacggcac     420
   ccatgctttc tttgaggagt tttgtgtttg tgggtgtggg gtcggggctc acctcctccc     480
   acatccctgc ccagaggtgg gcagagtggg ggcagtgcct tgctccccct gctcgctctc     540
   tgctgacctc cggctccctg tgctgcccca ggaccatgaa tggcacctac aacacctgtg     600
   gctccagcga cctcacctgg cccccagcga tcaagctggg cttctacgcc tacttgggcg     660
   tcctgctggt gctaggcctg ctgctcaaca gcctggcgct ctgggtgttc tgctgccgca     720
   tgcagcagtg gacggagacc cgcatctaca tgaccaacct ggcggtggcc gacctctgcc     780
   tgctgtgcac cttgcccttc gtgctgcact ccctgcgaga cacctcagac acgccgctgt     840
   gccagctctc ccagggcatc tacctgacca acaggtacat gagcatcagc ctggtcacgg     900
   ccatcgccgt ggaccgctat gtggccgtgc ggcacccgct gcgtgcccgc gggctgcggt     960
   cccccaggca ggctgcggcc gtgtgcgcgg tcctctgggt gctggtcatc ggctccctgg    1020
   tggctcgctg gctcctgggg attcaggagg gcggcttctg cttcaggagc acccggcaca    1080
   atttcaactc catggcgttc ccgctgctgg gattctacct gcccctggcc gtggtggtct    1140
   tctgctccct gaaggtggtg actgccctgg cccagaggcc acccaccgac gtggggcagg   1200
   cagaggccac ccgcaaggct gcccgcatgg tctgggccaa cctcctggtg ttcgtggtct   1260
   gcttcctgcc cctgcacgtg gggctgacag tgcgcctcgc agtgggctgg aacgcctgtg   1320
   ccctcctgga gacgatccgt cgcgccctgt acataaccag caagctctca gatgccaact   1380
   gctgcctgga cgccatctgc tactactaca tggccaagga gttccaggag gcgtctgcac   1440
   tggccgtggc tcccagtgct aaggcccaca aaagccagga ctctctgtgc gtgaccctcg   1500
   cctaagaggc gtgctgtggg cgctgtgggc caggtctcgg gggctccggg aggtgctgcc   1560
   tgccagggga agctggaacc agtagcaagg agcccgggat cagccctgaa ctcactgtgt   1620
   attctcttgg agccttgggt gggcagggac ggcccaggta cctgctctct tgggaagaga   1680
   gagggacagg gacaagggca agaggactga ggccagagca aggccaatgt cagagacccc   1740
   cgggatgggg cctcacactt gccaccccca gaaccagctc acctggccag agtgggttcc   1800
   tgctggccag ggtgcagcct tgatgacacc tgccgctgcc cctcggggct ggaataaaac   1860
   tccccaccca gagtc                                                    1875
<210> 2
   <211> 3222
   <212> DNA
   <213> Homo sapiens
<400> 2
   atgaagacgt tgctgttgga cttggctttg tggtcactgc tcttccagcc cgggtggctg     60
   tcctttagtt cccaggtgag tcagaactgc cacaatggca gctatgaaat cagcgtcctg    120
   atgatgggca actcagcctt tgcagagccc ctgaaaaact tggaagatgc ggtgaatgag    180
   gggctggaaa tagtgagagg acgtctgcaa aatgctggcc taaatgtgac tgtgaacgct    240
   actttcatgt attcggatgg tctgattcat aactcaggcg actgccggag tagcacctgt    300
   gaaggcctcg acctactcag gaaaatttca aatgcacaac ggatgggctg tgtcctcata    360
   gggccctcat gtacatactc caccttccag atgtaccttg acacagaatt gagctacccc    420
   atgatctcag ctggaagttt tggattgtca tgtgactata aagaaacctt aaccaggctg    480
   atgtctccag ctagaaagtt gatgtacttc ttggttaact tttggaaaac caacgatctg    540
   cccttcaaaa cttattcctg gagcacttcg tatgtttaca agaatggtac agaaactgag    600
   gactgtttct ggtaccttaa tgctctggag gctagcgttt cctatttctc ccacgaactc    660
   ggctttaagg tggtgttaag acaagataag gagtttcagg atatcttaat ggaccacaac    720
   aggaaaagca atgtgattat tatgtgtggt ggtccagagt tcctctacaa gctgaagggt    780
   gaccgagcag tggctgaaga cattgtcatt attctagtgg atcttttcaa tgaccagtac    840
   ttggaggaca atgtcacagc ccctgactat atgaaaaatg tccttgttct gacgctgtct    900
   cctgggaatt cccttctaaa tagctctttc tccaggaatc tatcaccaac aaaacgagac 960
   tttgctcttg cctatttgaa tggaatcctg ctctttggac atatgctgaa gatatttctt 1020
   gaaaatggag aaaatattac cacccccaaa tttgctcatg ctttcaggaa tctcactttt 1080
   gaagggtatg acggtccagt gaccttggat gactgggggg atgttgacag taccatggtg 1140
   cttctgtata cctctgtgga caccaagaaa tacaaggttc ttttgaccta tgatacccac 1200
   gtaaataaga cctatcctgt ggatatgagc cccacattca cttggaagaa ctctaaactt 1260
   cctaatgata ttacaggccg gggccctcag atcctgatga ttgcagtctt caccctcact 1320
   ggagctgtgg tgctgctcct gctcgtcgct ctcctgatgc tcagaaaata tagaaaagat 1380
   tatgaacttc gtcagaaaaa atggtcccac attcctcctg aaaatatctt tcctctggag 1440
   accaatgaga ccaatcatgt tagcctcaag atcgatgatg acaaaagacg agatacaatc 1500
   cagagactac gacagtgcaa atacgacaaa aagcgagtga ttctcaaaga tctcaagcac 1560
   aatgatggta atttcactga aaaacagaag atagaattga acaagttgct tcagattgac 1620
   tattacaacc tgaccaagtt ctacggcaca gtgaaacttg ataccatgat cttcggggtg 1680
   atagaatact gtgagagagg atccctccgg gaagttttaa atgacacaat ttcctaccct 1740
   gatggcacat tcatggattg ggagtttaag atctctgtct tgtatgacat tgctaaggga 1800
   atgtcatatc tgcactccag taagacagaa gtccatggtc gtctgaaatc taccaactgc 1860
   gtagtggaca gtagaatggt ggtgaagatc actgattttg gctgcaattc cattttacct 1920
   ccaaaaaagg acctgtggac agctccagag cacctccgcc aagccaacat ctctcagaaa 1980
   ggagatgtgt acagctatgg gatcatcgca caggagatca ttctgcggaa agaaaccttc 2040
   tacactttga gctgtcggga ccggaatgag aagattttca gagtggaaaa ttccaatgga 2100
   atgaaaccct tccgcccaga tttattcttg gaaacagcag aggaaaaaga gctagaagtg 2160
   tacctacttg taaaaaactg ttgggaggaa gatccagaaa agagaccaga tttcaaaaaa 2220
   attgagacta cacttgccaa gatatttgga ctttttcatg accaaaaaaa tgaaagctat 2280
   atggatacct tgatccgacg tctacagcta tattctcgaa acctggaaca tctggtagag 2340
   gaaaggacac agctgtacaa ggcagagagg gacagggctg acagacttaa ctttatgttg 2400
   cttccaaggc tagtggtaaa gtctctgaag gagaaaggct ttgtggagcc ggaactatat 2460
   gaggaagtta caatctactt cagtgacatt gtaggtttca ctactatctg caaatacagc 2520
   acccccatgg aagtggtgga catgcttaat gacatctata agagttttga ccacattgtt 2580
   gatcatcatg atgtctacaa ggtggaaacc atcggtgatg cgtacatggt ggctagtggt 2640
   ttgcctaaga gaaatggcaa tcggcatgca atagacattg ccaagatggc cttggaaatc 2700
   ctcagcttca tggggacctt tgagctggag catcttcctg gcctcccaat atggattcgc 2760
   attggagttc actctggtcc ctgtgctgct ggagttgtgg gaatcaagat gcctcgttat 2820
   tgtctatttg gagatacggt caacacagcc tctaggatgg aatccactgg cctccctttg 2880
   agaattcacg tgagtggctc caccatagcc atcctgaaga gaactgagtg ccagttcctt 2940
   tatgaagtga gaggagaaac atacttaaag ggaagaggaa atgagactac ctactggctg 3000
   actgggatga aggaccagaa attcaacctg ccaacccctc ctactgtgga gaatcaacag 3060
   cgtttgcaag cagaattttc agacatgatt gccaactctt tacagaaaag acaggcagca 3120
   gggataagaa gccaaaaacc cagacgggta gccagctata aaaaaggcac tctggaatac 3180
   ttgcagctga ataccacaga caaggagagc acctattttt aa 3222
<210> 3
   <211> 336
   <212> DNA
   <213> Homo sapiens
<400> 3
   atgaagacgt tgctgttgga cttggctttg tggtcactgc tcttccagcc cgggtggctg 60
   tcctttagtt cccaggtgag tcagaactgc cacaatggca gctatgaaat cagcgtcctg 120
   atgatgggca actcagcctt tgcagagccc ctgaaaaact tggaagatgc ggtgaatgag 180
   gggctggaaa tagtgagagg acgtctgcaa aatgctggcc taaatgtgac tgtgaacgct 240
   actttcatgt attcggatgg tctgattcat aactcaggcg actgccggag tagcacctgt 300
   gaaggcctcg acctactcag gaaaatttca ccttga 336
<210> 4
   <211> 777
   <212> DNA
   <213> Homo sapiens
<400> 4
   atgaagacgt tgctgttgga cttggctttg tggtcactgc tcttccagcc cgggtggctg      60
   tcctttagtt cccaggtgag tcagaactgc cacaatggca gctatgaaat cagcgtcctg     120
   atgatgggca actcagcctt tgcagagccc ctgaaaaact tggaagatgc ggtgaatgag     180
   gggctggaaa tagtgagagg acgtctgcaa aatgctggcc taaatgtgac tgtgaacgct     240
   actttcatgt attcggatgg tctgattcat aactcaggcg actgccggag tagcacctgt     300
   gaaggcctcg acctactcag gaaaatttca aatgcacaac ggatgggctg tgtcctcata     360
   gggccctcat gtacatactc caccttccag atgtaccttg acacagaatt gagctacccc     420
   atgatctcag ctggaagttt tggattgtca tgtgactata aagaaacctt aaccaggctg     480
   atgtctccag ctagaaagtt gatgtacttc ttggttaact tttggaaaac caacgatctg     540
   cccttcaaaa cttattcctg gagcacttcg tatgtttaca agaatggtac agaaactgag     600
   gactgtttct ggtaccttaa tgctctggag gctagcgttt cctatttctc ccacgaactc     660
   ggctttaagg tggtgttaag acaagataag gagtttcagg atatcttaat ggaccacaac     720
   aggaaaagca atgtgaccag tacttggagg acaatgtcac agcccctgac tatatga        777
<210> 5
   <211> 3213
   <212> DNA
   <213> Homo sapiens
<400> 5
   atgaagacgt tgctgttgga cttggctttg tggtcactgc tcttccagcc cgggtggctg 60
   tcctttagtt cccaggtgag tcagaactgc cacaatggca gctatgaaat cagcgtcctg 120
   atgatgggca actcagcctt tgcagagccc ctgaaaaact tggaagatgc ggtgaatgag 180
   gggctggaaa tagtgagagg acgtctgcaa aatgctggcc taaatgtgac tgtgaacgct 240
   actttcatgt attcggatgg tctgattcat aactcaggcg actgccggag tagcacctgt 300
   gaaggcctcg acctactcag gaaaatttca aatgcacaac ggatgggctg tgtcctcata 360
   gggccctcat gtacatactc caccttccag atgtaccttg acacagaatt gagctacccc 420
   atgatctcag ctggaagttt tggattgtca tgtgactata aagaaacctt aaccaggctg 480
   atgtctccag ctagaaagtt gatgtacttc ttggttaact tttggaaaac caacgatctg 540
   cccttcaaaa cttattcctg gagcacttcg tatgtttaca agaatggtac agaaactgag 600
   gactgtttct ggtaccttaa tgctctggag gctagcgttt cctatttctc ccacgaactc 660
   ggctttaagg tggtgttaag acaagataag gagtttcagg atatcttaat ggaccacaac 720
   aggaaaagca atgtgattat tatgtgtggt ggtccagagt tcctctacaa gctgaagggt 780
   gaccgagcag tggctgaaga cattgtcatt attctagtgg atcttttcaa tgaccagtac 840
   ttggaggaca atgtcacagc ccctgactat atgaaaaatg tccttgttct gacgctgtct 900
   cctgggaatt cccttctaaa tagctctttc tccaggaatc tatcaccaac aaaacgagac 960
   tttgctcttg cctatttgaa tggaatcctg ctctttggac atatgctgaa gatatttctt 1020
   gaaaatggag aaaatattac cacccccaaa tttgctcatg ctttcaggaa tctcactttt 1080
   gaagggtatg acggtccagt gaccttggat gactgggggg atgttgacag taccatggtg 1140
   cttctgtata cctctgtgga caccaagaaa tacaaggttc ttttgaccta tgatacccac 1200
   gtaaataaga cctatcctgt ggatatgagc cccacattca cttggaagaa ctctaaactt 1260
   cctaatgata ttacaggccg gggccctcag atcctgatga ttgcagtctt caccctcact 1320
   ggagctgtgg tgctgctcct gctcgtcgct ctcctgatgc tcagaaaata tagaaaagat 1380
   tatgaacttc gtcagaaaaa atggtcccac attcctcctg aaaatatctt tcctctggag 1440
   accaatgaga ccaatcatgt tagcctcaag atcgatgatg acaaaagacg agatacaatc 1500
   cagagactac gacagtgcaa atacgacaaa aagcgagtga ttctcaaaga tctcaagcac 1560
   aatgatggta atttcactga aaaacagaag atagaattga acaagattga ctattacaac 1620
   ctgaccaagt tctacggcac agtgaaactt gataccatga tcttcggggt gatagaatac 1680
   tgtgagagag gatccctccg ggaagtttta aatgacacaa tttcctaccc tgatggcaca 1740
   ttcatggatt gggagtttaa gatctctgtc ttgtatgaca ttgctaaggg aatgtcatat 1800
   ctgcactcca gtaagacaga agtccatggt cgtctgaaat ctaccaactg cgtagtggac 1860
   agtagaatgg tggtgaagat cactgatttt ggctgcaatt ccattttacc tccaaaaaag 1920
   gacctgtgga cagctccaga gcacctccgc caagccaaca tctctcagaa aggagatgtg 1980
   tacagctatg ggatcatcgc acaggagatc attctgcgga aagaaacctt ctacactttg 2040
   agctgtcggg accggaatga gaagattttc agagtggaaa attccaatgg aatgaaaccc 2100
   ttccgcccag atttattctt ggaaacagca gaggaaaaag agctagaagt gtacctactt 2160
   gtaaaaaact gttgggagga agatccagaa aagagaccag atttcaaaaa aattgagact 2220
   acacttgcca agatatttgg actttttcat gaccaaaaaa atgaaagcta tatggatacc 2280
   ttgatccgac gtctacagct atattctcga aacctggaac atctggtaga ggaaaggaca 2340
   cagctgtaca aggcagagag ggacagggct gacagactta actttatgtt gcttccaagg 2400
   ctagtggtaa agtctctgaa ggagaaaggc tttgtggagc cggaactata tgaggaagtt 2460
   acaatctact tcagtgacat tgtaggtttc actactatct gcaaatacag cacccccatg 2520
   gaagtggtgg acatgcttaa tgacatctat aagagttttg accacattgt tgatcatcat 2580
   gatgtctaca aggtggaaac catcggtgat gcgtacatgg tggctagtgg tttgcctaag 2640
   agaaatggca atcggcatgc aatagacatt gccaagatgg ccttggaaat cctcagcttc 2700
   atggggacct ttgagctgga gcatcttcct ggcctcccaa tatggattcg cattggagtt 2760
   cactctggtc cctgtgctgc tggagttgtg ggaatcaaga tgcctcgtta ttgtctattt 2820
   ggagatacgg tcaacacagc ctctaggatg gaatccactg gcctcccttt gagaattcac 2880
   gtgagtggct ccaccatagc catcctgaag agaactgagt gccagttcct ttatgaagtg 2940
   agaggagaaa catacttaaa gggaagagga aatgagacta cctactggct gactgggatg 3000
   aaggaccaga aattcaacct gccaacccct cctactgtgg agaatcaaca gcgtttgcaa 3060
   gcagaatttt cagacatgat tgccaactct ttacagaaaa gacaggcagc agggataaga 3120
   agccaaaaac ccagacgggt agccagctat aaaaaaggca ctctggaata cttgcagctg 3180
   aataccacag acaaggagag cacctatttt taa 3213
<210> 6
   <211> 550
   <212> DNA
   <213> Homo sapiens
<400> 6
   ggggacactt tgtatggcaa gtggaaccac tggcttggtg gattttgcta gatttttctg      60
   atttttaaac tcctgaaaaa tatcccagat aactgtcatg aagctggtaa ctatcttcct     120
   gctggtgacc atcagccttt gtagttactc tgctactgcc ttcctcatca acaaagtgcc     180
   ccttcctgtt gacaagttgg cacctttacc tctggacaac attcttccct ttatggatcc     240
   attaaagctt cttctgaaaa ctctgggcat ttctgttgag caccttgtgg aggggctaag     300
   gaagtgtgta aatgagctgg gaccagaggc ttctgaagct gtgaagaaac tgctggaggc     360
   gctatcacac ttggtgtgac atcaagataa agagcggagg tggatgggga tggaagatga     420
   tgctcctatc ctccctgcct gaaacctgtt ctaccaatta tagatcaaat gccctaaaat     480
   gtagtgaccc gtgaaaagga caaataaagc aatgaatact aaaaaaaaaa aaaaaaaaaa     540
   aaaaaaaaaa                                                            550
<210> 7
   <211> 786
   <212> DNA
   <213> Homo sapiens
<400> 7
   atggccgtga ctgcctgtca gggcttgggg ttcgtggttt cactgattgg gattgcgggc      60
   atcattgctg ccacctgcat ggaccagtgg agcacccaag acttgtacaa caaccccgta     120
   acagctgttt tcaactacca ggggctgtgg cgctcctgtg tccgagagag ctctggcttc     180
   accgagtgcc ggggctactt caccctgctg gggctgccag ccatgctgca ggcagtgcga     240
   gccctgatga tcgtaggcat cgtcctgggt gccattggcc tcctggtatc catctttgcc     300
   ctgaaatgca tccgcattgg cagcatggag gactctgcca aagccaacat gacactgacc     360
   tccgggatca tgttcattgt ctcaggtctt tgtgcaattg ctggagtgtc tgtgtttgcc     420
   aacatgctgg tgactaactt ctggatgtcc acagctaaca tgtacaccgg catgggtggg     480
   atggtgcaga ctgttcagac caggtacaca tttggtgcgg ctctgttcgt gggctgggtc     540
   gctggaggcc tcacactaat tgggggtgtg atgatgtgca tcgcctgccg gggcctggca     600
   ccagaagaaa ccaactacaa agccgtttct tatcatgcct caggccacag tgttgcctac     660
   aagcctggag gcttcaaggc cagcactggc tttgggtcca acaccaaaaa caagaagata     720
   tacgatggag gtgcccgcac agaggacgag gtacaatctt atccttccaa gcacgactat     780
   gtgtaa                                                                786
<210> 8
   <211> 180
   <212> DNA
   <213> Homo sapiens
<400> 8
   tgcgccacca tggccgtgac tgcctgtcag ggcttggggt tcgtggtttc actgattggg      60
   attgcgggca tcattgctgc cacctgcatg gaccagtgga gcacccaaga cttgtacaac     120
   aaccccgtaa cagctgtttt caactaccag gggctgtggc gctcctgtgt ccgagagagc     180
<210> 9
   <211> 309
   <212> PRT
   <213> Homo sapiens
<400> 9
   Met Asn Gly Thr Tyr Asn Thr Cys Gly Ser Ser Asp Leu Thr Trp Pro
   1               5                   10                  15
   Pro Ala Ile Lys Leu Gly Phe Tyr Ala Tyr Leu Gly Val Leu Leu Val
               20                  25                  30
   Leu Gly Leu Leu Leu Asn Ser Leu Ala Leu Trp Val Phe Cys Cys Arg
           35                  40                  45
   Met Gln Gln Trp Thr Glu Thr Arg Ile Tyr Met Thr Asn Leu Ala Val
       50                  55                  60
   Ala Asp Leu Cys Leu Leu Cys Thr Leu Pro Phe Val Leu His Ser Leu
   65                  70                  75                  80
   Arg Asp Thr Ser Asp Thr Pro Leu Cys Gln Leu Ser Gln Gly Ile Tyr
                   85                  90                  95
   Leu Thr Asn Arg Tyr Met Ser Ile Ser Leu Val Thr Ala Ile Ala Val
               100                 105                 110
   Asp Arg Tyr Val Ala Val Arg His Pro Leu Arg Ala Arg Gly Leu Arg
           115                 120                 125
   Ser Pro Arg Gln Ala Ala Ala Val Cys Ala Val Leu Trp Val Leu Val
       130                 135                 140
   Ile Gly Ser Leu Val Ala Arg Trp Leu Leu Gly Ile Gln Glu Gly Gly
   145                 150                 155                 160
   Phe Cys Phe Arg Ser Thr Arg His Asn Phe Asn Ser Met Arg Phe Pro
                   165                 170                 175
   Leu Leu Gly Phe Tyr Leu Pro Leu Ala Val Val Val Phe Cys Ser Leu
               180                 185                 190
   Lys Val Val Thr Ala Leu Ala Gln Arg Pro Pro Thr Asp Val Gly Gln
   195 200 205
   Ala Glu Ala Thr Arg Lys Ala Ala Arg Met Val Trp Ala Asn Leu Leu
   210 215 220
   Val Phe Val Val Cys Phe Leu Pro Leu His Val Gly Leu Thr Val Arg
   225 230 235 240
   Leu Ala Val Gly Trp Asn Ala Cys Ala Leu Leu Glu Thr Ile Arg Arg
   245 250 255
   Ala Leu Tyr Ile Thr Ser Lys Leu Ser Asp Ala Asn Cys Cys Leu Asp
   260 265 270
   Ala Ile Cys Tyr Tyr Tyr Met Ala Lys Glu Phe Gln Glu Ala Ser Ala
   275 280 285
   Leu Ala Val Ala Pro Arg Ala Lys Ala His Lys Ser Gln Asp Ser Leu
   290 295 300
   Cys Val Thr Leu Ala
   305
<210> 10
   <211> 394
   <212> PRT
   <213> Homo sapiens
<400> 10
   Met Thr Ala Gly Arg Ser Gln Glu Arg Arg Ala Gln Glu Met Gly Arg
   1 5 10 15
   Gly Ser Val Gln Gly Leu Asp Leu Lys Gly Asp Leu Glu Phe Phe Thr
   20 25 30
   Ala Pro Met Leu Ser Leu Arg Ser Phe Val Phe Val Gly Val Gly Ser
   35 40 45
   Gly Leu Thr Ser Ser His Ile Pro Ala Gln Arg Trp Ala Glu Trp Gly
   50 55 60
   Gln Cys Leu Ala Pro Pro Ala Arg Ser Leu Leu Thr Ser Gly Ser Leu
   65 70 75 80
   Cys Cys Pro Arg Thr Met Asn Gly Thr Tyr Asn Thr Cys Gly Ser Ser
   85 90 95
   Asp Leu Thr Trp Pro Pro Ala Ile Lys Leu Gly Phe Tyr Ala Tyr Leu
               100                 105                 110
   Gly Val Leu Leu Val Leu Gly Leu Leu Leu Asn Ser Leu Ala Leu Trp
           115                 120                 125
   Val Phe Cys Cys Arg Met Gln Gln Trp Thr Glu Thr Arg Ile Tyr Met
       130                 135                 140
   Thr Asn Leu Ala Val Ala Asp Leu Cys Leu Leu Cys Thr Leu Pro Phe
   145                 150                 155                 160
   Val Leu His Ser Leu Arg Asp Thr Ser Asp Thr Pro Leu Cys Gln Leu
                   165                 170                 175
   Ser Gln Gly Ile Tyr Leu Thr Asn Arg Tyr Met Ser Ile Ser Leu Val
               180                 185                 190
   Thr Ala Ile Ala Val Asp Arg Tyr Val Ala Val Arg His Pro Leu Arg
           195                 200                 205
   Ala Arg Gly Leu Arg Ser Pro Arg Gln Ala Ala Ala Val Cys Ala Val
       210                 215                 220
   Leu Trp Val Leu Val Ile Gly Ser Leu Val Ala Arg Trp Leu Leu Gly
   225                 230                 235                 240
   Ile Gln Glu Gly Gly Phe Cys Phe Arg Ser Thr Arg His Asn Phe Asn
                   245                 250                 255
   Ser Met Ala Phe Pro Leu Leu Gly Phe Tyr Leu Pro Leu Ala Val Val
               260                 265                 270
   Val Phe Cys Ser Leu Lys Val Val Thr Ala Leu Ala Gln Arg Pro Pro
           275                 280                 285
   Thr Asp Val Gly Gln Ala Glu Ala Thr Arg Lys Ala Ala Arg Met Val
       290                 295                 300
   Trp Ala Asn Leu Leu Val Phe Val Val Cys Phe Leu Pro Leu His Val
   305                 310                 315                 320
   Gly Leu Thr Val Arg Leu Ala Val Gly Trp Asn Ala Cys Ala Leu Leu
                   325                 330                 335
   Glu Thr Ile Arg Arg Ala Leu Tyr Ile Thr Ser Lys Leu Ser Asp Ala
               340                 345                 350
   Asn Cys Cys Leu Asp Ala Ile Cys Tyr Tyr Tyr Met Ala Lys Glu Phe
   355 360 365
   Gln Glu Ala Ser Ala Leu Ala Val Ala Pro Ser Ala Lys Ala His Lys
   370 375 380
   Ser Gln Asp Ser Leu Cys Val Thr Leu Ala
   385 390
<210> 11
   <211> 1073
   <212> PRT
   <213> Homo sapiens
<400> 11
   Met Lys Thr Leu Leu Leu Asp Leu Ala Leu Trp Ser Leu Leu Phe Gln
   1 5 10 15
   Pro Gly Trp Leu Ser Phe Ser Ser Gln Val Ser Gln Asn Cys His Asn
   20 25 30
   Gly Ser Tyr Glu Ile Ser Val Leu Met Met Gly Asn Ser Ala Phe Ala
   35 40 45
   Glu Pro Leu Lys Asn Leu Glu Asp Ala Val Asn Glu Gly Leu Glu Ile
   50 55 60
   Val Arg Gly Arg Leu Gln Asn Ala Gly Leu Asn Val Thr Val Asn Ala
   65 70 75 80
   Thr Phe Met Tyr Ser Asp Gly Leu Ile His Asn Ser Gly Asp Cys Arg
   85 90 95
   Ser Ser Thr Cys Glu Gly Leu Asp Leu Leu Arg Lys Ile Ser Asn Ala
   100 105 110
   Gln Arg Met Gly Cys Val Leu Ile Gly Pro Ser Cys Thr Tyr Ser Thr
   115 120 125
   Phe Gln Met Tyr Leu Asp Thr Glu Leu Ser Tyr Pro Met Ile Ser Ala
   130 135 140
   Gly Ser Phe Gly Leu Ser Cys Asp Tyr Lys Glu Thr Leu Thr Arg Leu
   145 150 155 160
   Met Ser Pro Ala Arg Lys Leu Met Tyr Phe Leu Val Asn Phe Trp Lys
   165 170 175
   Thr Asn Asp Leu Pro Phe Lys Thr Tyr Ser Trp Ser Thr Ser Tyr Val
               180                 185                 190
   Tyr Lys Asn Gly Thr Glu Thr Glu Asp Cys Phe Trp Tyr Leu Asn Ala
           195                 200                 205
   Leu Glu Ala Ser Val Ser Tyr Phe Ser His Glu Leu Gly Phe Lys Val
       210                 215                 220
   Val Leu Arg Gln Asp Lys Glu Phe Gln Asp Ile Leu Met Asp His Asn
   225                 230                 235                 240
   Arg Lys Ser Asn Val Ile Ile Met Cys Gly Gly Pro Glu Phe Leu Tyr
                   245                 250                 255
   Lys Leu Lys Gly Asp Arg Ala Val Ala Glu Asp Ile Val Ile Ile Leu
               260                 265                 270
   Val Asp Leu Phe Asn Asp Gln Tyr Leu Glu Asp Asn Val Thr Ala Pro
           275                 280                 285
   Asp Tyr Met Lys Asn Val Leu Val Leu Thr Leu Ser Pro Gly Asn Ser
       290                 295                 300
   Leu Leu Asn Ser Ser Phe Ser Arg Asn Leu Ser Pro Thr Lys Arg Asp
   305                 310                 315                 320
   Phe Ala Leu Ala Tyr Leu Asn Gly Ile Leu Leu Phe Gly His Met Leu
                   325                 330                 335
   Lys Ile Phe Leu Glu Asn Gly Glu Asn Ile Thr Thr Pro Lys Phe Ala
               340                 345                 350
   His Ala Phe Arg Asn Leu Thr Phe Glu Gly Tyr Asp Gly Pro Val Thr
           355                 360                 365
   Leu Asp Asp Trp Gly Asp Val Asp Ser Thr Met Val Leu Leu Tyr Thr
       370                 375                 380
   Ser Val Asp Thr Lys Lys Tyr Lys Val Leu Leu Thr Tyr Asp Thr His
   385                 390                 395                 400
   Val Asn Lys Thr Tyr Pro Val Asp Met Ser Pro Thr Phe Thr Trp Lys
                   405                 410                 415
   Asn Ser Lys Leu Pro Asn Asp Ile Thr Gly Arg Gly Pro Gln Ile Leu
   420 425 430
   Met Ile Ala Val Phe Thr Leu Thr Gly Ala Val Val Leu Leu Leu Leu
   435 440 445
   Val Ala Leu Leu Met Leu Arg Lys Tyr Arg Lys Asp Tyr Glu Leu Arg
   450 455 460
   Gln Lys Lys Trp Ser His Ile Pro Pro Glu Asn Ile Phe Pro Leu Glu
   465 470 475 480
   Thr Asn Glu Thr Asn His Val Ser Leu Lys Ile Asp Asp Asp Lys Arg
   485 490 495
   Arg Asp Thr Ile Gln Arg Leu Arg Gln Cys Lys Tyr Asp Lys Lys Arg
   500 505 510
   Val Ile Leu Lys Asp Leu Lys His Asn Asp Gly Asn Phe Thr Glu Lys
   515 520 525
   Gln Lys Ile Glu Leu Asn Lys Leu Leu Gln Ile Asp Tyr Tyr Asn Leu
   530 535 540
   Thr Lys Phe Tyr Gly Thr Val Lys Leu Asp Thr Met Ile Phe Gly Val
   545 550 555 560
   Ile Glu Tyr Cys Glu Arg Gly Ser Leu Arg Glu Val Leu Asn Asp Thr
   565 570 575
   Ile Ser Tyr Pro Asp Gly Thr Phe Met Asp Trp Glu Phe Lys Ile Ser
   580 585 590
   Val Leu Tyr Asp Ile Ala Lys Gly Met Ser Tyr Leu His Ser Ser Lys
   595 600 605
   Thr Glu Val His Gly Arg Leu Lys Ser Thr Asn Cys Val Val Asp Ser
   610 615 620
   Arg Met Val Val Lys Ile Thr Asp Phe Gly Cys Asn Ser Ile Leu Pro
   625 630 635 640
   Pro Lys Lys Asp Leu Trp Thr Ala Pro Glu His Leu Arg Gln Ala Asn
   645 650 655
   Ile Ser Gln Lys Gly Asp Val Tyr Ser Tyr Gly Ile Ile Ala Gln Glu
   660 665 670
   Ile Ile Leu Arg Lys Glu Thr Phe Tyr Thr Leu Ser Cys Arg Asp Arg
   675 680 685
   Asn Glu Lys Ile Phe Arg Val Glu Asn Ser Asn Gly Met Lys Pro Phe
   690 695 700
   Arg Pro Asp Leu Phe Leu Glu Thr Ala Glu Glu Lys Glu Leu Glu Val
   705 710 715 720
   Tyr Leu Leu Val Lys Asn Cys Trp Glu Glu Asp Pro Glu Lys Arg Pro
   725 730 735
   Asp Phe Lys Lys Ile Glu Thr Thr Leu Ala Lys Ile Phe Gly Leu Phe
   740 745 750
   His Asp Gln Lys Asn Glu Ser Tyr Met Asp Thr Leu Ile Arg Arg Leu
   755 760 765
   Gln Leu Tyr Ser Arg Asn Leu Glu His Leu Val Glu Glu Arg Thr Gln
   770 775 780
   Leu Tyr Lys Ala Glu Arg Asp Arg Ala Asp Arg Leu Asn Phe Met Leu
   785 790 795 800
   Leu Pro Arg Leu Val Val Lys Ser Leu Lys Glu Lys Gly Phe Val Glu
   805 810 815
   Pro Glu Leu Tyr Glu Glu Val Thr Ile Tyr Phe Ser Asp Ile Val Gly
   820 825 830
   Phe Thr Thr Ile Cys Lys Tyr Ser Thr Pro Met Glu Val Val Asp Met
   835 840 845
   Leu Asn Asp Ile Tyr Lys Ser Phe Asp His Ile Val Asp His His Asp
   850 855 860
   Val Tyr Lys Val Glu Thr Ile Gly Asp Ala Tyr Met Val Ala Ser Gly
   865 870 875 880
   Leu Pro Lys Arg Asn Gly Asn Arg His Ala Ile Asp Ile Ala Lys Met
   885 890 895
   Ala Leu Glu Ile Leu Ser Phe Met Gly Thr Phe Glu Leu Glu His Leu
   900 905 910
   Pro Gly Leu Pro Ile Trp Ile Arg Ile Gly Val His Ser Gly Pro Cys
   915 920 925
   Ala Ala Gly Val Val Gly Ile Lys Met Pro Arg Tyr Cys Leu Phe Gly
       930                 935                 940
   Asp Thr Val Asn Thr Ala Ser Arg Met Glu Ser Thr Gly Leu Pro Leu
   945                 950                 955                 960
   Arg Ile His Val Ser Gly Ser Thr Ile Ala Ile Leu Lys Arg Thr Glu
                   965                 970                 975
   Cys Gln Phe Leu Tyr Glu Val Arg Gly Glu Thr Tyr Leu Lys Gly Arg
               980                 985                 990
   Gly Asn Glu Thr Thr Tyr Trp Leu  Thr Gly Met Lys Asp  Gln Lys Phe
           995                 1000                1005
   Asn Leu  Pro Thr Pro Pro Thr  Val Glu Asn Gln Gln  Arg Leu Gln
       1010                1015                1020
   Ala Glu  Phe Ser Asp Met Ile  Ala Asn Ser Leu Gln  Lys Arg Gln
       1025                1030                1035
   Ala Ala  Gly Ile Arg Ser Gln  Lys Pro Arg Arg Val  Ala Ser Tyr
       1040                1045                1050
   Lys Lys  Gly Thr Leu Glu Tyr  Leu Gln Leu Asn Thr  Thr Asp Lys
       1055                1060                1065
   Glu Ser  Thr Tyr Phe
       1070
<210> 12
   <211> 111
   <212> PRT
   <213> Homo sapiens
<400> 12
   Met Lys Thr Leu Leu Leu Asp Leu Ala Leu Trp Ser Leu Leu Phe Gln
   1               5                   10                  15
   Pro Gly Trp Leu Ser Phe Ser Ser Gln Val Ser Gln Asn Cys His Asn
               20                  25                  30
   Gly Ser Tyr Glu Ile Ser Val Leu Met Met Gly Asn Ser Ala Phe Ala
           35                  40                  45
   Glu Pro Leu Lys Asn Leu Glu Asp Ala Val Asn Glu Gly Leu Glu Ile
       50                  55                  60
   Val Arg Gly Arg Leu Gln Asn Ala Gly Leu Asn Val Thr Val Asn Ala
   65 70 75 80
   Thr Phe Met Tyr Ser Asp Gly Leu Ile His Asn Ser Gly Asp Cys Arg
   85 90 95
   Ser Ser Thr Cys Glu Gly Leu Asp Leu Leu Arg Lys Ile Ser Pro
   100 105 110
<210> 13
   <211> 258
   <212> PRT
   <213> Homo sapiens
<400> 13
   Met Lys Thr Leu Leu Leu Asp Leu Ala Leu Trp Ser Leu Leu Phe Gln
   1 5 10 15
   Pro Gly Trp Leu Ser Phe Ser Ser Gln Val Ser Gln Asn Cys His Asn
   20 25 30
   Gly Ser Tyr Glu Ile Ser Val Leu Met Met Gly Asn Ser Ala Phe Ala
   35 40 45
   Glu Pro Leu Lys Asn Leu Glu Asp Ala Val Asn Glu Gly Leu Glu Ile
   50 55 60
   Val Arg Gly Arg Leu Gln Asn Ala Gly Leu Asn Val Thr Val Asn Ala
   65 70 75 80
   Thr Phe Met Tyr Ser Asp Gly Leu Ile His Asn Ser Gly Asp Cys Arg
   85 90 95
   Ser Ser Thr Cys Glu Gly Leu Asp Leu Leu Arg Lys Ile Ser Asn Ala
   100 105 110
   Gln Arg Met Gly Cys Val Leu Ile Gly Pro Ser Cys Thr Tyr Ser Thr
   115 120 125
   Phe Gln Met Tyr Leu Asp Thr Glu Leu Ser Tyr Pro Met Ile Ser Ala
   130 135 140
   Gly Ser Phe Gly Leu Ser Cys Asp Tyr Lys Glu Thr Leu Thr Arg Leu
   145 150 155 160
   Met Ser Pro Ala Arg Lys Leu Met Tyr Phe Leu Val Asn Phe Trp Lys
   165 170 175
   Thr Asn Asp Leu Pro Phe Lys Thr Tyr Ser Trp Ser Thr Ser Tyr Val
   180 185 190
   Tyr Lys Asn Gly Thr Glu Thr Glu Asp Cys Phe Trp Tyr Leu Asn Ala
   195 200 205
   Leu Glu Ala Ser Val Ser Tyr Phe Ser His Glu Leu Gly Phe Lys Val
   210 215 220
   Val Leu Arg Gln Asp Lys Glu Phe Gln Asp Ile Leu Met Asp His Asn
   225 230 235 240
   Arg Lys Ser Asn Val Thr Ser Thr Trp Arg Thr Met Ser Gln Pro Leu
   245 250 255
   Thr Ile
<210> 14
   <211> 1070
   <212> PRT
   <213> Homo sapiens
<400> 14
   Met Lys Thr Leu Leu Leu Asp Leu Ala Leu Trp Ser Leu Leu Phe Gln
   1 5 10 15
   Pro Gly Trp Leu Ser Phe Ser Ser Gln Val Ser Gln Asn Cys His Asn
   20 25 30
   Gly Ser Tyr Glu Ile Ser Val Leu Met Met Gly Asn Ser Ala Phe Ala
   35 40 45
   Glu Pro Leu Lys Asn Leu Glu Asp Ala Val Asn Glu Gly Leu Glu Ile
   50 55 60
   Val Arg Gly Arg Leu Gln Asn Ala Gly Leu Asn Val Thr Val Asn Ala
   65 70 75 80
   Thr Phe Met Tyr Ser Asp Gly Leu Ile His Asn Ser Gly Asp Cys Arg
   85 90 95
   Ser Ser Thr Cys Glu Gly Leu Asp Leu Leu Arg Lys Ile Ser Asn Ala
   100 105 110
   Gln Arg Met Gly Cys Val Leu Ile Gly Pro Ser Cys Thr Tyr Ser Thr
   115 120 125
   Phe Gln Met Tyr Leu Asp Thr Glu Leu Ser Tyr Pro Met Ile Ser Ala
       130                 135                 140
   Gly Ser Phe Gly Leu Ser Cys Asp Tyr Lys Glu Thr Leu Thr Arg Leu
   145                 150                 155                 160
   Met Ser Pro Ala Arg Lys Leu Met Tyr Phe Leu Val Asn Phe Trp Lys
                   165                 170                 175
   Thr Asn Asp Leu Pro Phe Lys Thr Tyr Ser Trp Ser Thr Ser Tyr Val
               180                 185                 190
   Tyr Lys Asn Gly Thr Glu Thr Glu Asp Cys Phe Trp Tyr Leu Asn Ala
           195                 200                 205
   Leu Glu Ala Ser Val Ser Tyr Phe Ser His Glu Leu Gly Phe Lys Val
       210                 215                 220
   Val Leu Arg Gln Asp Lys Glu Phe Gln Asp Ile Leu Met Asp His Asn
   225                 230                 235                 240
   Arg Lys Ser Asn Val Ile Ile Met Cys Gly Gly Pro Glu Phe Leu Tyr
                   245                 250                 255
   Lys Leu Lys Gly Asp Arg Ala Val Ala Glu Asp Ile Val Ile Ile Leu
               260                 265                 270
   Val Asp Leu Phe Asn Asp Gln Tyr Leu Glu Asp Asn Val Thr Ala Pro
           275                 280                 285
   Asp Tyr Met Lys Asn Val Leu Val Leu Thr Leu Ser Pro Gly Asn Ser
       290                 295                 300
   Leu Leu Asn Ser Ser Phe Ser Arg Asn Leu Ser Pro Thr Lys Arg Asp
   305                 310                 315                 320
   Phe Ala Leu Ala Tyr Leu Asn Gly Ile Leu Leu Phe Gly His Met Leu
                   325                 330                 335
   Lys Ile Phe Leu Glu Asn Gly Glu Asn Ile Thr Thr Pro Lys Phe Ala
               340                 345                 350
   His Ala Phe Arg Asn Leu Thr Phe Glu Gly Tyr Asp Gly Pro Val Thr
           355                 360                 365
   Leu Asp Asp Trp Gly Asp Val Asp Ser Thr Met Val Leu Leu Tyr Thr
       370                 375                 380
   Ser Val Asp Thr Lys Lys Tyr Lys Val Leu Leu Thr Tyr Asp Thr His
   385                 390                 395                 400
   Val Asn Lys Thr Tyr Pro Val Asp Met Ser Pro Thr Phe Thr Trp Lys
                   405                 410                 415
   Asn Ser Lys Leu Pro Asn Asp Ile Thr Gly Arg Gly Pro Gln Ile Leu
               420                 425                 430
   Met Ile Ala Val Phe Thr Leu Thr Gly Ala Val Val Leu Leu Leu Leu
           435                 440                 445
   Val Ala Leu Leu Met Leu Arg Lys Tyr Arg Lys Asp Tyr Glu Leu Arg
       450                 455                 460
   Gln Lys Lys Trp Ser His Ile Pro Pro Glu Asn Ile Phe Pro Leu Glu
   465                 470                 475                 480
   Thr Asn Glu Thr Asn His Val Ser Leu Lys Ile Asp Asp Asp Lys Arg
                   485                 490                 495
   Arg Asp Thr Ile Gln Arg Leu Arg Gln Cys Lys Tyr Asp Lys Lys Arg
               500                 505                 510
   Val Ile Leu Lys Asp Leu Lys His Asn Asp Gly Asn Phe Thr Glu Lys
           515                 520                 525
   Gln Lys Ile Glu Leu Asn Lys Ile Asp Tyr Tyr Asn Leu Thr Lys Phe
       530                 535                 540
   Tyr Gly Thr Val Lys Leu Asp Thr Met Ile Phe Gly Val Ile Glu Tyr
   545                 550                 555                 560
   Cys Glu Arg Gly Ser Leu Arg Glu Val Leu Asn Asp Thr Ile Ser Tyr
                   565                 570                 575
   Pro Asp Gly Thr Phe Met Asp Trp Glu Phe Lys Ile Ser Val Leu Tyr
               580                 585                 590
   Asp Ile Ala Lys Gly Met Ser Tyr Leu His Ser Ser Lys Thr Glu Val
           595                 600                 605
   His Gly Arg Leu Lys Ser Thr Asn Cys Val Val Asp Ser Arg Met Val
       610                 615                 620
   Val Lys Ile Thr Asp Phe Gly Cys Asn Ser Ile Leu Pro Pro Lys Lys
   625 630 635 640
   Asp Leu Trp Thr Ala Pro Glu His Leu Arg Gln Ala Asn Ile Ser Gln
   645 650 655
   Lys Gly Asp Val Tyr Ser Tyr Gly Ile Ile Ala Gln Glu Ile Ile Leu
   660 665 670
   Arg Lys Glu Thr Phe Tyr Thr Leu Ser Cys Arg Asp Arg Asn Glu Lys
   675 680 685
   Ile Phe Arg Val Glu Asn Ser Asn Gly Met Lys Pro Phe Arg Pro Asp
   690 695 700
   Leu Phe Leu Glu Thr Ala Glu Glu Lys Glu Leu Glu Val Tyr Leu Leu
   705 710 715 720
   Val Lys Asn Cys Trp Glu Glu Asp Pro Glu Lys Arg Pro Asp Phe Lys
   725 730 735
   Lys Ile Glu Thr Thr Leu Ala Lys Ile Phe Gly Leu Phe His Asp Gln
   740 745 750
   Lys Asn Glu Ser Tyr Met Asp Thr Leu Ile Arg Arg Leu Gln Leu Tyr
   755 760 765
   Ser Arg Asn Leu Glu His Leu Val Glu Glu Arg Thr Gln Leu Tyr Lys
   770 775 780
   Ala Glu Arg Asp Arg Ala Asp Arg Leu Asn Phe Met Leu Leu Pro Arg
   785 790 795 800
   Leu Val Val Lys Ser Leu Lys Glu Lys Gly Phe Val Glu Pro Glu Leu
   805 810 815
   Tyr Glu Glu Val Thr Ile Tyr Phe Ser Asp Ile Val Gly Phe Thr Thr
   820 825 830
   Ile Cys Lys Tyr Ser Thr Pro Met Glu Val Val Asp Met Leu Asn Asp
   835 840 845
   Ile Tyr Lys Ser Phe Asp His Ile Val Asp His His Asp Val Tyr Lys
   850 855 860
   Val Glu Thr Ile Gly Asp Ala Tyr Met Val Ala Ser Gly Leu Pro Lys
   865 870 875 880
   Arg Asn Gly Asn Arg His Ala Ile Asp Ile Ala Lys Met Ala Leu Glu
                   885                 890                 895
   Ile Leu Ser Phe Met Gly Thr Phe Glu Leu Glu His Leu Pro Gly Leu
               900                 905                 910
   Pro Ile Trp Ile Arg Ile Gly Val His Ser Gly Pro Cys Ala Ala Gly
           915                 920                 925
   Val Val Gly Ile Lys Met Pro Arg Tyr Cys Leu Phe Gly Asp Thr Val
       930                 935                 940
   Asn Thr Ala Ser Arg Met Glu Ser Thr Gly Leu Pro Leu Arg Ile His
   945                 950                 955                 960
   Val Ser Gly Ser Thr Ile Ala Ile Leu Lys Arg Thr Glu Cys Gln Phe
                   965                 970                 975
   Leu Tyr Glu Val Arg Gly Glu Thr Tyr Leu Lys Gly Arg Gly Asn Glu
               980                 985                 990
   Thr Thr Tyr Trp Leu Thr Gly Met  Lys Asp Gln Lys Phe  Asn Leu Pro
           995                 1000                1005
   Thr Pro  Pro Thr Val Glu Asn  Gln Gln Arg Leu Gln  Ala Glu Phe
       1010                1015                1020
   Ser Asp  Met Ile Ala Asn Ser  Leu Gln Lys Arg Gln  Ala Ala Gly
       1025                1030                1035
   Ile Arg  Ser Gln Lys Pro Arg  Arg Val Ala Ser Tyr  Lys Lys Gly
       1040                1045                1050
   Thr Leu  Glu Tyr Leu Gln Leu  Asn Thr Thr Asp Lys  Glu Ser Thr
       1055                1060                1065
   Tyr Phe
       1070
<210> 15
   <211> 93
   <212> PRT
   <213> Homo sapiens
<400> 15
   Met Lys Leu Val Thr Ile Phe Leu Leu Val Thr Ile Ser Leu Cys Ser
   1               5                   10                  15
   Tyr Ser Ala Thr Ala Lys Leu Ile Asn Lys Cys Pro Leu Pro Val Asp
   20 25 30
   Lys Leu Ala Pro Leu Pro Leu Asp Asn Ile Leu Pro Phe Met Asp Pro
   35 40 45
   Leu Lys Leu Leu Leu Lys Thr Leu Gly Ile Ser Val Glu His Leu Val
   50 55 60
   Glu Gly Leu Arg Lys Cys Val Asn Glu Leu Gly Pro Glu Ala Ser Glu
   65 70 75 80
   Ala Val Lys Lys Leu Leu Glu Ala Leu Ser His Leu Val
   85 90
<210> 16
   <211> 261
   <212> PRT
   <213> Homo sapiens
<400> 16
   Met Ala Val Thr Ala Cys Gln Gly Leu Gly Phe Val Val Ser Leu Ile
   1 5 10 15
   Gly Ile Ala Gly Ile Ile Ala Ala Thr Cys Met Asp Gln Trp Ser Thr
   20 25 30
   Gln Asp Leu Tyr Asn Asn Pro Val Thr Ala Val Phe Asn Tyr Gln Gly
   35 40 45
   Leu Trp Arg Ser Cys Val Arg Glu Ser Ser Gly Phe Thr Glu Cys Arg
   50 55 60
   Gly Tyr Phe Thr Leu Leu Gly Leu Pro Ala Met Leu Gln Ala Val Arg
   65 70 75 80
   Ala Leu Met Ile Val Gly Ile Val Leu Gly Ala Ile Gly Leu Leu Val
   85 90 95
   Ser Ile Phe Ala Leu Lys Cys Ile Arg Ile Gly Ser Met Glu Asp Ser
   100 105 110
   Ala Lys Ala Asn Met Thr Leu Thr Ser Gly Ile Met Phe Ile Val Ser
   115 120 125
   Gly Leu Cys Ala Ile Ala Gly Val Ser Val Phe Ala Asn Met Leu Val
   130 135 140
   Thr Asn Phe Trp Met Ser Thr Ala Asn Met Tyr Thr Gly Met Gly Gly
   145                 150                 155                 160
   Met Val Gln Thr Val Gln Thr Arg Tyr Thr Phe Gly Ala Ala Leu Phe
                   165                 170                 175
   Val Gly Trp Val Ala Gly Gly Leu Thr Leu Ile Gly Gly Val Met Met
               180                 185                 190
   Cys Ile Ala Cys Arg Gly Leu Ala Pro Glu Glu Thr Asn Tyr Lys Ala
           195                 200                 205
   Val Ser Tyr His Ala Ser Gly His Ser Val Ala Tyr Lys Pro Gly Gly
       210                 215                 220
   Phe Lys Ala Ser Thr Gly Phe Gly Ser Asn Thr Lys Asn Lys Lys Ile
   225                 230                 235                 240
   Tyr Asp Gly Gly Ala Arg Thr Glu Asp Glu Val Gln Ser Tyr Pro Ser
                   245                 250                 255
   Lys His Asp Tyr Val
               260
<210> 17
   <211> 10
   <212> PRT
   <213> Homo sapiens
<400> 17
   Asp Gln Trp Ser Thr Gln Asp Leu Tyr Asn
   1               5                   10
<210> 18
   <211> 11
   <212> PRT
   <213> Homo sapiens
<400> 18
   Asn Asn Pro Val Thr Ala Val Phe Asn Tyr Gln
   1               5                   10
<210> 19
   <211> 47
   <212> PRT
   <213> Homo sapiens
<400> 19
   Met Ala Val Thr Ala Cys Gln Gly Leu Gly Phe Val Val Ser Leu Ile
   1               5                   10                  15
   Gly Ile Ala Gly Ile Ile Ala Ala Thr Cys Met Asp Gln Trp Ser Thr
               20                  25                  30
   Gln Asp Leu Tyr Asn Asn Pro Val Thr Ala Val Phe Asn Tyr Gln
           35                  40                  45
<210> 20
   <211> 21
   <212> DNA
   <213> Künstliche Sequenz
<220>
   <223> Beschreibung der künstlichen Sequenz: Oligonukleotid
<400> 20
   aggtacatga gcatcagcct g 21
<210> 21
   <211> 21
   <212> DNA
   <213> Künstliche Sequenz
<220>
   <223> Beschreibung der künstlichen Sequenz: Oligonukleotid
<400> 21
   gcagcagttg gcatctgaga g 21
<210> 22
   <211> 21
   <212> DNA
   <213> Künstliche Sequenz
<220>
   <223> Beschreibung der künstlichen Sequenz: Oligonukleotid
<400> 22
   gcaatagaca ttgccaagat g 21
<210> 23
   <211> 21
   <212> DNA
   <213> Künstliche Sequenz
<220>
   <223> Beschreibung der künstlichen Sequenz: Oligonukleotid
<400> 23
   aacgctgttg attctccaca g 21
<210> 24
   <211> 33
   <212> DNA
   <213> Künstliche Sequenz
<220>
   <223> Beschreibung der künstlichen Sequenz: Oligonukleotid
<400> 24
   ggatcctcct ttagttccca ggtgagtcag aac 33
<210> 25
   <211> 21
   <212> DNA
   <213> Künstliche Sequenz
<220>
   <223> Beschreibung der künstlichen Sequenz: Oligonukleotid
<400> 25
   tgctctggag gctagcgttt c 21
<210> 26
   <211> 21
   <212> DNA
   <213> Künstliche Sequenz
<220>
   <223> Beschreibung der künstlichen Sequenz: Oligonukleotid
<400> 26
   accaatcatg ttagcctcaa g 21
<210> 27
   <211> 21
   <212> DNA
   <213> Künstliche Sequenz
<220>
   <223> Beschreibung der künstlichen Sequenz: Oligonukleotid
<400> 27
   agctatggga tcatcgcaca g 21
<210> 28
   <211> 21
   <212> DNA
   <213> Künstliche Sequenz
<220>
   <223> Beschreibung der künstlichen Sequenz: Oligonukleotid
<400> 28
   cctttgagct ggagcatctt c 21
<210> 29
   <211> 21
   <212> DNA
   <213> Künstliche Sequenz
<220>
   <223> Beschreibung der künstlichen Sequenz: Oligonukleotid
<400> 29
   ctttctagct ggagacatca g 21
<210> 30
   <211> 21
   <212> DNA
   <213> Künstliche Sequenz
<220>
   <223> Beschreibung der künstlichen Sequenz: Oligonukleotid
<400> 30
   caccatggta ctgtcaacat c 21
<210> 31
   <211> 21
   <212> DNA
   <213> Künstliche Sequenz
<220>
   <223> Beschreibung der künstlichen Sequenz: Oligonukleotid
<400> 31
   atgtcataca agacagagat c 21
<210> 32
   <211> 21
   <212> DNA
   <213> Künstliche Sequenz
<220>
   <223> Beschreibung der künstlichen Sequenz: Oligonukleotid
<400> 32
   tctgccttgt acagctgtgt c 21
<210> 33
   <211> 21
   <212> DNA
   <213> Künstliche Sequenz
<220>
   <223> Beschreibung der künstlichen Sequenz: Oligonukleotid
<400> 33
   tctgtggtat tcagctgcaa g 21
<210> 34
   <211> 22
   <212> DNA
   <213> Künstliche Sequenz
<220>
   <223> Beschreibung der künstlichen Sequenz: Oligonukleotid
<400> 34
   tactcaggaa aatttcacct tg 22
<210> 35
   <211> 27
   <212> DNA
   <213> Künstliche Sequenz
<220>
   <223> Beschreibung der künstlichen Sequenz: Oligonukleotid
<400> 35
   gaccacaaca ggaaaagcaa tgtgacc 27
<210> 36
   <211> 22
   <212> DNA
   <213> Künstliche Sequenz
<220>
   <223> Beschreibung der künstlichen Sequenz: Oligonukleotid
<400> 36
   gatagaattg aacaagattg ac 22
<210> 37
   <211> 21
   <212> DNA
   <213> Künstliche Sequenz
<220>
   <223> Beschreibung der künstlichen Sequenz: Oligonukleotid
<400> 37
   cagcctttgt agttactctg c 21
<210> 38
   <211> 21
   <212> DNA
   <213> Künstliche Sequenz
<220>
   <223> Beschreibung der künstlichen Sequenz: Oligonukleotid
<400> 38
   tgtcacacca agtgtgatag c 21
<210> 39
   <211> 28
   <212> DNA
   <213> Künstliche Sequenz
<220>
   <223> Beschreibung der künstlichen Sequenz: Oligonukleotid
<400> 39
   ggttcgtggt ttcactgatt gggattgc 28
<210> 40
   <211> 27
   <212> DNA
   <213> Künstliche Sequenz
<220>
   <223> Beschreibung der künstlichen Sequenz: Oligonukleotid
<400> 40
   cggctttgta gttggtttct tctggtg 27
<210> 41
   <211> 3814
   <212> DNA
   <213> Homo sapiens
<400> 41
   ctattgaagc cacctgctca ggacaatgaa attcttcagt tacattctgg tttatcgccg 60
   atttctcttc gtggttttca ctgtgttggt tttactacct ctgcccatcg tcctccacac 120
   caaggaagca gaatgtgcct acacactctt tgtggtcgcc acattttggc tcacagaagc 180
   attgcctctg tcggtaacag ctttgctacc tagtttaatg ttacccatgt ttgggatcat 240
   gccttctaag aaggtggcat ctgcttattt caaggatttt cacttactgc taattggagt 300
   tatctgttta gcaacatcca tagaaaaatg gaatttgcac aagagaattg ctctgaaaat 360
   ggtgatgatg gttggtgtaa atcctgcatg gctgacgctg gggttcatga gcagcactgc 420
   ctttttgtct atgtggctca gcaacacctc gacggctgcc atggtgatgc ccattgcgga 480
   ggctgtagtg cagcagatca tcaatgcaga agcagaggtc gaggccactc agatgactta 540
   cttcaacgga tcaaccaacc acggactaga aattgatgaa agtgttaatg gacatgaaat 600
   aaatgagagg aaagagaaaa caaaaccagt tccaggatac aataatgata cagggaaaat 660
   ttcaagcaag gtggagttgg aaaagaactc aggcatgaga accaaatatc gaacaaagaa 720
   gggccacgtg acacgtaaac ttacgtgttt gtgcattgcc tactcttcta ccattggtgg 780
   actgacaaca atcactggta cctccaccaa cttgatcttt gcagagtatt tcaatacacg 840
   ctatcctgac tgtcgttgcc tcaactttgg atcatggttt acgttttcct tcccagctgc 900
   ccttatcatt ctactcttat cctggatctg gcttcagtgg cttttcctag gattcaattt 960
   taaggagatg ttcaaatgtg gcaaaaccaa aacagtccaa caaaaagctt gtgctgaggt 1020
   gattaagcaa gaataccaaa agcttgggcc aataaggtat caagaaattg tgaccttggt 1080
   cctcttcatt ataatggctc tgctatggtt tagtcgagac cccggatttg ttcctggttg 1140
   gtctgcactt ttttcagagt accctggttt tgctacagat tcaactgttg ctttacttat 1200
   agggctgcta ttctttctta tcccagctaa gacactgact aaaactacac ctacaggaga 1260
   aattgttgct tttgattact ctccactgat tacttggaaa gaattccagt cattcatgcc 1320
   ctgggatata gccattcttg ttggtggagg gtttgccctg gcagatggtt gtgaggagtc 1380
   tggattatct aagtggatag gaaataaatt atctcctctg ggttcattac cagcatggct 1440
   aataattctg atatcttctt tgatggtgac atctttaact gaggtagcca gcaatccagc 1500
   taccattaca ctctttctcc caatattatc tccattggcc gaagccattc atgtgaaccc 1560
   tctttatatt ctgatacctt ctactctgtg tacttcattt gcattcctcc taccagtagc 1620
   aaatccaccc aatgctattg tcttttcata tggtcatctg aaagtcattg acatggttaa 1680
   agctggactt ggtgtcaaca ttgttggtgt tgctgtggtt atgcttggca tatgtacttg 1740
   gattgtaccc atgtttgacc tctacactta cccttcgtgg gctcctgcta tgagtaatga 1800
   gaccatgcca taataagcac aaaatttctg actatcttgc ggtaatttct ggaagacatt 1860
   aatgattgac tgtaaaatgt ggctctaaat aactaatgac acacatttaa atcagttatg 1920
   gtgtagctgc tgcaattccc gtgaataccc gaaacctgct ggtataactc agagtccata 1980
   tttgttattg cagtgcaact aaagagcatc tatgtgcctt catcaagaag cccatgtttt 2040
   gagattttgc tcatgaacca tctgcaactt gcttcatcat aagaataatt tataacttga 2100
   ccttcaaaga gattagagca tttgtttcat cttacagttg gagttcaatg taacatttta 2160
   aatgcaattt attatttcag aaatttccca tgaaactaaa aatagaaaat aagatataca 2220
   agttaattcg gtacttggat aaatcatttc tgcattgttg ttccagagaa tttgctgaga 2280
   aatcaaagcc atggtcatct ggtgatgaag agaaaaggtt aatctaaatg atatgtgcat 2340
   ttcctcattt aaaaaatcca attggattat tcttaatata tacatgtaat atgaaaattg 2400
   agattgaagc actaattcca aaattatggc tgaatatact aaataacaga aaagttacag 2460
   ataagaattt atttctactg aactctatag ttagtgtaat ataattcata tttttatgat 2520
   attggcacac tgagaaattc attttgtaga gctatggata aggcttgcta tgatttgcac 2580
   tattagtaca gtatagttag aaaggaaagc tgaacactat aaaactatta acatattttc 2640
   gtatatgagt aacaactttg cttaagtgtt tatcttagtt cagaaataca taatgtcata 2700
   tgttaaaaat aaagagatgt agaaatctaa atgaattatc actgtgtata cagacagaaa 2760
   aatcacataa ctctggtgtg ttaacattgc aatgaaaaaa tgaaaaaaag aaggaaaaaa 2820
   gaataagaat gaaaactgct gacgtattac aaaacagaaa aataaatgat ttaaaatcaa 2880
   atcaaaaaga aaaaaactaa acatttaaac aaaaatggga taagaatagt cttctagaag 2940
   tgaggatgcg taaaagaatg agtttccaat taccctgatg tgacaattac acattgtaga 3000
   caggtagcaa aatatcacat acacccccaa aatatgtaca aatattatat atcaataaat 3060
   aaatttttaa agagtaagtg ctattggcat tccaaaattc agctaaagga aaaatgatca 3120
   aaaacaaagt aaggtgcaca gttagcaaaa gatgcagatg ttatatcaca gcaattctca 3180
   tgctaaaaat acaacaaaag acaaagcaaa aaataaacct ttgctttttt tttttttttt 3240
   tttttttttt gagacggagt ctcgctctgt cgcccaggct ggagtgcagt ggcgggatct 3300
   cggctcactg caagctccgc ctcccaggtt cacgccattc tcctgcctca gccaaacctt   3360
   tgctattttt aatcttcgtt ggcactttcc agctgttact gaccttgtca ttttttgttc    3420
   aaataagatt atttacaaac ttattcttga aactaaatat agtaaagagg gtttttaaaa   3480
   taatatttaa catacgaatt attaattggc catgttcatt atttatctat gtttattaat   3540
   gggccaatgc aaaaaatcat tttttcaaag aaaaatttgt ccatgtaaag cttaaattat   3600
   aatattgctg ctttgtataa ctcttctatg tttattctat tcatttgttc ctttccctac   3660
   catattttac acatgtattt ataatctgta gtatttatta catttctgct tttttctagt   3720
   cattcaattt atcactgctg aattgcatca gatcatggat gcatttttat tatgaaaaaa   3780
   taaaatgact tttcaaatta aaaaaaaaaa aaaa                               3814
<210> 42
   <211> 734
   <212> DNA
   <213> Homo sapiens
<400> 42
   caggacaatg aaattcttca gttacattct ggtttatcgc cgatttctct tcgtggtttt     60
   cactgtgttg gttttactac ctctgcccat cgtcctccac accaaggaag cagaatgtgc    120
   ctacacactc tttgtggtcg ccacattttg gctcacagaa gcattgcctc tgtcggtaac    180
   agctttgcta cctagtttaa tgttacccat gtttgggatc atgccttcta agaaggtggc    240
   atctgcttat ttcaaggatt ttcacttact gctaattgga gttatctgtt tagcaacatc    300
   catagaaaaa tggaatttgc acaagagaat tgctctgaaa atggtgatga tggttggtgt    360
   aaatcctgca tggctgacgc tggggttcat gagcagcact gcctttttgt ctatgtggct    420
   cagcaacacc tcgacggctg ccatggtgat gcccattgcg gaggctgtag tgcagcagat    480
   catcaatgca gaagcagagg tcgaggccac tcagatgact tacttcaacg gatcaaccaa    540
   ccacggacta gaaattgatg aaagtgttaa tggacatgaa ataaatgaga ggaaagagaa    600
   aacaaaacca gttccaggat acaataatga tacagggaaa atttcaagca aggtggagtt    660
   ggaaaagact gtttaactac tgaaatgaag ctattctcct gactaaacat aactgaaaaa    720
   ccattcatta aatg                                                      734
<210> 43
   <211> 539
   <212> DNA
   <213> Homo sapiens
<400> 43
   gccactcaga tgacttactt caacggatca accaaccacg gactagaaat tgatgaaagt 60
   gttaatggac atgaaataaa tgagaggaaa gagaaaacaa aaccagttcc aggatacaat 120
   aatgatacag ggaaaatttc aagcaaggtg gagttggaaa agcactggaa acttgcagtt 180
   caagatggct ccccatctcc ctctgtccat tctgtatcgc agctagctgc tcaaggaaag 240
   gagaaagtgg aaggcatatg tacttagaaa ttattctatt actttcctgg atttaagagt 300
   attcagattt tctatttcaa catcaaacaa ttgcattttt aaaaagaaat ttatgtgttc 360
   catgtcaaat ttagtagtgt gtggttgttt ataatatttt cttatatcta cttaatttct 420
   atagtattta tagttatatg tctttatttc taacattttt cttgtgcttt taaagattat 480
   ttaaagatta tttttaaata atctttattt catttaaata aaatatttta tttaagtct 539
<210> 44
   <211> 556
   <212> DNA
   <213> Homo sapiens
<400> 44
   cacggactag aaattgatga aagtgttaat ggacatgaaa taaatgagag gaaagagaaa 60
   acaaaaccag ttccaggata caataatgat acagggaaaa tttcaagcaa ggtggagttg 120
   gaaaagaact caggcatgag aaccaaatat cgaacaaaga agggccacgt gacacgtaaa 180
   cttacgtgtt tgtgcattgc ctactcttct accattggtg gactgacaac aatcactggt 240
   acctccacca acttgatctt tgcagagtat ttcaatacat tccatccaca cagaagagga 300
   gatcgtacaa ggcatgtaca ccaggaggca gaaatttgag gcatatcttg gaactctgtc 360
   taccacatcc tgaacatcac acagtttcca ctcttgttgc cttcaatcct gagaatgcat 420
   ccaggagcca ttctgtttta tgtcaattac taattagatc atgtcacgtt actaacttac 480
   tacgttccaa ttagtcctta ttgcatttgt aataaaatcc gcatactttc ggactggcta 540
   caaggttata catgat 556
<210> 45
   <211> 595
   <212> PRT
   <213> Homo sapiens
<400> 45
   Met Lys Phe Phe Ser Tyr Ile Leu Val Tyr Arg Arg Phe Leu Phe Val
   1               5                   10                  15
   Val Phe Thr Val Leu Val Leu Leu Pro Leu Pro Ile Val Leu His Thr
               20                  25                  30
   Lys Glu Ala Glu Cys Ala Tyr Thr Leu Phe Val Val Ala Thr Phe Trp
           35                  40                  45
   Leu Thr Glu Ala Leu Pro Leu Ser Val Thr Ala Leu Leu Pro Ser Leu
       50                  55                  60
   Met Leu Pro Met Phe Gly Ile Met Pro Ser Lys Lys Val Ala Ser Ala
   65                  70                  75                  80
   Tyr Phe Lys Asp Phe His Leu Leu Leu Ile Gly Val Ile Cys Leu Ala
                   85                  90                  95
   Thr Ser Ile Glu Lys Trp Asn Leu His Lys Arg Ile Ala Leu Lys Met
               100                 105                 110
   Val Met Met Val Gly Val Asn Pro Ala Trp Leu Thr Leu Gly Phe Met
           115                 120                 125
   Ser Ser Thr Ala Phe Leu Ser Met Trp Leu Ser Asn Thr Ser Thr Ala
       130                 135                 140
   Ala Met Val Met Pro Ile Ala Glu Ala Val Val Gln Gln Ile Ile Asn
   145                 150                 155                 160
   Ala Glu Ala Glu Val Glu Ala Thr Gln Met Thr Tyr Phe Asn Gly Ser
                   165                 170                 175
   Thr Asn His Gly Leu Glu Ile Asp Glu Ser Val Asn Gly His Glu Ile
               180                 185                 190
   Asn Glu Arg Lys Glu Lys Thr Lys Pro Val Pro Gly Tyr Asn Asn Asp
           195                 200                 205
   Thr Gly Lys Ile Ser Ser Lys Val Glu Leu Glu Lys Asn Ser Gly Met
       210                 215                 220
   Arg Thr Lys Tyr Arg Thr Lys Lys Gly His Val Thr Arg Lys Leu Thr
   225                 230                 235                 240
   Cys Leu Cys Ile Ala Tyr Ser Ser Thr Ile Gly Gly Leu Thr Thr Ile
                   245                 250                 255
   Thr Gly Thr Ser Thr Asn Leu Ile Phe Ala Glu Tyr Phe Asn Thr Arg
               260                 265                 270
   Tyr Pro Asp Cys Arg Cys Leu Asn Phe Gly Ser Trp Phe Thr Phe Ser
           275                 280                 285
   Phe Pro Ala Ala Leu Ile Ile Leu Leu Leu Ser Trp Ile Trp Leu Gln
       290                 295                 300
   Trp Leu Phe Leu Gly Phe Asn Phe Lys Glu Met Phe Lys Cys Gly Lys
   305                 310                 315                 320
   Thr Lys Thr Val Gln Gln Lys Ala Cys Ala Glu Val Ile Lys Gln Glu
                   325                 330                 335
   Tyr Gln Lys Leu Gly Pro Ile Arg Tyr Gln Glu Ile Val Thr Leu Val
               340                 345                 350
   Leu Phe Ile Ile Met Ala Leu Leu Trp Phe Ser Arg Asp Pro Gly Phe
           355                 360                 365
   Val Pro Gly Trp Ser Ala Leu Phe Ser Glu Tyr Pro Gly Phe Ala Thr
       370                 375                 380
   Asp Ser Thr Val Ala Leu Leu Ile Gly Leu Leu Phe Phe Leu Ile Pro
   385                 390                 395                 400
   Ala Lys Thr Leu Thr Lys Thr Thr Pro Thr Gly Glu Ile Val Ala Phe
                   405                 410                 415
   Asp Tyr Ser Pro Leu Ile Thr Trp Lys Glu Phe Gln Ser Phe Met Pro
               420                 425                 430
   Trp Asp Ile Ala Ile Leu Val Gly Gly Gly Phe Ala Leu Ala Asp Gly
           435                 440                 445
   Cys Glu Glu Ser Gly Leu Ser Lys Trp Ile Gly Asn Lys Leu Ser Pro
       450                 455                 460
   Leu Gly Ser Leu Pro Ala Trp Leu Ile Ile Leu Ile Ser Ser Leu Met
   465                 470                 475                 480
   Val Thr Ser Leu Thr Glu Val Ala Ser Asn Pro Ala Thr Ile Thr Leu
                   485                 490                 495
   Phe Leu Pro Ile Leu Ser Pro Leu Ala Glu Ala Ile His Val Asn Pro
               500                 505                 510
   Leu Tyr Ile Leu Ile Pro Ser Thr Leu Cys Thr Ser Phe Ala Phe Leu
           515                 520                 525
   Leu Pro Val Ala Asn Pro Pro Asn Ala Ile Val Phe Ser Tyr Gly His
       530                 535                 540
   Leu Lys Val Ile Asp Met Val Lys Ala Gly Leu Gly Val Asn Ile Val
   545                 550                 555                 560
   Gly Val Ala Val Val Met Leu Gly Ile Cys Thr Trp Ile Val Pro Met
   565 570 575
   Phe Asp Leu Tyr Thr Tyr Pro Ser Trp Ala Pro Ala Met Ser Asn Glu
   580 585 590
   Thr Met Pro
   595
<210> 46
   <211> 224
   <212> PRT
   <213> Homo sapiens
<400> 46
   Arg Thr Met Lys Phe Phe Ser Tyr Ile Leu Val Tyr Arg Arg Phe Leu
   1               5                   10                  15
   Phe Val Val Phe Thr Val Leu Val Leu Leu Pro Leu Pro Ile Val Leu
               20                  25                  30
   His Thr Lys Glu Ala Glu Cys Ala Tyr Thr Leu Phe Val Val Ala Thr
           35                  40                  45
   Phe Trp Leu Thr Glu Ala Leu Pro Leu Ser Val Thr Ala Leu Leu Pro
       50                  55                  60
   Ser Leu Met Leu Pro Met Phe Gly Ile Met Pro Ser Lys Lys Val Ala
   65                  70                  75                  80
   Ser Ala Tyr Phe Lys Asp Phe His Leu Leu Leu Ile Gly Val Ile Cys
                   85                  90                  95
   Leu Ala Thr Ser Ile Glu Lys Trp Asn Leu His Lys Arg Ile Ala Leu
               100                 105                 110
   Lys Met Val Met Met Val Gly Val Asn Pro Ala Trp Leu Thr Leu Gly
           115                 120                 125
   Phe Met Ser Ser Thr Ala Phe Leu Ser Met Trp Leu Ser Asn Thr Ser
       130                 135                 140
   Thr Ala Ala Met Val Met Pro Ile Ala Glu Ala Val Val Gln Gln Ile
   145                 150                 155                 160
   Ile Asn Ala Glu Ala Glu Val Glu Ala Thr Gln Met Thr Tyr Phe Asn
                   165                 170                 175
   Gly Ser Thr Asn His Gly Leu Glu Ile Asp Glu Ser Val Asn Gly His
               180                 185                 190
   Glu Ile Asn Glu Arg Lys Glu Lys Thr Lys Pro Val Pro Gly Tyr Asn
           195                 200                 205
   Asn Asp Thr Gly Lys Ile Ser Ser Lys Val Glu Leu Glu Lys Thr Val
       210                 215                 220
<210> 47
   <211> 88
   <212> PRT
   <213> Homo sapiens
<400> 47
   Ala Thr Gln Met Thr Tyr Phe Asn Gly Ser Thr Asn His Gly Leu Glu
   1               5                   10                  15
   Ile Asp Glu Ser Val Asn Gly His Glu Ile Asn Glu Arg Lys Glu Lys
               20                  25                  30
   Thr Lys Pro Val Pro Gly Tyr Asn Asn Asp Thr Gly Lys Ile Ser Ser
           35                  40                  45
   Lys Val Glu Leu Glu Lys His Trp Lys Leu Ala Val Gln Asp Gly Ser
       50                  55                  60
   Pro Ser Pro Ser Val His Ser Val Ser Gln Leu Ala Ala Gln Gly Lys
   65                  70                  75                  80
   Glu Lys Val Glu Gly Ile Cys Thr
                   85
<210> 48
   <211> 112
   <212> PRT
   <213> Homo sapiens
<400> 48
   His Gly Leu Glu Ile Asp Glu Ser Val Asn Gly His Glu Ile Asn Glu
   1               5                   10                  15
   Arg Lys Glu Lys Thr Lys Pro Val Pro Gly Tyr Asn Asn Asp Thr Gly
               20                  25                  30
   Lys Ile Ser Ser Lys Val Glu Leu Glu Lys Asn Ser Gly Met Arg Thr
           35                  40                  45
   Lys Tyr Arg Thr Lys Lys Gly His Val Thr Arg Lys Leu Thr Cys Leu
       50                  55                  60
   Cys Ile Ala Tyr Ser Ser Thr Ile Gly Gly Leu Thr Thr Ile Thr Gly
   65                  70                  75                  80
   Thr Ser Thr Asn Leu Ile Phe Ala Glu Tyr Phe Asn Thr Phe His Pro
                   85                  90                  95
   His Arg Arg Gly Asp Arg Thr Arg His Val His Gln Glu Ala Glu Ile
               100                 105                 110
<210> 49
   <211> 21
   <212> DNA
   <213> Künstliche Sequenz
<220>
   <223> Beschreibung der künstlichen Sequenz: Oligonukleotid
<400> 49
   ccagctttaa ccatgtcaat g 21
<210> 50
   <211> 21
   <212> DNA
   <213> Künstliche Sequenz
<220>
   <223> Beschreibung der künstlichen Sequenz: Oligonukleotid
<400> 50
   cagatggttg tgaggagtct g 21
<210> 51
   <211> 3311
   <212> DNA
   <213> Homo sapiens
<400> 51
   tgctaatgct tttggtacaa atggatgtgg aatataattg aatattttct tgtttaaggg      60
   gagcatgaag aggtgttgag gttatgtcaa gcatctggca cagctgaagg cagatggaaa     120
   tatttacaag tacgcaattt gagactaaga tattgttatc attctcctat tgaagacaag     180
   agcaatagta aaacacatca ggtcaggggg ttaaagacct gtgataaacc acttccgata     240
   agttggaaac gtgtgtctat attttcatat ctgtatatat ataatggtaa agaaagacac     300
   cttcgtaacc cgcattttcc aaagagagga atcacaggga gatgtacagc aatggggcca     360
   tttaagagtt ctgtgttcat cttgattctt caccttctag aaggggccct gagtaattca     420
   ctcattcagc tgaacaacaa tggctatgaa ggcattgtcg ttgcaatcga ccccaatgtg     480
   ccagaagatg aaacactcat tcaacaaata aaggacatgg tgacccaggc atctctgtat     540
   ctgtttgaag ctacaggaaa gcgattttat ttcaaaaatg ttgccatttt gattcctgaa     600
   acatggaaga caaaggctga ctatgtgaga ccaaaacttg agacctacaa aaatgctgat 660
   gttctggttg ctgagtctac tcctccaggt aatgatgaac cctacactga gcagatgggc 720
   aactgtggag agaagggtga aaggatccac ctcactcctg atttcattgc aggaaaaaag 780
   ttagctgaat atggaccaca aggtaaggca tttgtccatg agtgggctca tctacgatgg 840
   ggagtatttg acgagtacaa taatgatgag aaattctact tatccaatgg aagaatacaa 900
   gcagtaagat gttcagcagg tattactggt acaaatgtag taaagaagtg tcagggaggc 960
   agctgttaca ccaaaagatg cacattcaat aaagttacag gactctatga aaaaggatgt 1020
   gagtttgttc tccaatcccg ccagacggag aaggcttcta taatgtttgc acaacatgtt 1080
   gattctatag ttgaattctg tacagaacaa aaccacaaca aagaagctcc aaacaagcaa 1140
   aatcaaaaat gcaatctccg aagcacatgg gaagtgatcc gtgattctga ggactttaag 1200
   aaaaccactc ctatgacaac acagccacca aatcccacct tctcattgct gcagattgga 1260
   caaagaattg tgtgtttagt ccttgacaaa tctggaagca tggcgactgg taaccgcctc 1320
   aatcgactga atcaagcagg ccagcttttc ctgctgcaga cagttgagct ggggtcctgg 1380
   gttgggatgg tgacatttga cagtgctgcc catgtacaaa gtgaactcat acagataaac 1440
   agtggcagtg acagggacac actcgccaaa agattacctg cagcagcttc aggagggacg 1500
   tccatctgca gcgggcttcg atcggcattt actgtgatta ggaagaaata tccaactgat 1560
   ggatctgaaa ttgtgctgct gacggatggg gaagacaaca ctataagtgg gtgctttaac 1620
   gaggtcaaac aaagtggtgc catcatccac acagtcgctt tggggccctc tgcagctcaa 1680
   gaactagagg agctgtccaa aatgacagga ggtttacaga catatgcttc agatcaagtt 1740
   cagaacaatg gcctcattga tgcttttggg gccctttcat caggaaatgg agctgtctct 1800
   cagcgctcca tccagcttga gagtaaggga ttaaccctcc agaacagcca gtggatgaat 1860
   ggcacagtga tcgtggacag caccgtggga aaggacactt tgtttcttat cacctggaca 1920
   acgcagcctc cccaaatcct tctctgggat cccagtggac agaagcaagg tggctttgta 1980
   gtggacaaaa acaccaaaat ggcctacctc caaatcccag gcattgctaa ggttggcact 2040
   tggaaataca gtctgcaagc aagctcacaa accttgaccc tgactgtcac gtcccgtgcg 2100
   tccaatgcta ccctgcctcc aattacagtg acttccaaaa cgaacaagga caccagcaaa 2160
   ttccccagcc ctctggtagt ttatgcaaat attcgccaag gagcctcccc aattctcagg 2220
   gccagtgtca cagccctgat tgaatcagtg aatggaaaaa cagttacctt ggaactactg 2280
   gataatggag caggtgctga tgctactaag gatgacggtg tctactcaag gtatttcaca 2340
   acttatgaca cgaatggtag atacagtgta aaagtgcggg ctctgggagg agttaacgca 2400
   gccagacgga gagtgatacc ccagcagagt ggagcactgt acatacctgg ctggattgag 2460
   aatgatgaaa tacaatggaa tccaccaaga cctgaaatta ataaggatga tgttcaacac   2520
   aagcaagtgt gtttcagcag aacatcctcg ggaggctcat ttgtggcttc tgatgtccca   2580
   aatgctccca tacctgatct cttcccacct ggccaaatca ccgacctgaa ggcggaaatt   2640
   cacgggggca gtctcattaa tctgacttgg acagctcctg gggatgatta tgaccatgga   2700
   acagctcaca agtatatcat tcgaataagt acaagtattc ttgatctcag agacaagttc   2760
   aatgaatctc ttcaagtgaa tactactgct ctcatcccaa aggaagccaa ctctgaggaa   2820
   gtctttttgt ttaaaccaga aaacattact tttgaaaatg gcacagatct tttcattgct   2880
   attcaggctg ttgataaggt cgatctgaaa tcagaaatat ccaacattgc acgagtatct   2940
   ttgtttattc ctccacagac tccgccagag acacctagtc ctgatgaaac gtctgctcct   3000
   tgtcctaata ttcatatcaa cagcaccatt cctggcattc acattttaaa aattatgtgg   3060
   aagtggatag gagaactgca gctgtcaata gcctagggct gaatttttgt cagataaata   3120
   aaataaatca ttcatccttt ttttgattat aaaattttct aaaatgtatt ttagacttcc   3180
   tgtagggggc gatatactaa atgtatatag tacatttata ctaaatgtat tcctgtaggg   3240
   ggcgatatac taaatgtatt ttagacttcc tgtagggggc gataaaataa aatgctaaac   3300
   aactgggtaa a                                                        3311
<210> 52
   <211> 3067
   <212> DNA
   <213> Homo sapiens
<400> 52
   aattaaatta tgagaattaa aaagacaaca ttgagcagag atgaaaaagg aagggaggaa     60
   aaggtggaaa agaaaagaag acaagaagcg agtagtggtc tctaacttgc tctttgaagg    120
   atggtctcac aaagagaacc ccaacagaca tcatcgtggg aatcaaatca agaccagcaa    180
   gtacaccgtg ttgtccttcg tccccaaaaa catttttgag cagctacacc ggtttgccaa    240
   tctctatttt gtgggcattg cggttctgaa ttttatccct gtggtcaatg ctttccagcc    300
   tgaggtgagc atgataccaa tctgtgttat cctggcagtc actgccatca aggacgcttg    360
   ggaagacctc cggaggtaca aatcggataa agtcatcaat aaccgagagt gcctcatcta    420
   cagcagaaaa gagcagacct atgtgcagaa gtgctggaag gatgtgcgtg tgggagactt    480
   catccaaatg aaatgcaatg agattgtccc agcagacata ctcctccttt tttcctctga    540
   ccccaatggg atatgccatc tggaaactgc cagcttggat ggagagacaa acctcaagca    600
   aagacgtgtc gtgaagggct tctcacagca ggaggtacag ttcgaaccag agcttttcca    660
   caataccatc gtgtgtgaga aacccaacaa ccacctcaac aaatttaagg gttatatgga    720
   gcatcctgac cagaccagga ctggctttgg ctgtgagagt cttctgcttc gaggctgcac    780
   catcagaaac accgagatgg ctgttggcat tgtcatctat gcaggccatg agacgaaagc 840
   catgctgaac aacagtggcc cccggtacaa acgcagcaag attgagcggc gcatgaatat 900
   agacatcttc ttctgcattg ggatcctcat cctcatgtgc cttattggag ctgtaggtca 960
   cagcatctgg aatgggacct ttgaagaaca ccctcccttc gatgtgccag atgccaatgg 1020
   cagcttcctt cccagtgccc ttgggggctt ctacatgttc ctcacaatga tcatcctgct 1080
   ccaggtgctg atccccatct ctttgtatgt ctccattgag ctggtgaagc tcgggcaagt 1140
   gttcttcttg agcaatgacc ttgacctgta tgatgaagag accgatttat ccattcaatg 1200
   tcgagccctc aacatcgcag aggacttggg ccagatccag tacatcttct ccgataagac 1260
   ggggaccctg acagagaaca agatggtgtt ccgacgttgc accatcatgg gcagcgagta 1320
   ttctcaccaa gaaaatggta tagaagctcc caagggctcc atccctcttt ctaaaaggaa 1380
   ataccctgct ctcctaagaa acgaggagat aaaagacatt ctcctggctc tcttagaggc 1440
   tgtgtggcat ttccacaagt tgcttcctgt atccctgtgg tcttccttgt cacagatcag 1500
   ggctgttcca attacttgta aactttcatt tgtttacaaa ggttagaagt tatcccatat 1560
   gtggttcccc ttcagctgat ctttgtctgg tgccagacaa agcactttat gagacgagtt 1620
   ttttatctgt cagcaatgga ttggagacat ttcccaattg tgtgccagtc acacaaccaa 1680
   ggcttaggaa tttctcaggc caccttacct gacatgtcag ggcaggtctg tgtctaggtg 1740
   catggtcaga tttaatacat ccagaagatg tcttctattc taacagatct cttagcttgt 1800
   cactgaggca aagttttgat ttaggagata gggctataaa atgcctggac tgttaccttg 1860
   catggactga atatgactca taaaactgat ctgattcctt cagccatcat ctgcccaact 1920
   tggttcccct ccccaccccc ccacaacaca cacacacact ttctaagaaa agaaaagaaa 1980
   ttcttttttt tcaatacttt aagttctggg atacatgtgc agaatgtgca ggtttgttac 2040
   ataggtatac atgtgtcatg gtggtttgca gcacccacca acccatcatc taccttaggt 2100
   atttctccta atgctatccc tcccctagcc cccaaccccc cgatgggctc cagtgtgtga 2160
   tgttcccctc catgtccatg tgttctcatt gttcaattcc cacttatgag tgagaacatg 2220
   cagtatttgg ttttctgttc ttgtgttagt ttgctgatgg tttcctgttc atccgtgtcc 2280
   ctgcaaagga catgaactca tcctttttta tggctgcata atattccatg gtgtatatgt 2340
   gccacatttt ctttatccag tctatcgctg atgggcactg gggttggttc caagtctttg 2400
   ctattgtgaa cagtgctgca ataaacttac atgtgcatgt gtctttagta gaatgattta 2460
   taatcctttg ggtatatacc cagtaatggg attgctggtc aaatggtatt tctggttcta 2520
   gatccttgag gaatctttgt cttccacaat ggttgaacta atttgtactc ccaccaacag 2580
   tgtaaaagta ttcctgtttc tctacatcct cttcagcatc tgttgtgtcc tgacatttta 2640
   atgatcacta ttctcactgg cgtgagatgt tatctcattg tggttttgat ttgcatttct 2700
   ctaatgacca gtaatgatga gctttttttc atatgtttgt tggctgcata aatgtcttct   2760
   tttgagaagt gtctgttcat atccttcacc cattttttga agaaaacaaa ctcttaagag   2820
   agcagtattc attcttttga gtgtgaggga tggagaaaga gaaagatgga gagagtatta   2880
   taagcagctg tatccccttt gccatggtga tagcagacca ttcacatggg agcttctggt   2940
   ctctttgtaa taataataag agccacatta ccagtactta gagtatgcta gttattttaa   3000
   cacattgtat cattaaatct tcaaaacatc cctatgagtt agaaacctaa aaaaaaaaaa   3060
   aaaaaaa                                                             3067
<210> 53
   <211> 2778
   <212> DNA
   <213> Homo sapiens
<400> 53
   ctcattttga tgtctagaat caggggatcc aggatcatca ccaaggtcat tttcccaggt      60
   atggaggggt ctttctgctt ctttcttgtc atgcacagct gctgaggaag gggctgggag     120
   taaagacagt gaaatgggga ggaggagtcc attcaaaccg agaaacaaag tgtttggttt     180
   ttcttacccc tggtgtagaa gctaccaacc ttttccaaga aagagggcct ggcccccttc     240
   tcgggtctgg ctgggtgcct gctgtgcctc tctggcctcc cctccgaagg gcaccattcc     300
   ctcgggtgag tactaccggc ctgcaccgtc ttccagtggg gacagcctga gaagagagtc     360
   tggggcctta cttcagtacc ttccttcact ggcctcaccc tgtgcaaatc atgccacacg     420
   ctgcagcctc cttttcccta tctataaaat aaaaatgacc ctgctctatc tcactgggct     480
   ggcaagaaca cactgttgtt gccttgcaga cagatgtgct gaggctgtag aaagtgcttt     540
   ttatttggtt gggagcttgt gcataaatgc gagaggggct gcacatctga cggactagag     600
   gtgactcatg gctgaaccgg aacaggacat cggggagaag ccagcagcca tgctgaactc     660
   tccacagggc cctgtgaaaa gctcttcacc tcctctgccc tctggatcta gtgaagccta     720
   ttcatccttc agatgtcagc tcaaataatc aaccttcatg gaggcctccc ttgaccccta     780
   acatgctttc aaagtactgt gtatttcaca ttcatcatgc cccgacaact gtgatttccc     840
   atttattaat atctgtctct tctgctggcc tgcaaactcc aggagcacag agacatcttt     900
   gggatttttg aacatgattt ccccagggct tagcccagtg cctggtgcaa agcaggcttt     960
   caacatgttc agtggatatt gtaagaaaga aagaaataca caaaaggcct ggcatatgca    1020
   aagcactcta aatattcact cctttccctt ccctctgggt gagaaaattt ctccttataa    1080
   agacaccctc ctaactgtat ctctgctaga gaactgaaga cataaagcac tctgtgccaa    1140
   aaatatttaa gtaaaaactt gagctaagca cagagattat aaatatttct tccccagatt    1200
   acgcaccatt taaaaatact gtctcagctc cttttcatga tttgggtggt gattaaagaa    1260
   aattactctt caagactgaa agtcattact gcccttttcc tgacttgcct tttcccttga 1320
   gaaggggagg ataagctgca gggcaggaag tggaagtggg gcatccttgt cctttgtctg 1380
   gcagacagcc aactggtcag gtactgctcc ttctcaactc tttcctgatt cccaggtgaa 1440
   tataaacaag aaggcacaaa tccacacttg ccaacaacgg acccaagtga taacaagaaa 1500
   cccagtgaca cctgtctagg tgaagactca gcccctatgt gaccaggttg caaagccaaa 1560
   ctgaccatct gctttccatt tggactttta gttcatactg tatcttctca ggacagttaa 1620
   gttggaatac aatgccactg tcctgaaaga tggtagaatt atcctatttc tggaggagtg 1680
   ggggtggtgg gtaggaatct caagagcgat ttgctcctct gcacaatagc ttctttaagg 1740
   acaccagggc ccccagggct atacatttcc ctgaagcttt ccagataagc aacaaggtat 1800
   gagcacctgc tatgtattgc ccaagggtga tgtgtttaaa tatccattgc atattttaaa 1860
   tccttggctg gcttaaagct gcaagctttc tgtcttcagt ggatataatg ggggcataca 1920
   tcccagagct tgcccaacac tccaagaaaa gaaccctcag ctaatgcaaa gtgtgtatgt 1980
   gcccatgaaa gctccatgtc tacttaacat tcagttttta ggattattta tgctgtaata 2040
   atagatatga aaatctctga caggtatttt gtttccttta caaactgtat ttgaatttat 2100
   gggtgattta gagcttgtgt ttaaagtcag aattcagaac cccaaagaaa atgacttcat 2160
   tgaaattgaa ctgaagagac aagaactgag ttaccaaaac ctactaaacg tgagttgctg 2220
   tgaactgggg attaaaccag aacgagtgga gaagatcaga aagctaccaa acacactgct 2280
   cagaaaggac aaagacattc gaagactgcg ggactttcag gaagtggaac tcattttaat 2340
   gaaaaatgga agctccagat tgacagaata tgtgccatct ctgacagaaa ggccctgcta 2400
   tgatagcaaa gctgcaaaaa tgacttatta aatactccca ggaatggccg cgcatggtgg 2460
   ctcaccccct gtaatcccag cactttggga agccaaggtg ggcggatcac ctgaggtcag 2520
   gagttctaga ccagcctggc caacatatag tgaaacccag tctctactaa aaaaaataca 2580
   aaaattagct aggtgtggtg gcgcacacct gtagtagtcc cagctacatg ggaagctgag 2640
   gcaggagaat cacctgaacc caggaggcag aggttgcagt gagctgagat tgcgccactg 2700
   cactccagcc tggcgacaga gcaagactct gtctctcaaa ataaataaat aaataaataa 2760
   ataaataaat aaataatc 2778
<210> 54
   <211> 1646
   <212> DNA
   <213> Homo sapiens
<400> 54
   gcccgggaga ggagaggagc gggccgagga ctccagcgtg cccaggtctg gcatcctgca 60
   cttgctgccc tctgacacct gggaagatgg ccggcccgtg gaccttcacc cttctctgtg 120
   gtttgctggc agccaccttg atccaagcca ccctcagtcc cactgcagtt ctcatcctcg 180
   gcccaaaagt catcaaagaa aagctgacac aggagctgaa ggaccacaac gccaccagca 240
   tcctgcagca gctgccgctg ctcagtgcca tgcgggaaaa gccagccgga ggcatccctg 300
   tgctgggcag cctggtgaac accgtcctga agcacatcat ctggctgaag gtcatcacag 360
   ctaacatcct ccagctgcag gtgaagccct cggccaatga ccaggagctg ctagtcaaga 420
   tccccctgga catggtggct ggattcaaca cgcccctggt caagaccatc gtggagttcc 480
   acatgacgac tgaggcccaa gccaccatcc gcatggacac cagtgcaagt ggccccaccc 540
   gcctggtcct cagtgactgt gccaccagcc atgggagcct gcgcatccaa ctgctgcata 600
   agctctcctt cctggtgaac gccttagcta agcaggtcat gaacctccta gtgccatccc 660
   tgcccaatct agtgaaaaac cagctgtgtc ccgtgatcga ggcttccttc aatggcatgt 720
   atgcagacct cctgcagctg gtgaaggtgc ccatttccct cagcattgac cgtctggagt 780
   ttgaccttct gtatcctgcc atcaagggtg acaccattca gctctacctg ggggccaagt 840
   tgttggactc acagggaaag gtgaccaagt ggttcaataa ctctgcagct tccctgacaa 900
   tgcccaccct ggacaacatc ccgttcagcc tcatcgtgag tcaggacgtg gtgaaagctg 960
   cagtggctgc tgtgctctct ccagaagaat tcatggtcct gttggactct gtgcttcctg 1020
   agagtgccca tcggctgaag tcaagcatcg ggctgatcaa tgaaaaggct gcagataagc 1080
   tgggatctac ccagatcgtg aagatcctaa ctcaggacac tcccgagttt tttatagacc 1140
   aaggccatgc caaggtggcc caactgatcg tgctggaagt gtttccctcc agtgaagccc 1200
   tccgcccttt gttcaccctg ggcatcgaag ccagctcgga agctcagttt tacaccaaag 1260
   gtgaccaact tatactcaac ttgaataaca tcagctctga tcggatccag ctgatgaact 1320
   ctgggattgg ctggttccaa cctgatgttc tgaaaaacat catcactgag atcatccact 1380
   ccatcctgct gccgaaccag aatggcaaat taagatctgg ggtcccagtg tcattggtga 1440
   aggccttggg attcgaggca gctgagtcct cactgaccaa ggatgccctt gtgcttactc 1500
   cagcctcctt gtggaaaccc agctctcctg tctcccagtg aagacttgga tggcagccat 1560
   cagggaaggc tgggtcccag ctgggagtat gggtgtgagc tctatagacc atccctctct 1620
   gcaatcaata aacacttgcc tgtgat 1646
<210> 55
   <211> 1049
   <212> DNA
   <213> Homo sapiens
<400> 55
   ggagtggggg agagagagga gaccaggaca gctgctgaga cctctaagaa gtccagatac      60
   taagagcaaa gatgtttcaa actgggggcc tcattgtctt ctacgggctg ttagcccaga     120
   ccatggccca gtttggaggc ctgcccgtgc cctggaccca gaccctgccc ttgaatgtga     180
   atccagccct gcccttgagt cccacaggtc ttgcaggaag cttgacaaat gccctcagca     240
   atggcctgct gtctgggggc ctgttgggca ttctggaaaa ccttccgctc ctggacatcc     300
   tgaagcctgg aggaggtact tctggtggcc tccttggggg actgcttgga aaagtgacgt     360
   cagtgattcc tggcctgaac aacatcattg acataaaggt cactgacccc cagctgctgg     420
   aacttggcct tgtgcagagc cctgatggcc accgtctcta tgtcaccatc cctctcggca     480
   taaagctcca agtgaatacg cccctggtcg gtgcaagtct gttgaggctg gctgtgaagc     540
   tggacatcac tgcagaaatc ttagctgtga gagataagca ggagaggatc cacctggtcc     600
   ttggtgactg cacccattcc cctggaagcc tgcaaatttc tctgcttgat ggacttggcc     660
   ccctccccat tcaaggtctt ctggacagcc tcacagggat cttgaataaa gtcctgcctg     720
   agttggttca gggcaacgtg tgccctctgg tcaatgaggt tctcagaggc ttggacatca     780
   ccctggtgca tgacattgtt aacatgctga tccacggact acagtttgtc atcaaggtct     840
   aagccttcca ggaaggggct ggcctctgct gagctgcttc ccagtgctca cagatggctg     900
   gcccatgtgc tggaagatga cacagttgcc ttctctccga ggaacctgcc ccctctcctt     960
   tcccaccagg cgtgtgtaac atcccatgtg cctcacctaa taaaatggct cttcttctgc    1020
   aaaaaaaaaa aaaaaaaaaa aaaaaaaaa                                      1049
<210> 56
   <211> 4815
   <212> DNA
   <213> Homo sapiens
<400> 56
   gagcagagcc ctttcacaca cctcaggaac acctttcggc tgcccgctcc ccagacacac     60
   ctgcagccct gcccagccgg ctttgctcac ccactgcttg taaatgcccc agatatgagc    120
   cagcccaggc cccgctacgt ggtagacaga gccgcatact cccttaccct cttcgacgat    180
   gagtttgaga agaaggaccg gacataccca gtgggagaga aacttcgcaa tgccttcaga    240
   tgttcctcag ccaagatcaa agctgtggtg tttgggctgc tgcctgtgct ctcctggctc    300
   cccaagtaca agattaaaga ctacatcatt cctgacctgc tcggtggact cagcggggga    360
   tccatccagg tcccacaagg catggcattt gctctgctgg ccaaccttcc tgcagtcaat    420
   ggcctctact cctccttctt ccccctcctg acctacttct tcctgggggg tgttcaccag    480
   atggtgccag gtacctttgc cgttatcagc atcctggtgg gtaacatctg tctgcagctg    540
   gccccagagt cgaaattcca ggtcttcaac aatgccacca atgagagcta tgtggacaca    600
   gcagccatgg aggctgagag gctgcacgtg tcagctacgc tagcctgcct caccgccatc    660
   atccagatgg gtctgggctt catgcagttt ggctttgtgg ccatctacct ctccgagtcc    720
   ttcatccggg gcttcatgac ggccgccggc ctgcagatcc tgatttcggt gctcaagtac 780
   atcttcggac tgaccatccc ctcctacaca ggcccagggt ccatcgtctt taccttcatt 840
   gacatttgca aaaacctccc ccacaccaac atcgcctcgc tcatcttcgc tctcatcagc 900
   ggtgccttcc tggtgctggt gaaggagctc aatgctcgct acatgcacaa gattcgcttc 960
   cccatcccta cagagatgat tgtggtggtg gtggcaacag ctatctccgg gggctgtaag 1020
   atgcccaaaa agtatcacat gcagatcgtg ggagaaatcc aacgcgggtt ccccaccccg 1080
   gtgtcgcctg tggtctcaca gtggaaggac atgataggca cagccttctc cctagccatc 1140
   gtgagctacg tcatcaacct ggctatgggc cggaccctgg ccaacaagca cggctacgac 1200
   gtggattcga accaggagat gatcgctctc ggctgcagca acttctttgg ctccttcttt 1260
   aaaattcatg tcatttgctg tgcgctttct gtcactctgg ctgtggatgg agctggagga 1320
   aaatcccagg tggccagcct gtgtgtgtct ctggtggtga tgatcaccat gctggtcctg 1380
   gggatctatc tgtatcctct ccctaagtct gtgctaggag ccctgatcgc tgtcaatctc 1440
   aagaactccc tcaagcaact caccgacccc tactacctgt ggaggaagag caagctggac 1500
   tgttgcatct gggtagtgag cttcctctcc tccttcttcc tcagcctgcc ctatggtgtg 1560
   gcagtgggtg tcgccttctc cgtcctggtc gtggtcttcc agactcagtt tcgaaatggc 1620
   tatgcactgg cccaggtcat ggacactgac atttatgtga atcccaagac ctataatagg 1680
   gcccaggata tccaggggat taaaatcatc acgtactgct cccctctcta ctttgccaac 1740
   tcagagatct tcaggcaaaa ggtcatcgcc aagacaggca tggaccccca gaaagtatta 1800
   ctagccaagc aaaaatacct caagaagcag gagaagcgga gaatgaggcc cacacaacag 1860
   aggaggtctc tattcatgaa aaccaagact gtctccctgc aggagctgca gcaggacttt 1920
   gagaatgcgc cccccaccga ccccaacaac aaccagaccc cggctaacgg caccagcgtg 1980
   tcctatatca ccttcagccc tgacagctcc tcacctgccc agagtgagcc accagcctcc 2040
   gctgaggccc ccggcgagcc cagtgacatg ctggccagcg tcccaccctt cgtcaccttc 2100
   cacaccctca tcctggacat gagtggagtc agcttcgtgg acttgatggg catcaaggcc 2160
   ctggccaagc tgagctccac ctatgggaag atcggcgtga aggtcttctt ggtgaacatc 2220
   catgcccagg tgtacaatga cattagccat ggaggcgtct ttgaggatgg gagtctagaa 2280
   tgcaagcacg tctttcccag catacatgac gcagtcctct ttgcccaggc aaatgctaga 2340
   gacgtgaccc caggacacaa cttccaaggg gctccagggg atgctgagct ctccttgtac 2400
   gactcagagg aggacattcg cagctactgg gacttagagc aggagatgtt cgggagcatg 2460
   tttcacgcag agaccctgac cgccctgtga gggctcagcc agtcctcatg ctgcctacag 2520
   agtgcctggc acttgggact tccataaagg atgagcctgg ggtcacaggg ggtgtcgggc 2580
   ggaggaaagt gcatccccca gagcttgggt tcctctctcc tctccccctc tctcctccct 2640
   tccttccctc cccgcatctc cagagagagc ctctcagcag caggggggtg ctacccttac 2700
   gggagtgaga gtctggtgag cccactcttc acccgtcagg ccctggccgc aatggacaag 2760
   cctcctgctc actccacccc acccacatct gccctgtcct tggcagctga aggacacctt 2820
   gacttccagc ttttacgagt gagccaaaaa cagaaggaca agtacaactg tgctggcctg 2880
   ctgtacaagc ttcaaaaagt gtcccagagc ccgcacggct cggtgtcaga tggtgtcagg 2940
   ctgtcacgga catagggata aacttggtta ggactctggc ttgccttccc cagctgcctc 3000
   aactctgtct ctggcagctc tgcacccagg gaccatgtgc tctccacacc caggagtcta 3060
   ggccttggta actatgcgcc ccccctccat catccccaag gctgcccaaa ccaccactgc 3120
   tgtcagcaag cacatcagac tctagcctgg acagtggcca ggaccgtcga gaccaccaga 3180
   gctacctccc cggggacagc ccactaaggt tctgcctcag cctcctgaaa catcactgcc 3240
   ctcagaggct gctcccttcc cctggaggct ggctagaaac cccaaagagg gggatgggta 3300
   gctggcagaa tcatctggca tcctagtaat agataccagt tattctgcac aaaacttttg 3360
   ggaattcctc tttgcaccca gagactcaga ggggaagagg gtgctagtac caacacaggg 3420
   aaaacggatg ggacctgggc ccagacagtc ccccttgacc ccagggccca tcagggaaat 3480
   gcctcccttt ggtaaatctg ccttatcctt ctttacctgg caaagagcca atcatgttaa 3540
   ctcttcctta tcagcctgtg gcccagagac acaatggggt ccttctgtag gcaaaggtgg 3600
   aagtcctcca gggatccgct acatccccta actgcatgca gatgtggaaa ggggctgatc 3660
   cagattgggt cttcctgcac aggaagactc tttaacaccc ttaggacctc aggccatctt 3720
   ctcctatgaa gatgaaaata ggggttaagt tttccatatg tacaaggagg tattgagagg 3780
   aaccctactg ttgacttgaa aataaatagg ttccatgtgt aagtgttttg taaaatttca 3840
   gtggaaatgc acagaaaatc ttctggcctc tcatcactgc ttttctcaag cttcttcagc 3900
   ttaacaaccc cttccctaac aggttgggct ggcccagcct aggaaaacat ccccatttct 3960
   aacttcagcc agacctgcgt tgtgtgtctg tgtgttgagt gagctggtca gctaacaagt 4020
   cttcttagag ttaaaggagg gggtgctggc caagagccaa cacattcttg gcccaggagc 4080
   attgcttttc tgtgaattca ttatgccatc tggctgccaa tggaactcaa aacttggaag 4140
   gcgaaggaca atgttatctg ggattcaccg tgcccagcac ccgaagtgcc aaattccagg 4200
   aggacaagag ccttagccaa tgacaactca ctctccccta ctccacctcc ttccaagtcc 4260
   agctcaggcc caggaggtgg gagaaggtca cagagcctca ggaatttcca agtcagagtc 4320
   ccctttgaac caagtatcta gatcccctga ggacttgatg aagtgatcct taacccccaa 4380
   gtaatcatta acccccagac cagcctcaga actgaaggag attgttgacc cagtgacctg 4440
   gagttgaggc tcagggagag atctgccaca tgtctgaggg ttgcagagcc cgctgtggag 4500
   gtaagattgg aaacacatga ggcagaggga agacattgaa gaaaacatct ctgctggaat 4560
   atttggaaaa gaacactctt ctggacctgg ttgaagcagg aaagatggag gcaaagtagt 4620
   gaaataatcc agaatttcaa tgcttttgaa tgttcttagt gatactgacc tgtgataata 4680
   taattcccag ggaggactgg gaaccttatc tcttgagata tttgcataat ttatttaatt 4740
   taagcctcat tctccttttg ttcattttgg taataaactg gatttgaatt gtgaacaaaa 4800
   aaaaaaaaaa aaaaa 4815
<210> 57
   <211> 2572
   <212> DNA
   <213> Homo sapiens
<400> 57
   aatgctctaa gacctctcag cacgggcgga agaaactccc ggagagctca cccaaaaaac 60
   aaggagatcc catctagatt tcttcttgct tttgactcac agctggaagt tagaaaagcc 120
   tcgatttcat ctttggagag gccaaatggt cttagcctca gtctctgtct ctaaatattc 180
   caccataaaa cagctgagtt atttatgaat tagaggctat agctcacatt ttcaatcctc 240
   tatttctttt tttaaatata actttctact ctgatgagag aatgtggttt taatctctct 300
   ctcacatttt gatgatttag acagactccc cctcttcctc ctagtcaata aacccattga 360
   tgatctattt cccagcttat ccccaagaaa acttttgaaa ggaaagagta gacccaaaga 420
   tgttattttc tgctgtttga attttgtctc cccaccccca acttggctag taataaacac 480
   ttactgaaga agaagcaata agagaaagat atttgtaatc tctccagccc atgatctcgg 540
   ttttcttaca ctgtgatctt aaaagttacc aaaccaaagt cattttcagt ttgaggcaac 600
   caaacctttc tactgctgtt gacatcttct tattacagca acaccattct aggagtttcc 660
   tgagctctcc actggagtcc tctttctgtc gcgggtcaga aattgtccct agatgaatga 720
   gaaaattatt ttttttaatt taagtcctaa atatagttaa aataaataat gttttagtaa 780
   aatgatacac tatctctgtg aaatagcctc acccctacat gtggatagaa ggaaatgaaa 840
   aaataattgc tttgacattg tctatatggt actttgtaaa gtcatgctta agtacaaatt 900
   ccatgaaaag ctcactgatc ctaattcttt ccctttgagg tctctatggc tctgattgta 960
   catgatagta agtgtaagcc atgtaaaaag taaataatgt ctgggcacag tggctcacgc 1020
   ctgtaatcct agcactttgg gaggctgagg aggaaggatc acttgagccc agaagttcga 1080
   gactagcctg ggcaacatgg agaagccctg tctctacaaa atacagagag aaaaaatcag 1140
   ccagtcatgg tggcatacac ctgtagtccc agcattccgg gaggctgagg tgggaggatc 1200
   acttgagccc agggaggttg gggctgcagt gagccatgat cacaccactg cactccagcc 1260
   aggtgacata gcgagatcct gtctaaaaaa ataaaaaata aataatggaa cacagcaagt 1320
   cctaggaagt aggttaaaac taattcttta aaaaaaaaaa aaagttgagc ctgaattaaa 1380
   tgtaatgttt ccaagtgaca ggtatccaca tttgcatggt tacaagccac tgccagttgg 1440
   cagtagcact ttcctggcac tgtggtcggt tttgttttgt tttgctttgt ttagagacgg 1500
   ggtctcactt tccaggctgg cctcaaactc ctgcactcaa gcaattcttc taccctggcc 1560
   tcccaagtag ctggaattac aggtgtgcgc catcacaact agctggtggt cagttttgtt 1620
   actctgagag ctgttcactt ctctgaattc acctagagtg gttggaccat cagatgtttg 1680
   ggcaaaactg aaagctcttt gcaaccacac accttccctg agcttacatc actgcccttt 1740
   tgagcagaaa gtctaaattc cttccaagac agtagaattc catcccagta ccaaagccag 1800
   ataggccccc taggaaactg aggtaagagc agtctctaaa aactacccac agcagcattg 1860
   gtgcagggga acttggccat taggttatta tttgagagga aagtcctcac atcaatagta 1920
   catatgaaag tgacctccaa ggggattggt gaatactcat aaggatcttc aggctgaaca 1980
   gactatgtct ggggaaagaa cggattatgc cccattaaat aacaagttgt gttcaagagt 2040
   cagagcagtg agctcagagg cccttctcac tgagacagca acatttaaac caaaccagag 2100
   gaagtatttg tggaactcac tgcctcagtt tgggtaaagg atgagcagac aagtcaacta 2160
   aagaaaaaag aaaagcaagg aggagggttg agcaatctag agcatggagt ttgttaagtg 2220
   ctctctggat ttgagttgaa gagcatccat ttgagttgaa ggccacaggg cacaatgagc 2280
   tctcccttct accaccagaa agtccctggt caggtctcag gtagtgcggt gtggctcagc   2340
   tgggttttta attagcgcat tctctatcca acatttaatt gtttgaaagc ctccatatag   2400
   ttagattgtg ctttgtaatt ttgttgttgt tgctctatct tattgtatat gcattgagta 2460
   ttaacctgaa tgttttgtta cttaaatatt aaaaacactg ttatcctaca aaaaaaccct 2520
   caaaggctga aaataaagaa ggaagatgga gacaccctct gggggtcctc tc 2572
<210> 58
   <211> 1324
   <212> DNA
   <213> Homo sapiens
<400> 58
   ctttgcagtg gatgcccttg gcagggtgag cccacaagga gcaatggagc agggcagcgg     60
   ccgcttggag gacttccctg tcaatgtgtt ctccgtcact ccttacacac ccagcaccgc    120
   tgacatccag gtgtccgatg atgacaaggc gggggccacc ttgctcttct caggcatctt    180
   tctgggactg gtggggatca cattcactgt catgggctgg atcaaatacc aaggtgtctc    240
   ccactttgaa tggacccagc tccttgggcc cgtcctgctg tcagttgggg tgacattcat    300
   cctgattgct gtgtgcaagt tcaaaatgct ctcctgccag ttgtgcaaag aaagtgagga    360
   aagggtcccg gactcggaac agacaccagg aggaccatca tttgttttca ctggcatcaa    420
   ccaacccatc accttccatg gggccactgt ggtgcagtac atccctcctc cttatggttc    480
   tccagagcct atggggataa ataccagcta cctgcagtct gtggtgagcc cctgcggcct    540
   cataacctct ggaggggcag cagccgccat gtcaagtcct cctcaatact acaccatcta    600
   ccctcaagat aactctgcat ttgtggttga tgagggctgc ctttctttca cggacggtgg    660
   aaatcacagg cccaatcctg atgttgacca gctagaagag acacagctgg aagaggaggc    720
   ctgtgcctgc ttctctcctc ccccttatga agaaatatac tctctccctc gctagaggct    780
   attctgatat aataacacaa tgctcagctc agggagcaag tgtttccgtc attgttacct    840
   gacaaccgtg gtgttctatg ttgtaacctt cagaagttac agcagcgccc aggcagcctg    900
   acagagatca ttcaaggggg gaaaggggaa gtgggaggtg caatttctca gattggtaaa    960
   aattaggctg ggctggggaa attctcctcc ggaacagttt caaattccct cgggtaagaa   1020
   atctcctgta taaggttcag gagcaggaat ttcacttttt catccaccac cctccccctt   1080
   ctctgtagga aggcattggt ggctcaattt taaccccagc agccaatgga aaaatcacga   1140
   cttctgagac tttgggagtt tccacagagg tgagagtcgg gtgggaagga agcagggaag   1200
   agaaagcagg cccagctgga gatttcctgg tggctgtcct tggccccaaa gcagactcac   1260
   taatcccaaa caactcagct gccatctggc ctctctgagg actctgggta ccttaaagac   1320
   tata                                                                1324
<210> 59
   <211> 683
   <212> DNA
   <213> Homo sapiens
<400> 59
   caggaaagtt cgtgctgcta ggcagaggaa ctgcagcttg ttggcaggtg aagggagcct      60
   gtttagctgt gtccagcaac aacttacgtg gtcctgcttg tgttccaggt gaagcgtctg     120
   gccgccgagc agaggaatca agacctgctc attctttcct cgggggatcc atccagcaat     180
   gacatcatct catgctgcca caaggacccc aagtctgggc tgctggggac cagccacgct     240
   ccccactgct cattccttca tcctagagac attctgactc tcctccgact gcgctgtgca     300
   caggcgtgac aagctctttt acatctcagt ctgcacaact tcaggcactt agcagattga     360
   tatgcatcca acaaatattg attgaatatc tgctaaatac ccagtaatgt ttcatgagtg     420
   attgggtgaa taaaggaatg ctggttcctt ctggccatat taactcctgc acaatactaa     480
   gaaaaataaa ttgcactagc tgtggaataa tgtgaatccc aatgtcatct attgaaatat     540
   tacctgacta ttaagaggta tttatttttg tatcttttct agcaaagtaa ataaaattct     600
   taatacagca tatcccctta ttcacggggg gtatgttcca agacccccgg tggatgcctg     660
   aaactatgga taataccaga tcc                                             683
<210> 60
   <211> 914
   <212> PRT
   <213> Homo sapiens
<400> 60
   Met Gly Pro Phe Lys Ser Ser Val Phe Ile Leu Ile Leu His Leu Leu
   1               5                   10                  15
   Glu Gly Ala Leu Ser Asn Ser Leu Ile Gln Leu Asn Asn Asn Gly Tyr
               20                  25                  30
   Glu Gly Ile Val Val Ala Ile Asp Pro Asn Val Pro Glu Asp Glu Thr
           35                  40                  45
   Leu Ile Gln Gln Ile Lys Asp Met Val Thr Gln Ala Ser Leu Tyr Leu
       50                  55                  60
   Phe Glu Ala Thr Gly Lys Arg Phe Tyr Phe Lys Asn Val Ala Ile Leu
   65                  70                  75                  80
   Ile Pro Glu Thr Trp Lys Thr Lys Ala Asp Tyr Val Arg Pro Lys Leu
                   85                  90                  95
   Glu Thr Tyr Lys Asn Ala Asp Val Leu Val Ala Glu Ser Thr Pro Pro
               100                 105                 110
   Gly Asn Asp Glu Pro Tyr Thr Glu Gln Met Gly Asn Cys Gly Glu Lys
           115                 120                 125
   Gly Glu Arg Ile His Leu Thr Pro Asp Phe Ile Ala Gly Lys Lys Leu
       130                 135                 140
   Ala Glu Tyr Gly Pro Gln Gly Lys Ala Phe Val His Glu Trp Ala His
   145                 150                 155                 160
   Leu Arg Trp Gly Val Phe Asp Glu Tyr Asn Asn Asp Glu Lys Phe Tyr
                   165                 170                 175
   Leu Ser Asn Gly Arg Ile Gln Ala Val Arg Cys Ser Ala Gly Ile Thr
               180                 185                 190
   Gly Thr Asn Val Val Lys Lys Cys Gln Gly Gly Ser Cys Tyr Thr Lys
           195                 200                 205
   Arg Cys Thr Phe Asn Lys Val Thr Gly Leu Tyr Glu Lys Gly Cys Glu
       210                 215                 220
   Phe Val Leu Gln Ser Arg Gln Thr Glu Lys Ala Ser Ile Met Phe Ala
   225 230 235 240
   Gln His Val Asp Ser Ile Val Glu Phe Cys Thr Glu Gln Asn His Asn
   245 250 255
   Lys Glu Ala Pro Asn Lys Gln Asn Gln Lys Cys Asn Leu Arg Ser Thr
   260 265 270
   Trp Glu Val Ile Arg Asp Ser Glu Asp Phe Lys Lys Thr Thr Pro Met
   275 280 285
   Thr Thr Gln Pro Pro Asn Pro Thr Phe Ser Leu Leu Gln Ile Gly Gln
   290 295 300
   Arg Ile Val Cys Leu Val Leu Asp Lys Ser Gly Ser Met Ala Thr Gly
   305 310 315 320
   Asn Arg Leu Asn Arg Leu Asn Gln Ala Gly Gln Leu Phe Leu Leu Gln
   325 330 335
   Thr Val Glu Leu Gly Ser Trp Val Gly Met Val Thr Phe Asp Ser Ala
   340 345 350
   Ala His Val Gln Ser Glu Leu Ile Gln Ile Asn Ser Gly Ser Asp Arg
   355 360 365
   Asp Thr Leu Ala Lys Arg Leu Pro Ala Ala Ala Ser Gly Gly Thr Ser
   370 375 380
   Ile Cys Ser Gly Leu Arg Ser Ala Phe Thr Val Ile Arg Lys Lys Tyr
   385 390 395 400
   Pro Thr Asp Gly Ser Glu Ile Val Leu Leu Thr Asp Gly Glu Asp Asn
   405 410 415
   Thr Ile Ser Gly Cys Phe Asn Glu Val Lys Gln Ser Gly Ala Ile Ile
   420 425 430
   His Thr Val Ala Leu Gly Pro Ser Ala Ala Gln Glu Leu Glu Glu Leu
   435 440 445
   Ser Lys Met Thr Gly Gly Leu Gln Thr Tyr Ala Ser Asp Gln Val Gln
   450 455 460
   Asn Asn Gly Leu Ile Asp Ala Phe Gly Ala Leu Ser Ser Gly Asn Gly
   465 470 475 480
   Ala Val Ser Gln Arg Ser Ile Gln Leu Glu Ser Lys Gly Leu Thr Leu
   485 490 495
   Gln Asn Ser Gln Trp Met Asn Gly Thr Val Ile Val Asp Ser Thr Val
   500 505 510
   Gly Lys Asp Thr Leu Phe Leu Ile Thr Trp Thr Thr Gln Pro Pro Gln
   515 520 525
   Ile Leu Leu Trp Asp Pro Ser Gly Gln Lys Gln Gly Gly Phe Val Val
   530 535 540
   Asp Lys Asn Thr Lys Met Ala Tyr Leu Gln Ile Pro Gly Ile Ala Lys
   545 550 555 560
   Val Gly Thr Trp Lys Tyr Ser Leu Gln Ala Ser Ser Gln Thr Leu Thr
   565 570 575
   Leu Thr Val Thr Ser Arg Ala Ser Asn Ala Thr Leu Pro Pro Ile Thr
   580 585 590
   Val Thr Ser Lys Thr Asn Lys Asp Thr Ser Lys Phe Pro Ser Pro Leu
   595 600 605
   Val Val Tyr Ala Asn Ile Arg Gln Gly Ala Ser Pro Ile Leu Arg Ala
   610 615 620
   Ser Val Thr Ala Leu Ile Glu Ser Val Asn Gly Lys Thr Val Thr Leu
   625 630 635 640
   Glu Leu Leu Asp Asn Gly Ala Gly Ala Asp Ala Thr Lys Asp Asp Gly
   645 650 655
   Val Tyr Ser Arg Tyr Phe Thr Thr Tyr Asp Thr Asn Gly Arg Tyr Ser
   660 665 670
   Val Lys Val Arg Ala Leu Gly Gly Val Asn Ala Ala Arg Arg Arg Val
   675 680 685
   Ile Pro Gln Gln Ser Gly Ala Leu Tyr Ile Pro Gly Trp Ile Glu Asn
   690 695 700
   Asp Glu Ile Gln Trp Asn Pro Pro Arg Pro Glu Ile Asn Lys Asp Asp
   705 710 715 720
   Val Gln His Lys Gln Val Cys Phe Ser Arg Thr Ser Ser Gly Gly Ser
   725 730 735
   Phe Val Ala Ser Asp Val Pro Asn Ala Pro Ile Pro Asp Leu Phe Pro
   740 745 750
   Pro Gly Gln Ile Thr Asp Leu Lys Ala Glu Ile His Gly Gly Ser Leu
   755 760 765
   Ile Asn Leu Thr Trp Thr Ala Pro Gly Asp Asp Tyr Asp His Gly Thr
   770 775 780
   Ala His Lys Tyr Ile Ile Arg Ile Ser Thr Ser Ile Leu Asp Leu Arg
   785 790 795 800
   Asp Lys Phe Asn Glu Ser Leu Gln Val Asn Thr Thr Ala Leu Ile Pro
   805 810 815
   Lys Glu Ala Asn Ser Glu Glu Val Phe Leu Phe Lys Pro Glu Asn Ile
   820 825 830
   Thr Phe Glu Asn Gly Thr Asp Leu Phe Ile Ala Ile Gln Ala Val Asp
   835 840 845
   Lys Val Asp Leu Lys Ser Glu Ile Ser Asn Ile Ala Arg Val Ser Leu
   850 855 860
   Phe Ile Pro Pro Gln Thr Pro Pro Glu Thr Pro Ser Pro Asp Glu Thr
   865 870 875 880
   Ser Ala Pro Cys Pro Asn Ile His Ile Asn Ser Thr Ile Pro Gly Ile
   885 890 895
   His Ile Leu Lys Ile Met Trp Lys Trp Ile Gly Glu Leu Gln Leu Ser
   900 905 910
   Ile Ala
<210> 61
   <211> 501
   <212> PRT
   <213> Homo sapiens
<400> 61
   Met Lys Lys Glu Gly Arg Lys Arg Trp Lys Arg Lys Glu Asp Lys Lys
   1 5 10 15
   Arg Val Val Val Ser Asn Leu Leu Phe Glu Gly Trp Ser His Lys Glu
               20                  25                  30
   Asn Pro Asn Arg His His Arg Gly Asn Gln Ile Lys Thr Ser Lys Tyr
           35                  40                  45
   Thr Val Leu Ser Phe Val Pro Lys Asn Ile Phe Glu Gln Leu His Arg
       50                  55                  60
   Phe Ala Asn Leu Tyr Phe Val Gly Ile Ala Val Leu Asn Phe Ile Pro
   65                  70                  75                  80
   Val Val Asn Ala Phe Gln Pro Glu Val Ser Met Ile Pro Ile Cys Val
                   85                  90                  95
   Ile Leu Ala Val Thr Ala Ile Lys Asp Ala Trp Glu Asp Leu Arg Arg
               100                 105                 110
   Tyr Lys Ser Asp Lys Val Ile Asn Asn Arg Glu Cys Leu Ile Tyr Ser
           115                 120                 125
   Arg Lys Glu Gln Thr Tyr Val Gln Lys Cys Trp Lys Asp Val Arg Val
       130                 135                 140
   Gly Asp Phe Ile Gln Met Lys Cys Asn Glu Ile Val Pro Ala Asp Ile
   145                 150                 155                 160
   Leu Leu Leu Phe Ser Ser Asp Pro Asn Gly Ile Cys His Leu Glu Thr
                   165                 170                 175
   Ala Ser Leu Asp Gly Glu Thr Asn Leu Lys Gln Arg Arg Val Val Lys
               180                 185                 190
   Gly Phe Ser Gln Gln Glu Val Gln Phe Glu Pro Glu Leu Phe His Asn
           195                 200                 205
   Thr Ile Val Cys Glu Lys Pro Asn Asn His Leu Asn Lys Phe Lys Gly
       210                 215                 220
   Tyr Met Glu His Pro Asp Gln Thr Arg Thr Gly Phe Gly Cys Glu Ser
   225                 230                 235                 240
   Leu Leu Leu Arg Gly Cys Thr Ile Arg Asn Thr Glu Met Ala Val Gly
                   245                 250                 255
   Ile Val Ile Tyr Ala Gly His Glu Thr Lys Ala Met Leu Asn Asn Ser
               260                 265                 270
   Gly Pro Arg Tyr Lys Arg Ser Lys Ile Glu Arg Arg Met Asn Ile Asp
   275 280 285
   Ile Phe Phe Cys Ile Gly Ile Leu Ile Leu Met Cys Leu Ile Gly Ala
   290 295 300
   Val Gly His Ser Ile Trp Asn Gly Thr Phe Glu Glu His Pro Pro Phe
   305 310 315 320
   Asp Val Pro Asp Ala Asn Gly Ser Phe Leu Pro Ser Ala Leu Gly Gly
   325 330 335
   Phe Tyr Met Phe Leu Thr Met Ile Ile Leu Leu Gln Val Leu Ile Pro
   340 345 350
   Ile Ser Leu Tyr Val Ser Ile Glu Leu Val Lys Leu Gly Gln Val Phe
   355 360 365
   Phe Leu Ser Asn Asp Leu Asp Leu Tyr Asp Glu Glu Thr Asp Leu Ser
   370 375 380
   Ile Gln Cys Arg Ala Leu Asn Ile Ala Glu Asp Leu Gly Gln Ile Gln
   385 390 395 400
   Tyr Ile Phe Ser Asp Lys Thr Gly Thr Leu Thr Glu Asn Lys Met Val
   405 410 415
   Phe Arg Arg Cys Thr Ile Met Gly Ser Glu Tyr Ser His Gln Glu Asn
   420 425 430
   Gly Ile Glu Ala Pro Lys Gly Ser Ile Pro Leu Ser Lys Arg Lys Tyr
   435 440 445
   Pro Ala Leu Leu Arg Asn Glu Glu Ile Lys Asp Ile Leu Leu Ala Leu
   450 455 460
   Leu Glu Ala Val Trp His Phe His Lys Leu Leu Pro Val Ser Leu Trp
   465 470 475 480
   Ser Ser Leu Ser Gln Ile Arg Ala Val Pro Ile Thr Cys Lys Leu Ser
   485 490 495
   Phe Val Tyr Lys Gly
   500
<210> 62
   <211> 154
   <212> PRT
   <213> Homo sapiens
<400> 62
   Met Gly Arg Arg Ser Pro Phe Lys Pro Arg Asn Lys Val Phe Gly Phe
   1               5                   10                  15
   Ser Tyr Pro Trp Cys Arg Ser Tyr Gln Pro Phe Pro Arg Lys Arg Ala
               20                  25                  30
   Trp Pro Pro Ser Arg Val Trp Leu Gly Ala Cys Cys Ala Ser Leu Ala
           35                  40                  45
   Ser Pro Pro Lys Gly Thr Ile Pro Ser Gly Glu Tyr Tyr Arg Pro Ala
       50                  55                  60
   Pro Ser Ser Ser Gly Asp Ser Leu Arg Arg Glu Ser Gly Ala Leu Leu
   65                  70                  75                  80
   Gln Tyr Leu Pro Ser Leu Ala Ser Pro Cys Ala Asn His Ala Thr Arg
                   85                  90                  95
   Cys Ser Leu Leu Phe Pro Ile Tyr Lys Ile Lys Met Thr Leu Leu Tyr
               100                 105                 110
   Leu Thr Gly Leu Ala Arg Thr His Cys Cys Cys Leu Ala Asp Arg Cys
           115                 120                 125
   Ala Glu Ala Val Glu Ser Ala Phe Tyr Leu Val Gly Ser Leu Cys Ile
       130                 135                 140
   Asn Ala Arg Gly Ala Ala His Leu Thr Asp
   145                 150
<210> 63
   <211> 484
   <212> PRT
   <213> Homo sapiens
<400> 63
   Met Ala Gly Pro Trp Thr Phe Thr Leu Leu Cys Gly Leu Leu Ala Ala
   1               5                   10                  15
   Thr Leu Ile Gln Ala Thr Leu Ser Pro Thr Ala Val Leu Ile Leu Gly
               20                  25                  30
   Pro Lys Val Ile Lys Glu Lys Leu Thr Gln Glu Leu Lys Asp His Asn
           35                  40                  45
   Ala Thr Ser Ile Leu Gln Gln Leu Pro Leu Leu Ser Ala Met Arg Glu
       50                  55                  60
   Lys Pro Ala Gly Gly Ile Pro Val Leu Gly Ser Leu Val Asn Thr Val
   65                  70                  75                  80
   Leu Lys His Ile Ile Trp Leu Lys Val Ile Thr Ala Asn Ile Leu Gln
                   85                  90                  95
   Leu Gln Val Lys Pro Ser Ala Asn Asp Gln Glu Leu Leu Val Lys Ile
               100                 105                 110
   Pro Leu Asp Met Val Ala Gly Phe Asn Thr Pro Leu Val Lys Thr Ile
           115                 120                 125
   Val Glu Phe His Met Thr Thr Glu Ala Gln Ala Thr Ile Arg Met Asp
       130                 135                 140
   Thr Ser Ala Ser Gly Pro Thr Arg Leu Val Leu Ser Asp Cys Ala Thr
   145                 150                 155                 160
   Ser His Gly Ser Leu Arg Ile Gln Leu Leu His Lys Leu Ser Phe Leu
                   165                 170                 175
   Val Asn Ala Leu Ala Lys Gln Val Met Asn Leu Leu Val Pro Ser Leu
               180                 185                 190
   Pro Asn Leu Val Lys Asn Gln Leu Cys Pro Val Ile Glu Ala Ser Phe
           195                 200                 205
   Asn Gly Met Tyr Ala Asp Leu Leu Gln Leu Val Lys Val Pro Ile Ser
       210                 215                 220
   Leu Ser Ile Asp Arg Leu Glu Phe Asp Leu Leu Tyr Pro Ala Ile Lys
   225                 230                 235                 240
   Gly Asp Thr Ile Gln Leu Tyr Leu Gly Ala Lys Leu Leu Asp Ser Gln
                   245                 250                 255
   Gly Lys Val Thr Lys Trp Phe Asn Asn Ser Ala Ala Ser Leu Thr Met
               260                 265                 270
   Pro Thr Leu Asp Asn Ile Pro Phe Ser Leu Ile Val Ser Gln Asp Val
           275                 280                 285
   Val Lys Ala Ala Val Ala Ala Val Leu Ser Pro Glu Glu Phe Met Val
       290                 295                 300
   Leu Leu Asp Ser Val Leu Pro Glu Ser Ala His Arg Leu Lys Ser Ser
   305 310 315 320
   Ile Gly Leu Ile Asn Glu Lys Ala Ala Asp Lys Leu Gly Ser Thr Gln
   325 330 335
   Ile Val Lys Ile Leu Thr Gln Asp Thr Pro Glu Phe Phe Ile Asp Gln
   340 345 350
   Gly His Ala Lys Val Ala Gln Leu Ile Val Leu Glu Val Phe Pro Ser
   355 360 365
   Ser Glu Ala Leu Arg Pro Leu Phe Thr Leu Gly Ile Glu Ala Ser Ser
   370 375 380
   Glu Ala Gln Phe Tyr Thr Lys Gly Asp Gln Leu Ile Leu Asn Leu Asn
   385 390 395 400
   Asn Ile Ser Ser Asp Arg Ile Gln Leu Met Asn Ser Gly Ile Gly Trp
   405 410 415
   Phe Gln Pro Asp Val Leu Lys Asn Ile Ile Thr Glu Ile Ile His Ser
   420 425 430
   Ile Leu Leu Pro Asn Gln Asn Gly Lys Leu Arg Ser Gly Val Pro Val
   435 440 445
   Ser Leu Val Lys Ala Leu Gly Phe Glu Ala Ala Glu Ser Ser Leu Thr
   450 455 460
   Lys Asp Ala Leu Val Leu Thr Pro Ala Ser Leu Trp Lys Pro Ser Ser
   465 470 475 480
   Pro Val Ser Gln
<210> 64
   <211> 256
   <212> PRT
   <213> Homo sapiens
<400> 64
   Met Phe Gln Thr Gly Gly Leu Ile Val Phe Tyr Gly Leu Leu Ala Gln
   1 5 10 15
   Thr Met Ala Gln Phe Gly Gly Leu Pro Val Pro Leu Asp Gln Thr Leu
   20 25 30
   Pro Leu Asn Val Asn Pro Ala Leu Pro Leu Ser Pro Thr Gly Leu Ala
           35                  40                  45
   Gly Ser Leu Thr Asn Ala Leu Ser Asn Gly Leu Leu Ser Gly Gly Leu
       50                  55                  60
   Leu Gly Ile Leu Glu Asn Leu Pro Leu Leu Asp Ile Leu Lys Pro Gly
   65                  70                  75                  80
   Gly Gly Thr Ser Gly Gly Leu Leu Gly Gly Leu Leu Gly Lys Val Thr
                   85                  90                  95
   Ser Val Ile Pro Gly Leu Asn Asn Ile Ile Asp Ile Lys Val Thr Asp
               100                 105                 110
   Pro Gln Leu Leu Glu Leu Gly Leu Val Gln Ser Pro Asp Gly His Arg
           115                 120                 125
   Leu Tyr Val Thr Ile Pro Leu Gly Ile Lys Leu Gln Val Asn Thr Pro
       130                 135                 140
   Leu Val Gly Ala Ser Leu Leu Arg Leu Ala Val Lys Leu Asp Ile Thr
   145                 150                 155                 160
   Ala Glu Ile Leu Ala Val Arg Asp Lys Gln Glu Arg Ile His Leu Val
                   165                 170                 175
   Leu Gly Asp Cys Thr His Ser Pro Gly Ser Leu Gln Ile Ser Leu Leu
               180                 185                 190
   Asp Gly Leu Gly Pro Leu Pro Ile Gln Gly Leu Leu Asp Ser Leu Thr
           195                 200                 205
   Gly Ile Leu Asn Lys Val Leu Pro Glu Leu Val Gln Gly Asn Val Cys
       210                 215                 220
   Pro Leu Val Asn Glu Val Leu Arg Gly Leu Asp Ile Thr Leu Val His
   225                 230                 235                 240
   Asp Ile Val Asn Met Leu Ile His Gly Leu Gln Phe Val Ile Lys Val
                   245                 250                 255
<210> 65
   <211> 791
   <212> PRT
   <213> Homo sapiens
<400> 65
   Met Ser Gln Pro Arg Pro Arg Tyr Val Val Asp Arg Ala Ala Tyr Ser
   1               5                   10                  15
   Leu Thr Leu Phe Asp Asp Glu Phe Glu Lys Lys Asp Arg Thr Tyr Pro
               20                  25                  30
   Val Gly Glu Lys Leu Arg Asn Ala Phe Arg Cys Ser Ser Ala Lys Ile
           35                  40                  45
   Lys Ala Val Val Phe Gly Leu Leu Pro Val Leu Ser Trp Leu Pro Lys
       50                  55                  60
   Tyr Lys Ile Lys Asp Tyr Ile Ile Pro Asp Leu Leu Gly Gly Leu Ser
   65                  70                  75                  80
   Gly Gly Ser Ile Gln Val Pro Gln Gly Met Ala Phe Ala Leu Leu Ala
                   85                  90                  95
   Asn Leu Pro Ala Val Asn Gly Leu Tyr Ser Ser Phe Phe Pro Leu Leu
               100                 105                 110
   Thr Tyr Phe Phe Leu Gly Gly Val His Gln Met Val Pro Gly Thr Phe
           115                 120                 125
   Ala Val Ile Ser Ile Leu Val Gly Asn Ile Cys Leu Gln Leu Ala Pro
       130                 135                 140
   Glu Ser Lys Phe Gln Val Phe Asn Asn Ala Thr Asn Glu Ser Tyr Val
   145                 150                 155                 160
   Asp Thr Ala Ala Met Glu Ala Glu Arg Leu His Val Ser Ala Thr Leu
                   165                 170                 175
   Ala Cys Leu Thr Ala Ile Ile Gln Met Gly Leu Gly Phe Met Gln Phe
               180                 185                 190
   Gly Phe Val Ala Ile Tyr Leu Ser Glu Ser Phe Ile Arg Gly Phe Met
           195                 200                 205
   Thr Ala Ala Gly Leu Gln Ile Leu Ile Ser Val Leu Lys Tyr Ile Phe
       210                 215                 220
   Gly Leu Thr Ile Pro Ser Tyr Thr Gly Pro Gly Ser Ile Val Phe Thr
   225                 230                 235                 240
   Phe Ile Asp Ile Cys Lys Asn Leu Pro His Thr Asn Ile Ala Ser Leu
                   245                 250                 255
   Ile Phe Ala Leu Ile Ser Gly Ala Phe Leu Val Leu Val Lys Glu Leu
               260                 265                 270
   Asn Ala Arg Tyr Met His Lys Ile Arg Phe Pro Ile Pro Thr Glu Met
           275                 280                 285
   Ile Val Val Val Val Ala Thr Ala Ile Ser Gly Gly Cys Lys Met Pro
       290                 295                 300
   Lys Lys Tyr His Met Gln Ile Val Gly Glu Ile Gln Arg Gly Phe Pro
   305                 310                 315                 320
   Thr Pro Val Ser Pro Val Val Ser Gln Trp Lys Asp Met Ile Gly Thr
                   325                 330                 335
   Ala Phe Ser Leu Ala Ile Val Ser Tyr Val Ile Asn Leu Ala Met Gly
               340                 345                 350
   Arg Thr Leu Ala Asn Lys His Gly Tyr Asp Val Asp Ser Asn Gln Glu
           355                 360                 365
   Met Ile Ala Leu Gly Cys Ser Asn Phe Phe Gly Ser Phe Phe Lys Ile
       370                 375                 380
   His Val Ile Cys Cys Ala Leu Ser Val Thr Leu Ala Val Asp Gly Ala
   385                 390                 395                 400
   Gly Gly Lys Ser Gln Val Ala Ser Leu Cys Val Ser Leu Val Val Met
                   405                 410                 415
   Ile Thr Met Leu Val Leu Gly Ile Tyr Leu Tyr Pro Leu Pro Lys Ser
               420                 425                 430
   Val Leu Gly Ala Leu Ile Ala Val Asn Leu Lys Asn Ser Leu Lys Gln
           435                 440                 445
   Leu Thr Asp Pro Tyr Tyr Leu Trp Arg Lys Ser Lys Leu Asp Cys Cys
       450                 455                 460
   Ile Trp Val Val Ser Phe Leu Ser Ser Phe Phe Leu Ser Leu Pro Tyr
   465                 470                 475                 480
   Gly Val Ala Val Gly Val Ala Phe Ser Val Leu Val Val Val Phe Gln
                   485                 490                 495
   Thr Gln Phe Arg Asn Gly Tyr Ala Leu Ala Gln Val Met Asp Thr Asp
               500                 505                 510
   Ile Tyr Val Asn Pro Lys Thr Tyr Asn Arg Ala Gln Asp Ile Gln Gly
           515                 520                 525
   Ile Lys Ile Ile Thr Tyr Cys Ser Pro Leu Tyr Phe Ala Asn Ser Glu
       530                 535                 540
   Ile Phe Arg Gln Lys Val Ile Ala Lys Thr Gly Met Asp Pro Gln Lys
   545                 550                 555                 560
   Val Leu Leu Ala Lys Gln Lys Tyr Leu Lys Lys Gln Glu Lys Arg Arg
                   565                 570                 575
   Met Arg Pro Thr Gln Gln Arg Arg Ser Leu Phe Met Lys Thr Lys Thr
               580                 585                 590
   Val Ser Leu Gln Glu Leu Gln Gln Asp Phe Glu Asn Ala Pro Pro Thr
           595                 600                 605
   Asp Pro Asn Asn Asn Gln Thr Pro Ala Asn Gly Thr Ser Val Ser Tyr
       610                 615                 620
   Ile Thr Phe Ser Pro Asp Ser Ser Ser Pro Ala Gln Ser Glu Pro Pro
   625                 630                 635                 640
   Ala Ser Ala Glu Ala Pro Gly Glu Pro Ser Asp Met Leu Ala Ser Val
                   645                 650                 655
   Pro Pro Phe Val Thr Phe His Thr Leu Ile Leu Asp Met Ser Gly Val
               660                 665                 670
   Ser Phe Val Asp Leu Met Gly Ile Lys Ala Leu Ala Lys Leu Ser Ser
           675                 680                 685
   Thr Tyr Gly Lys Ile Gly Val Lys Val Phe Leu Val Asn Ile His Ala
       690                 695                 700
   Gln Val Tyr Asn Asp Ile Ser His Gly Gly Val Phe Glu Asp Gly Ser
   705                 710                 715                 720
   Leu Glu Cys Lys His Val Phe Pro Ser Ile His Asp Ala Val Leu Phe
                   725                 730                 735
   Ala Gln Ala Asn Ala Arg Asp Val Thr Pro Gly His Asn Phe Gln Gly
               740                 745                 750
   Ala Pro Gly Asp Ala Glu Leu Ser Leu Tyr Asp Ser Glu Glu Asp Ile
           755                 760                 765
   Arg Ser Tyr Trp Asp Leu Glu Gln Glu Met Phe Gly Ser Met Phe His
       770                 775                 780
   Ala Glu Thr Leu Thr Ala Leu
   785                 790
<210> 66
   <211> 243
   <212> PRT
   <213> Homo sapiens
<400> 66
   Met Glu Gln Gly Ser Gly Arg Leu Glu Asp Phe Pro Val Asn Val Phe
   1               5                   10                  15
   Ser Val Thr Pro Tyr Thr Pro Ser Thr Ala Asp Ile Gln Val Ser Asp
               20                  25                  30
   Asp Asp Lys Ala Gly Ala Thr Leu Leu Phe Ser Gly Ile Phe Leu Gly
           35                  40                  45
   Leu Val Gly Ile Thr Phe Thr Val Met Gly Trp Ile Lys Tyr Gln Gly
       50                  55                  60
   Val Ser His Phe Glu Trp Thr Gln Leu Leu Gly Pro Val Leu Leu Ser
   65                  70                  75                  80
   Val Gly Val Thr Phe Ile Leu Ile Ala Val Cys Lys Phe Lys Met Leu
                   85                  90                  95
   Ser Cys Gln Leu Cys Lys Glu Ser Glu Glu Arg Val Pro Asp Ser Glu
               100                 105                 110
   Gln Thr Pro Gly Gly Pro Ser Phe Val Phe Thr Gly Ile Asn Gln Pro
           115                 120                 125
   Ile Thr Phe His Gly Ala Thr Val Val Gln Tyr Ile Pro Pro Pro Tyr
       130                 135                 140
   Gly Ser Pro Glu Pro Met Gly Ile Asn Thr Ser Tyr Leu Gln Ser Val
   145                 150                 155                 160
   Val Ser Pro Cys Gly Leu Ile Thr Ser Gly Gly Ala Ala Ala Ala Met
                   165                 170                 175
   Ser Ser Pro Pro Gln Tyr Tyr Thr Ile Tyr Pro Gln Asp Asn Ser Ala
               180                 185                 190
   Phe Val Val Asp Glu Gly Cys Leu Ser Phe Thr Asp Gly Gly Asn His
           195                 200                 205
   Arg Pro Asn Pro Asp Val Asp Gln Leu Glu Glu Thr Gln Leu Glu Glu
       210                 215                 220
   Glu Ala Cys Ala Cys Phe Ser Pro Pro Pro Tyr Glu Glu Ile Tyr Ser
   225                 230                 235                 240
   Leu Pro Arg
<210> 67
   <211> 21
   <212> DNA
   <213> Künstliche Sequenz
<220>
   <223> Beschreibung der künstlichen Sequenz: Oligonukleotid
<400> 67
   acacgaatgg tagatacagt g 21
<210> 68
   <211> 21
   <212> DNA
   <213> Künstliche Sequenz
<220>
   <223> Beschreibung der künstlichen Sequenz: Oligonukleotid
<400> 68
   atacttgtga gctgttccat g 21
<210> 69
   <211> 21
   <212> DNA
   <213> Künstliche Sequenz
<220>
   <223> Beschreibung der künstlichen Sequenz: Oligonukleotid
<400> 69
   actgttacct tgcatggact g 21
<210> 70
   <211> 21
   <212> DNA
   <213> Künstliche Sequenz
<220>
   <223> Beschreibung der künstlichen Sequenz: Oligonukleotid
<400> 70
   caatgagaac acatggacat g 21
<210> 71
   <211> 21
   <212> DNA
   <213> Künstliche Sequenz
<220>
   <223> Beschreibung der künstlichen Sequenz: Oligonukleotid
<400> 71
   ccatgaaagc tccatgtcta c 21
<210> 72
   <211> 21
   <212> DNA
   <213> Künstliche Sequenz
<220>
   <223> Beschreibung der künstlichen Sequenz: Oligonukleotid
<400> 72
   agagatggca catattctgt c 21
<210> 73
   <211> 21
   <212> DNA
   <213> Künstliche Sequenz
<220>
   <223> Beschreibung der künstlichen Sequenz: Oligonukleotid
<400> 73
   atcggctgaa gtcaagcatc g 21
<210> 74
   <211> 21
   <212> DNA
   <213> Künstliche Sequenz
<220>
   <223> Beschreibung der künstlichen Sequenz: Oligonukleotid
<400> 74
   tggtcagtga ggactcagct g 21
<210> 75
   <211> 21
   <212> DNA
   <213> Künstliche Sequenz
<220>
   <223> Beschreibung der künstlichen Sequenz: Oligonukleotid
<400> 75
   tttctctgct tgatgcactt g 21
<210> 76
   <211> 21
   <212> DNA
   <213> Künstliche Sequenz
<220>
   <223> Beschreibung der künstlichen Sequenz: Oligonukleotid
<400> 76
   gtgagcactg ggaagcagct c 21
<210> 77
   <211> 21
   <212> DNA
   <213> Künstliche Sequenz
<220>
   <223> Beschreibung der künstlichen Sequenz: Oligonukleotid
<400> 77
   ggcaaatgct agagacgtga c 21
<210> 78
   <211> 21
   <212> DNA
   <213> Künstliche Sequenz
<220>
   <223> Beschreibung der künstlichen Sequenz: Oligonukleotid
<400> 78
   aggtgtcctt cagctgccaa g 21
<210> 79
   <211> 21
   <212> DNA
   <213> Künstliche Sequenz
<220>
   <223> Beschreibung der künstlichen Sequenz: Oligonukleotid
<400> 79
   gttaagtgct ctctggattt g 21
<210> 80
   <211> 21
   <212> DNA
   <213> Künstliche Sequenz
<220>
   <223> Beschreibung der künstlichen Sequenz: Oligonukleotid
<400> 80
   atcctgattg ctgtgtgcaa g 21
<210> 81
   <211> 21
   <212> DNA
   <213> Künstliche Sequenz
<220>
   <223> Beschreibung der künstlichen Sequenz: Oligonukleotid
<400> 81
   ctcttctagc tggtcaacat c 21
<210> 82
   <211> 21
   <212> DNA
   <213> Künstliche Sequenz
<220>
   <223> Beschreibung der künstlichen Sequenz: Oligonukleotid
<400> 82
   ccagcaacaa cttacgtggt c 21
<210> 83
   <211> 21
   <212> DNA
   <213> Künstliche Sequenz
<220>
   <223> Beschreibung der künstlichen Sequenz: Oligonukleotid
<400> 83
   cctttattca cccaatcact c 21
<210> 84
   <211> 2165
   <212> DNA
   <213> Homo sapiens
<400> 84
   agaacagcgc agtttgccct ccgctcacgc agagcctctc cgtggcctcc gcaccttgag     60
   cattaggcca gttctcctct tctctctaat ccatccgtca cctctcctgt catccgtttc    120
   catgccgtga ggtccattca cagaacacat ccatggctct catgctcagt ttggttctga    180
   gtctcctcaa gctgggatca gggcagtggc aggtgtttgg gccagacaag cctgtccagg    240
   ccttggtggg ggaggacgca gcattctcct gtttcctgtc tcctaagacc aatgcagagg    300
   ccatggaagt gcggttcttc aggggccagt tctctagcgt ggtccacctc tacagggacg    360
   ggaaggacca gccatttatg cagatgccac agtatcaagg caggacaaaa ctggtgaagg    420
   attctattgc ggaggggcgc atctctctga ggctggaaaa cattactgtg ttggatgctg    480
   gcctctatgg gtgcaggatt agttcccagt cttactacca gaaggccatc tgggagctac    540
   aggtgtcagc actgggctca gttcctctca tttccatcac gggatatgtt gatagagaca    600
   tccagctact ctgtcagtcc tcgggctggt tcccccggcc cacagcgaag tggaaaggtc    660
   cacaaggaca ggatttgtcc acagactcca ggacaaacag agacatgcat ggcctgtttg 720
   atgtggagat ctctctgacc gtccaagaga acgccgggag catatcctgt tccatgcggc 780
   atgctcatct gagccgagag gtggaatcca gggtacagat aggagatacc tttttcgagc 840
   ctatatcgtg gcacctggct accaaagtac tgggaatact ctgctgtggc ctattttttg 900
   gcattgttgg actgaagatt ttcttctcca aattccagtg taagcgagag agagaagcat 960
   gggccggtgc cttattcatg gttccagcag ggacaggatc agagatgctc ccacatccag 1020
   ctgcttctct tcttctagtc ctagcctcca ggggcccagg cccaaaaaag gaaaatccag 1080
   gcggaactgg actggagaag aaagcacgga caggcagaat tgagagacgc ccggaaacac 1140
   gcagtggagg tgactctgga tccagagacg gctcacccga agctctgcgt ttctgatctg 1200
   aaaactgtaa cccatagaaa agctccccag gaggtgcctc actctgagaa gagatttaca 1260
   aggaagagtg tggtggcttc tcagagtttc caagcaggga aacattactg ggaggtggac 1320
   ggaggacaca ataaaaggtg gcgcgtggga gtgtgccggg atgatgtgga caggaggaag 1380
   gagtacgtga ctttgtctcc cgatcatggg tactgggtcc tcagactgaa tggagaacat 1440
   ttgtatttca cattaaatcc ccgttttatc agcgtcttcc ccaggacccc acctacaaaa 1500
   ataggggtct tcctggacta tgagtgtggg accatctcct tcttcaacat aaatgaccag 1560
   tcccttattt ataccctgac atgtcggttt gaaggcttat tgaggcccta cattgagtat 1620
   ccgtcctata atgagcaaaa tggaactccc atagtcatct gcccagtcac ccaggaatca 1680
   gagaaagagg cctcttggca aagggcctct gcaatcccag agacaagcaa cagtgagtcc 1740
   tcctcacagg caaccacgcc cttcctcccc aggggtgaaa tgtaggatga atcacatccc 1800
   acattcttct ttagggatat taaggtctct ctcccagatc caaagtcccg cagcagccgg 1860
   ccaaggtggc ttccagatga agggggactg gcctgtccac atgggagtca ggtgtcatgg 1920
   ctgccctgag ctgggaggga agaaggctga cattacattt agtttgctct cactccatct 1980
   ggctaagtga tcttgaaata ccacctctca ggtgaagaac cgtcaggaat tcccatctca 2040
   caggctgtgg tgtagattaa gtagacaagg aatgtgaata atgcttagat cttattgatg 2100
   acagagtgta tcctaatggt ttgttcatta tattacactt tcagtaaaaa aaaaaaaaaa 2160
   aaaaa 2165
<210> 85
   <211> 347
   <212> PRT
   <213> Homo sapiens
<400> 85
   Met Ala Leu Met Leu Ser Leu Val Leu Ser Leu Leu Lys Leu Gly Ser
   1 5 10 15
   Gly Gln Trp Gln Val Phe Gly Pro Asp Lys Pro Val Gln Ala Leu Val
               20                  25                  30
   Gly Glu Asp Ala Ala Phe Ser Cys Phe Leu Ser Pro Lys Thr Asn Ala
           35                  40                  45
   Glu Ala Met Glu Val Arg Phe Phe Arg Gly Gln Phe Ser Ser Val Val
       50                  55                  60
   His Leu Tyr Arg Asp Gly Lys Asp Gln Pro Phe Met Gln Met Pro Gln
   65                  70                  75                  80
   Tyr Gln Gly Arg Thr Lys Leu Val Lys Asp Ser Ile Ala Glu Gly Arg
                   85                  90                  95
   Ile Ser Leu Arg Leu Glu Asn Ile Thr Val Leu Asp Ala Gly Leu Tyr
               100                 105                 110
   Gly Cys Arg Ile Ser Ser Gln Ser Tyr Tyr Gln Lys Ala Ile Trp Glu
           115                 120                 125
   Leu Gln Val Ser Ala Leu Gly Ser Val Pro Leu Ile Ser Ile Thr Gly
       130                 135                 140
   Tyr Val Asp Arg Asp Ile Gln Leu Leu Cys Gln Ser Ser Gly Trp Phe
   145                 150                 155                 160
   Pro Arg Pro Thr Ala Lys Trp Lys Gly Pro Gln Gly Gln Asp Leu Ser
                   165                 170                 175
   Thr Asp Ser Arg Thr Asn Arg Asp Met His Gly Leu Phe Asp Val Glu
               180                 185                 190
   Ile Ser Leu Thr Val Gln Glu Asn Ala Gly Ser Ile Ser Cys Ser Met
           195                 200                 205
   Arg His Ala His Leu Ser Arg Glu Val Glu Ser Arg Val Gln Ile Gly
       210                 215                 220
   Asp Thr Phe Phe Glu Pro Ile Ser Trp His Leu Ala Thr Lys Val Leu
   225                 230                 235                 240
   Gly Ile Leu Cys Cys Gly Leu Phe Phe Gly Ile Val Gly Leu Lys Ile
                   245                 250                 255
   Phe Phe Ser Lys Phe Gln Cys Lys Arg Glu Arg Glu Ala Trp Ala Gly
   260 265 270
   Ala Leu Phe Met Val Pro Ala Gly Thr Gly Ser Glu Met Leu Pro His
   275 280 285
   Pro Ala Ala Ser Leu Leu Leu Val Leu Ala Ser Arg Gly Pro Gly Pro
   290 295 300
   Lys Lys Glu Asn Pro Gly Gly Thr Gly Leu Glu Lys Lys Ala Arg Thr
   305 310 315 320
   Gly Arg Ile Glu Arg Arg Pro Glu Thr Arg Ser Gly Gly Asp Ser Gly
   325 330 335
   Ser Arg Asp Gly Ser Pro Glu Ala Leu Arg Phe
   340 345
<210> 86
   <211> 21
   <212> DNA
   <213> Künstliche Sequenz
<220>
   <223> Beschreibung der künstlichen Sequenz: Oligonukleotid
<400> 86
   attcatggtt ccagcaggga c 21
<210> 87
   <211> 21
   <212> DNA
   <213> Künstliche Sequenz
<220>
   <223> Beschreibung der künstlichen Sequenz: Oligonukleotid
<400> 87
   gggagacaaa gtcacgtact c 21
<210> 88
   <211> 22
   <212> DNA
   <213> künstliche Sequenz
<220>
   <223> Beschreibung der künstlichen Sequenz: Oligonukleotid
<400> 88
   tcctggtgtt cgtggtctgc tt 22
<210> 89
   <211> 22
   <212> DNA
   <213> künstliche Sequenz
<220>
   <223> Beschreibung der künstlichen Sequenz: Oligonukleotid
<400> 89
   gagagtcctg gcttttgtgg gc 22
<210> 90
   <211> 15
   <212> PRT
   <213> Homo sapiens
<400> 90
   Gly Ser Ser Asp Leu Thr Trp Pro Pro Ala Ile Lys Leu Gly Cys
   1               5                   10                  15
<210> 91
   <211> 16
   <212> **PRT**
   <213> Homo sapiens
<400> 91
   Asp Arg Tyr Val Ala Val Arg His Pro Leu Arg Ala Arg Gly Leu Arg
   1               5                   10                  15
<210> 92
   <211> 15
   <212> **PRT**
   <213> Homo sapiens
<400> 92
   Val Ala Pro Arg Ala Lys Ala His Lys Ser Gln Asp Ser Leu Cys
   1               5                   10                  15
<210> 93
   <211> 13
   <212> PRT
   <213> Homo sapiens
<400> 93
   Cys Phe Arg Ser Thr Arg His Asn Phe Asn Ser Met Arg
   1               5                   10
<210> 94
   <211> 22
   <212> PRT
   <213> Homo sapiens
<400> 94
   Met Asn Gly Thr Tyr Asn Thr Cys Gly Ser Ser Asp Leu Thr Trp Pro
   1 5 10 15
   Pro Ala Ile Lys Leu Gly
   20
<210> 95
   <211> 14
   <212> **PRT**
   <213> Homo sapiens
<400> 95
   Arg Asp Thr Ser Asp Thr Pro Leu Cys Gln Leu Ser Gln Gly
   1 5 10
<210> 96
   <211> 22
   <212> PRT
   <213> Homo sapiens
<400> 96
   Gly Ile Gln Glu Gly Gly Phe Cys Phe Arg Ser Thr Arg His Asn Phe
   1 5 10 15
   Asn Ser Met Arg Phe Pro
   20
<210> 97
   <211> 30
   <212> **PRT**
   <213> Homo sapiens
<400> 97
   Ala Lys Glu Phe Gln Glu Ala Ser Ala Leu Ala Val Ala Pro Arg Ala
   1 5 10 15
   Lys Ala His Lys Ser Gln Asp Ser Leu Cys Val Thr Leu Ala
   20 25 30
<210> 98
   <211> 22
   <212> DNA
   <213> künstliche Sequenz
<220>
   <223> Beschreibung der künstlichen Sequenz: Oligonukleotid
<400> 98
   tcctgctcgt cgctctcctg at 22
<210> 99
   <211> 20
   <212> DNA
   <213> künstliche Sequenz
<220>
   <223> Beschreibung der künstlichen Sequenz: Oligonukleotid
<400> 99
   tcgctttttg tcgtatttgc 20
<210> 100
   <211> 15
   <212> PRT
   <213> Homo sapiens
<400> 100
   His Asn Gly Ser Tyr Glu Ile Ser Val Leu Met Met Gly Asn Ser
   1               5                   10                  15
<210> 101
   <211> 15
   <212> PRT
   <213> Homo sapiens
<400> 101
   Asn Leu Pro Thr Pro Pro Thr Val Glu Asn Gln Gln Arg Leu Ala
   1               5                   10                  15
<210> 102
   <211> 619
   <212> PRT
   <213> Homo sapiens
<400> 102
   Arg Lys Tyr Arg Lys Asp Tyr Glu Leu Arg Gln Lys Lys Trp Ser His
   1               5                   10                  15
   Ile Pro Pro Glu Asn Ile Phe Pro Leu Glu Thr Asn Glu Thr Asn His
               20                  25                  30
   Val Ser Leu Lys Ile Asp Asp Asp Lys Arg Arg Asp Thr Ile Gln Arg
           35                  40                  45
   Leu Arg Gln Cys Lys Tyr Asp Lys Lys Arg Val Ile Leu Lys Asp Leu
       50                  55                  60
   Lys His Asn Asp Gly Asn Phe Thr Glu Lys Gln Lys Ile Glu Leu Asn
   65                  70                  75                  80
   Lys Leu Leu Gln Ile Asp Tyr Tyr Asn Leu Thr Lys Phe Tyr Gly Thr
                   85                  90                  95
   Val Lys Leu Asp Thr Met Ile Phe Gly Val Ile Glu Tyr Cys Glu Arg
   100 105 110
   Gly Ser Leu Arg Glu Val Leu Asn Asp Thr Ile Ser Tyr Pro Asp Gly
   115 120 125
   Thr Phe Met Asp Trp Glu Phe Lys Ile Ser Val Leu Tyr Asp Ile Ala
   130 135 140
   Lys Gly Met Ser Tyr Leu His Ser Ser Lys Thr Glu Val His Gly Arg
   145 150 155 160
   Leu Lys Ser Thr Asn Cys Val Val Asp Ser Arg Met Val Val Lys Ile
   165 170 175
   Thr Asp Phe Gly Cys Asn Ser Ile Leu Pro Pro Lys Lys Asp Leu Trp
   180 185 190
   Thr Ala Pro Glu His Leu Arg Gln Ala Asn Ile Ser Gln Lys Gly Asp
   195 200 205
   Val Tyr Ser Tyr Gly Ile Ile Ala Gln Glu Ile Ile Leu Arg Lys Glu
   210 215 220
   Thr Phe Tyr Thr Leu Ser Cys Arg Asp Arg Asn Glu Lys Ile Phe Arg
   225 230 235 240
   Val Glu Asn Ser Asn Gly Met Lys Pro Phe Arg Pro Asp Leu Phe Leu
   245 250 255
   Glu Thr Ala Glu Glu Lys Glu Leu Glu Val Tyr Leu Leu Val Lys Asn
   260 265 270
   Cys Trp Glu Glu Asp Pro Glu Lys Arg Pro Asp Phe Lys Lys Ile Glu
   275 280 285
   Thr Thr Leu Ala Lys Ile Phe Gly Leu Phe His Asp Gln Lys Asn Glu
   290 295 300
   Ser Tyr Met Asp Thr Leu Ile Arg Arg Leu Gln Leu Tyr Ser Arg Asn
   305 310 315 320
   Leu Glu His Leu Val Glu Glu Arg Thr Gln Leu Tyr Lys Ala Glu Arg
   325 330 335
   Asp Arg Ala Asp Arg Leu Asn Phe Met Leu Leu Pro Arg Leu Val Val
   340 345 350
   Lys Ser Leu Lys Glu Lys Gly Phe Val Glu Pro Glu Leu Tyr Glu Glu
           355                 360                 365
   Val Thr Ile Tyr Phe Ser Asp Ile Val Gly Phe Thr Thr Ile Cys Lys
       370                 375                 380
   Tyr Ser Thr Pro Met Glu Val Val Asp Met Leu Asn Asp Ile Tyr Lys
   385                 390                 395                 400
   Ser Phe Asp His Ile Val Asp His His Asp Val Tyr Lys Val Glu Thr
                   405                 410                 415
   Ile Gly Asp Ala Tyr Met Val Ala Ser Gly Leu Pro Lys Arg Asn Gly
               420                 425                 430
   Asn Arg His Ala Ile Asp Ile Ala Lys Met Ala Leu Glu Ile Leu Ser
           435                 440                 445
   Phe Met Gly Thr Phe Glu Leu Glu His Leu Pro Gly Leu Pro Ile Trp
       450                 455                 460
   Ile Arg Ile Gly Val His Ser Gly Pro Cys Ala Ala Gly Val Val Gly
   465                 470                 475                 480
   Ile Lys Met Pro Arg Tyr Cys Leu Phe Gly Asp Thr Val Asn Thr Ala
                   485                 490                 495
   Ser Arg Met Glu Ser Thr Gly Leu Pro Leu Arg Ile His Val Ser Gly
               500                 505                 510
   Ser Thr Ile Ala Ile Leu Lys Arg Thr Glu Cys Gln Phe Leu Tyr Glu
           515                 520                 525
   Val Arg Gly Glu Thr Tyr Leu Lys Gly Arg Gly Asn Glu Thr Thr Tyr
       530                 535                 540
   Trp Leu Thr Gly Met Lys Asp Gln Lys Phe Asn Leu Pro Thr Pro Pro
   545                 550                 555                 560
   Thr Val Glu Asn Gln Gln Arg Leu Gln Ala Glu Phe Ser Asp Met Ile
                   565                 570                 575
   Ala Asn Ser Leu Gln Lys Arg Gln Ala Ala Gly Ile Arg Ser Gln Lys
               580                 585                 590
   Pro Arg Arg Val Ala Ser Tyr Lys Lys Gly Thr Leu Glu Tyr Leu Gln
           595                 600                 605
   Leu Asn Thr Thr Asp Lys Glu Ser Thr Tyr Phe
   610 615
<210> 103
   <211> 20
   <212> DNA
   <213> künstliche Sequenz
<220>
   <223> Beschreibung der künstlichen Sequenz: Oligonukleotid
<400> 103
   gctggtaact atcttcctgc 20
<210> 104
   <211> 20
   <212> DNA
   <213> künstliche Sequenz
<220>
   <223> Beschreibung der künstlichen Sequenz: Oligonukleotid
<400> 104
   gaagaatgtt gtccagaggt 20
<210> 105
   <211> 15
   <212> PRT
   <213> Homo sapiens
<400> 105
   Leu Ile Asn Lys Val Pro Leu Pro Val Asp Lys Leu Ala Pro Leu
   1 5 10 15
<210> 106
   <211> 15
   <212> **PRT**
   <213> Homo sapiens
<400> 106
   Ser Glu Ala Val Lys Lys Leu Leu Glu Ala Leu Ser His Leu Val
   1 5 10 15
<210> 107
   <211> 20
   <212> DNA
   <213> künstliche Sequenz
<220>
   <223> Beschreibung der künstlichen Sequenz: Oligonukleotid
<400> 107
   tgttttcaac taccaggggc 20
<210> 108
   <211> 20
   <212> DNA
   <213> künstliche Sequenz
<220>
   <223> Beschreibung der künstlichen Sequenz: Oligonukleotid
<400> 108
   tgttggcttt ggcagagtcc 20
<210> 109
   <211> 24
   <212> DNA
   <213> künstliche Sequenz
<220>
   <223> Beschreibung der künstlichen Sequenz: Oligonukleotid
<400> 109
   gaggcagagt tcaggcttca ccga 24
<210> 110
   <211> 20
   <212> DNA
   <213> künstliche Sequenz
<220>
   <223> Beschreibung der künstlichen Sequenz: Oligonukleotid
<400> 110
   tgttggcttt ggcagagtcc 20
<210> 111
   <211> 56
   <212> PRT
   <213> Homo sapiens
<400> 111
   Thr Gly Met Asp Met Trp Ser Thr Gln Asp Leu Tyr Asp Asn Pro Val
   1               5                   10                  15
   Thr Ser Val Phe Gln Tyr Glu Gly Leu Trp Arg Ser Cys Val Arg Gln
               20                  25                  30
   Ser Ser Gly Phe Thr Glu Cys Arg Pro Tyr Phe Thr Ile Leu Gly Leu
           35                  40                  45
   Pro Ala Met Leu Gln Ala Val Arg
       50                  55
<210> 112
   <211> 53
   <212> PRT
   <213> Homo sapiens
<400> 112
   Asp Gln Trp Ser Thr Gln Asp Leu Tyr Asn Asn Pro Val Thr Ala Val
   1               5                   10                  15
   Phe Asn Tyr Gln Gly Leu Trp Arg Ser Cys Val Arg Glu Ser Ser Gly
               20                  25                  30
   Phe Thr Glu Cys Arg Gly Tyr Phe Thr Leu Leu Gly Leu Pro Ala Met
           35                  40                  45
   Leu Gln Ala Val Arg
       50
<210> 113
   <211> 14
   <212> PRT
   <213> Homo sapiens
<400> 113
   Ser Thr Gln Asp Leu Tyr Asn Asn Pro Val Thr Ala Val Phe
   1               5                   10
<210> 114
   <211> 12
   <212> PRT
   <213> Homo sapiens
<400> 114
   Asp Met Trp Ser Thr Gln Asp Leu Tyr Asp Asn Pro
   1               5                   10
<210> 115
   <211> 12
   <212> PRT
   <213> Homo sapiens
<400> 115
   Cys Arg Pro Tyr Phe Thr Ile Leu Gly Leu Pro Ala
   1               5                   10
<210> 116
   <211> 13
   <212> PRT
   <213> Homo sapiens
<400> 116
   Thr Asn Phe Trp Met Ser Thr Ala Asn Met Tyr Thr Gly
   1               5                   10
<210> 117
   <211> 816
   <212> DNA
   <213> Homo sapiens
<400> 117
   gccaggatca tgtccaccac cacatgccaa gtggtggcgt tcctcctgtc catcctgggg      60
   ctggccggct gcatcgcggc caccgggatg gacatgtgga gcacccagga cctgtacgac     120
   aaccccgtca cctccgtgtt ccagtacgaa gggctctgga ggagctgcgt gaggcagagt     180
   tcaggcttca ccgaatgcag gccctatttc accatcctgg gacttccagc catgctgcag     240
   gcagtgcgag ccctgatgat cgtaggcatc gtcctgggtg ccattggcct cctggtatcc     300
   atctttgccc tgaaatgcat ccgcattggc agcatggagg actctgccaa agccaacatg     360
   acactgacct ccgggatcat gttcattgtc tcaggtcttt gtgcaattgc tggagtgtct     420
   gtgtttgcca acatgctggt gactaacttc tggatgtcca cagctaacat gtacaccggc     480
   atgggtggga tggtgcagac tgttcagacc aggtacacat ttggtgcggc tctgttcgtg     540
   ggctgggtcg ctggaggcct cacactaatt gggggtgtga tgatgtgcat cgcctgccgg     600
   ggcctggcac cagaagaaac caactacaaa gccgtttctt atcatgcctc aggccacagt     660
   gttgcctaca agcctggagg cttcaaggcc agcactggct ttgggtccaa caccaaaaac     720
   aagaagatat acgatggagg tgcccgcaca gaggacgagg tacaatctta tccttccaag     780
   cacgactatg tgtaatgctc taagacctct cagcac                               816
<210> 118
   <211> 261
   <212> PRT
   <213> Homo sapiens
<400> 118
   Met Ser Thr Thr Thr Cys Gln Val Val Ala Phe Leu Leu Ser Ile Leu
   1               5                   10                  15
   Gly Leu Ala Gly Cys Ile Ala Ala Thr Gly Met Asp Met Trp Ser Thr
               20                  25                  30
   Gln Asp Leu Tyr Asp Asn Pro Val Thr Ser Val Phe Gln Tyr Glu Gly
           35                  40                  45
   Leu Trp Arg Ser Cys Val Arg Gln Ser Ser Gly Phe Thr Glu Cys Arg
       50                  55                  60
   Pro Tyr Phe Thr Ile Leu Gly Leu Pro Ala Met Leu Gln Ala Val Arg
   65                  70                  75                  80
   Ala Leu Met Ile Val Gly Ile Val Leu Gly Ala Ile Gly Leu Leu Val
   85 90 95
   Ser Ile Phe Ala Leu Lys Cys Ile Arg Ile Gly Ser Met Glu Asp Ser
   100 105 110
   Ala Lys Ala Asn Met Thr Leu Thr Ser Gly Ile Met Phe Ile Val Ser
   115 120 125
   Gly Leu Cys Ala Ile Ala Gly Val Ser Val Phe Ala Asn Met Leu Val
   130 135 140
   Thr Asn Phe Trp Met Ser Thr Ala Asn Met Tyr Thr Gly Met Gly Gly
   145 150 155 160
   Met Val Gln Thr Val Gln Thr Arg Tyr Thr Phe Gly Ala Ala Leu Phe
   165 170 175
   Val Gly Trp Val Ala Gly Gly Leu Thr Leu Ile Gly Gly Val Met Met
   180 185 190
   Cys Ile Ala Cys Arg Gly Leu Ala Pro Glu Glu Thr Asn Tyr Lys Ala
   195 200 205
   Val Ser Tyr His Ala Ser Gly His Ser Val Ala Tyr Lys Pro Gly Gly
   210 215 220
   Phe Lys Ala Ser Thr Gly Phe Gly Ser Asn Thr Lys Asn Lys Lys Ile
   225 230 235 240
   Tyr Asp Gly Gly Ala Arg Thr Glu Asp Glu Val Gln Ser Tyr Pro Ser
   245 250 255
   Lys His Asp Tyr Val
   260
<210> 119
   <211> 227
   <212> DNA
   <213> Homo sapiens
<400> 119
   gccaggatca tgtccaccac cacatgccaa gtggtggcgt tcctcctgtc catcctgggg 60
   ctggccggct gcatcgcggc caccgggatg gacatgtgga gcacccagga cctgtacgac 120
   aaccccgtca cctccgtgtt ccagtacgaa gggctctgga ggagctgcgt gaggcagagt 180
   tcaggcttca ccgaatgcag gccctatttc accatcctgg gacttcc 227
<210> 120
   <211> 69
   <212> PRT
   <213> Homo sapiens
<400> 120
   Met Ser Thr Thr Thr Cys Gln Val Val Ala Phe Leu Leu Ser Ile Leu
   1               5                   10                  15
   Gly Leu Ala Gly Cys Ile Ala Ala Thr Gly Met Asp Met Trp Ser Thr
               20                  25                  30
   Gln Asp Leu Tyr Asp Asn Pro Val Thr Ser Val Phe Gln Tyr Glu Gly
           35                  40                  45
   Leu Trp Arg Ser Cys Val Arg Gln Ser Ser Gly Phe Thr Glu Cys Arg
       50                  55                  60
   Pro Tyr Phe Thr Ile
   65
<210> 121
   <211> 20
   <212> DNA
   <213> künstliche Sequenz
<220>
   <223> Beschreibung der künstlichen Sequenz: Oligonukleotid
<400> 121
   aatgagagga aagagaaaac 20
<210> 122
   <211> 20
   <212> DNA
   <213> künstliche Sequenz
<220>
   <223> Beschreibung der künstlichen Sequenz: Oligonukleotid
<400> 122
   atggtagaag agtaggcaat 20
<210> 123
   <211> 15
   <212> PRT
   <213> Homo sapiens
<400> 123
   Glu Lys Trp Asn Leu His Lys Arg Ile Ala Leu Lys Met Val Cys
   1               5                   10                  15
<210> 124
   <211> 11
   <212> PRT
   <213> Homo sapiens
<400> 124
   Cys Leu Gly Phe Asn Phe Lys Glu Met Phe Lys
   1               5                   10
<210> 125
   <211> 23
   <212> DNA
   <213> künstliche Sequenz
<220>
   <223> Beschreibung der künstlichen Sequenz: Oligonukleotid
<400> 125
   taatgatgaa ccctacactg agc 23
<210> 126
   <211> 20
   <212> DNA
   <213> künstliche Sequenz
<220>
   <223> Beschreibung der künstlichen Sequenz: Oligonukleotid
<400> 126
   atggacaaat gccctacctt 20
<210> 127
   <211> 22
   <212> DNA
   <213> künstliche Sequenz
<220>
   <223> Beschreibung der künstlichen Sequenz: Oligonukleotid
<400> 127
   agtgctggaa ggatgtgcgt gt 22
<210> 128
   <211> 20
   <212> DNA
   <213> künstliche Sequenz
<220>
   <223> Beschreibung der künstlichen Sequenz: Oligonukleotid
<400> 128
   ttgaggtggt tgttgggttt 20
<210> 129
   <211> 20
   <212> DNA
   <213> künstliche Sequenz
<220>
   <223> Beschreibung der künstlichen Sequenz: Oligonukleotid
<400> 129
   agatgtgctg aggctgtaga 20
<210> 130
   <211> 20
   <212> DNA
   <213> künstliche Sequenz
<220>
   <223> Beschreibung der künstlichen Sequenz: Oligonukleotid
<400> 130
   atgaaggttg attatttgag 20
<210> 131
   <211> 23
   <212> DNA
   <213> künstliche Sequenz
<220>
   <223> Beschreibung der künstlichen Sequenz: Oligonukleotid
<400> 131
   agccgcatac tcccttaccc tct 23
<210> 132
   <211> 20
   <212> DNA
   <213> künstliche Sequenz
<220>
   <223> Beschreibung der künstlichen Sequenz: Oligonukleotid
<400> 132
   gcagcagccc aaacaccaca 20
<210> 133
   <211> 20
   <212> DNA
   <213> künstliche Sequenz
<220>
   <223> Beschreibung der künstlichen Sequenz: Oligonukleotid
<400> 133
   ctgagccgag aggtggaatc 20
<210> 134
   <211> 20
   <212> DNA
   <213> künstliche Sequenz
<220>
   <223> Beschreibung der künstlichen Sequenz: Oligonukleotid
<400> 134
   ctctctcgct tacactggaa 20
<210> 135
   <211> 14
   <212> PRT
   <213> Homo sapiens
<400> 135
   Gln Trp Gln Val Phe Gly Pro Asp Lys Pro Val Gln Ala Leu
   1 5 10
<210> 136
   <211> 15
   <212> PRT
   <213> Homo sapiens
<400> 136
   Ala Lys Trp Lys Gly Pro Gln Gly Gln Asp Leu Ser Thr Asp Ser
   1 5 10 15
<210> 137
   <211> 32
   <212> PRT
   <213> Homo sapiens
<400> 137
   Asn Met Leu Val Thr Asn Phe Trp Met Ser Thr Ala Asn Met Tyr Thr
   1 5 10 15
   Gly Met Gly Gly Met Val Gln Thr Val Gln Thr Arg Tyr Thr Phe Gly
   20 25 30
<210> 138
   <211> 2052
   <212> DNA
   <213> Homo sapiens
<400> 138
   gacagctgtg tctcgatgga gtagactctc agaacagcgc agtttgccct ccgctcacgc 60
   agagcctctc cgtggcttcc gcaccttgag cattaggcca gttctcctct tctctctaat 120
   ccatccgtca cctctcctgt catccgtttc catgccgtga ggtccattca cagaacacat 180
   ccatggctct catgctcagt ttggttctga gtctcctcaa gctgggatca gggcagtggc 240
   aggtgtttgg gccagacaag cctgtccagg ccttggtggg ggaggacgca gcattctcct 300
   gtttcctgtc tcctaagacc aatgcagagg ccatggaagt gcggttcttc aggggccagt 360
   tctctagcgt ggtccacctc tacagggacg ggaaggacca gccatttatg cagatgccac 420
   agtatcaagg caggacaaaa ctggtgaagg attctattgc ggaggggcgc atctctctga 480
   ggctggaaaa cattactgtg ttggatgctg gcctctatgg gtgcaggatt agttcccagt     540
   cttactacca gaaggccatc tgggagctac aggtgtcagc actgggctca gttcctctca     600
   tttccatcac gggatatgtt gatagagaca tccagctact ctgtcagtcc tcgggctggt     660
   tcccccggcc cacagcgaag tggaaaggtc cacaaggaca ggatttgtcc acagactcca     720
   ggacaaacag agacatgcat ggcctgtttg atgtggagat ctctctgacc gtccaagaga     780
   acgccgggag catatcctgt tccatgcggc atgctcatct gagccgagag gtggaatcca     840
   gggtacagat aggagatacc tttttcgagc ctatatcgtg gcacctggct accaaagtac     900
   tgggaatact ctgctgtggc ctattttttg gcattgttgg actgaagatt ttcttctcca     960
   aattccagtg gaaaatccag gcggaactgg actggagaag aaagcacgga caggcagaat    1020
   tgagagacgc ccggaaacac gcagtggagg tgactctgga tccagagacg gctcacccga    1080
   agctctgcgt ttctgatctg aaaactgtaa cccatagaaa agctccccag gaggtgcctc    1140
   actctgagaa gagatttaca aggaagagtg tggtggcttc tcagagtttc caagcaggga    1200
   aacattactg ggaggtggac ggaggacaca ataaaaggtg gcgcgtggga gtgtgccggg    1260
   atgatgtgga caggaggaag gagtacgtga ctttgtctcc cgatcatggg tactgggtcc    1320
   tcagactgaa tggagaacat ttgtatttca cattaaatcc ccgttttatc agcgtcttcc    1380
   ccaggacccc acctacaaaa ataggggtct tcctggacta tgagtgtggg accatctcct    1440
   tcttcaacat aaatgaccag tcccttattt ataccctgac atgtcggttt gaaggcttat    1500
   tgaggcccta cattgagtat ccgtcctata atgagcaaaa tggaactccc atagtcatct    1560
   gcccagtcac ccaggaatca gagaaagagg cctcttggca aagggcctct gcaatcccag    1620
   agacaagcaa cagtgagtcc tcctcacagg caaccacgcc cttcctcccc aggggtgaaa    1680
   tgtaggatga atcacatccc acattcttct ttagggatat taaggtctct ctcccagatc    1740
   caaagtcccg cagcagccgg ccaaggtggc ttccagatga agggggactg gcctgtccac    1800
   atgggagtca ggtgtcatgg ctgccctgag ctgggaggga agaaggctga cattacattt    1860
   agtttgctct cactccatct ggctaagtga tcttgaaata ccacctctca ggtgaagaac    1920
   cgtcaggaat tcccatctca caggctgtgg tgtagattaa gtagacaagg aatgtgaata    1980
   atgcttagat cttattgatg acagagtgta tcctaatggt ttgttcatta tattacactt    2040
   tcagtaaaaa aa                                                        2052
<210> 139
   <211> 500
   <212> PRT
   <213> Homo sapiens
<400> 139
   Met Ala Leu Met Leu Ser Leu Val Leu Ser Leu Leu Lys Leu Gly Ser
   1               5                   10                  15
   Gly Gln Trp Gln Val Phe Gly Pro Asp Lys Pro Val Gln Ala Leu Val
               20                  25                  30
   Gly Glu Asp Ala Ala Phe Ser Cys Phe Leu Ser Pro Lys Thr Asn Ala
           35                  40                  45
   Glu Ala Met Glu Val Arg Phe Phe Arg Gly Gln Phe Ser Ser Val Val
       50                  55                  60
   His Leu Tyr Arg Asp Gly Lys Asp Gln Pro Phe Met Gln Met Pro Gln
   65                  70                  75                  80
   Tyr Gln Gly Arg Thr Lys Leu Val Lys Asp Ser Ile Ala Glu Gly Arg
                   85                  90                  95
   Ile Ser Leu Arg Leu Glu Asn Ile Thr Val Leu Asp Ala Gly Leu Tyr
               100                 105                 110
   Gly Cys Arg Ile Ser Ser Gln Ser Tyr Tyr Gln Lys Ala Ile Trp Glu
           115                 120                 125
   Leu Gln Val Ser Ala Leu Gly Ser Val Pro Leu Ile Ser Ile Thr Gly
       130                 135                 140
   Tyr Val Asp Arg Asp Ile Gln Leu Leu Cys Gln Ser Ser Gly Trp Phe
   145                 150                 155                 160
   Pro Arg Pro Thr Ala Lys Trp Lys Gly Pro Gln Gly Gln Asp Leu Ser
                   165                 170                 175
   Thr Asp Ser Arg Thr Asn Arg Asp Met His Gly Leu Phe Asp Val Glu
               180                 185                 190
   Ile Ser Leu Thr Val Gln Glu Asn Ala Gly Ser Ile Ser Cys Ser Met
           195                 200                 205
   Arg His Ala His Leu Ser Arg Glu Val Glu Ser Arg Val Gln Ile Gly
       210                 215                 220
   Asp Thr Phe Phe Glu Pro Ile Ser Trp His Leu Ala Thr Lys Val Leu
   225                 230                 235                 240
   Gly Ile Leu Cys Cys Gly Leu Phe Phe Gly Ile Val Gly Leu Lys Ile
                   245                 250                 255
   Phe Phe Ser Lys Phe Gln Trp Lys Ile Gln Ala Glu Leu Asp Trp Arg
   260 265 270
   Arg Lys His Gly Gln Ala Glu Leu Arg Asp Ala Arg Lys His Ala Val
   275 280 285
   Glu Val Thr Leu Asp Pro Glu Thr Ala His Pro Lys Leu Cys Val Ser
   290 295 300
   Asp Leu Lys Thr Val Thr His Arg Lys Ala Pro Gln Glu Val Pro His
   305 310 315 320
   Ser Glu Lys Arg Phe Thr Arg Lys Ser Val Val Ala Ser Gln Ser Phe
   325 330 335
   Gln Ala Gly Lys His Tyr Trp Glu Val Asp Gly Gly His Asn Lys Arg
   340 345 350
   Trp Arg Val Gly Val Cys Arg Asp Asp Val Asp Arg Arg Lys Glu Tyr
   355 360 365
   Val Thr Leu Ser Pro Asp His Gly Tyr Trp Val Leu Arg Leu Asn Gly
   370 375 380
   Glu His Leu Tyr Phe Thr Leu Asn Pro Arg Phe Ile Ser Val Phe Pro
   385 390 395 400
   Arg Thr Pro Pro Thr Lys Ile Gly Val Phe Leu Asp Tyr Glu Cys Gly
   405 410 415
   Thr Ile Ser Phe Phe Asn Ile Asn Asp Gln Ser Leu Ile Tyr Thr Leu
   420 425 430
   Thr Cys Arg Phe Glu Gly Leu Leu Arg Pro Tyr Ile Glu Tyr Pro Ser
   435 440 445
   Tyr Asn Glu Gln Asn Gly Thr Pro Ile Val Ile Cys Pro Val Thr Gln
   450 455 460
   Glu Ser Glu Lys Glu Ala Ser Trp Gln Arg Ala Ser Ala Ile Pro Glu
   465 470 475 480
   Thr Ser Asn Ser Glu Ser Ser Ser Gln Ala Thr Thr Pro Phe Leu Pro
   485 490 495
   Arg Gly Glu Met
   500
<210> 140
   <211> 21
   <212> DNA
   <213> künstliche Sequenz
<220>
   <223> Beschreibung der künstlichen Sequenz: Oligonukleotid
<400> 140
   tccaaattcc agtggaaaat c 21
<210> 141
   <211> 21
   <212> DNA
   <213> künstliche Sequenz
<220>
   <223> Beschreibung der künstlichen Sequenz: Oligonukleotid
<400> 141
   ccacactcat agtccaggaa g 21
<210> 142
   <211> 116
   <212> PRT
   <213> Homo sapiens
<400> 142
   Ala Leu Met Ile Val Gly Ile Val Leu Gly Ala Ile Gly Leu Leu Val
   1               5                   10                  15
   Ser Ile Phe Ala Leu Lys Cys Ile Arg Ile Gly Ser Met Glu Asp Ser
               20                  25                  30
   Ala Lys Ala Asn Met Thr Leu Thr Ser Gly Ile Met Phe Ile Val Ser
           35                  40                  45
   Gly Leu Cys Ala Ile Ala Gly Val Ser Val Phe Ala Asn Met Leu Val
       50                  55                  60
   Thr Asn Phe Trp Met Ser Thr Ala Asn Met Tyr Thr Gly Met Gly Gly
   65                  70                  75                  80
   Met Val Gln Thr Val Gln Thr Arg Tyr Thr Phe Gly Ala Ala Leu Phe
                   85                  90                  95
   Val Gly Trp Val Ala Gly Gly Leu Thr Leu Ile Gly Gly Val Met Met
               100                 105                 110
   Cys Ile Ala Cys
           115
<210> 143
   <211> 24
   <212> **PRT**
   <213> Homo sapiens
<400> 143
   Arg Ile Gly Ser Met Glu Asp Ser Ala Lys Ala Asn Met Thr Leu Thr
   1               5                   10                  15
   Ser Gly Ile Met Phe Ile Val Ser
               20
<210> 144
   <211> 8
   <212> **PRT**
   <213> Homo sapiens
<400> 144
   Ala Lys Ala Asn Met Thr Leu Thr
   1               5
<210> 145
   <211> 14
   <212> **PRT**
   <213> Homo sapiens
<400> 145
   Met Glu Asp Ser Ala Lys Ala Asn Met Thr Leu Thr Ser Gly
   1               5                   10
<210> 146
   <211> 14
   <212> **PRT**
   <213> Homo sapiens
<400> 146
   Met Glu Asp Ser Ala Lys Ala Asp Met Thr Leu Thr Ser Gly
   1               5                   10
<210> 147
   <211> 9
   <212> **PRT**
   <213> Homo sapiens
<400> 147
   Ser Ala Lys Ala Asp Met Thr Leu Thr
   1               5
<210> 148
   <211> 9
   <212> **PRT**
   <213> Homo sapiens
<400> 148
   Ala Lys Ala Asp Met Thr Leu Thr Leu
   1               5
<210> 149
   <211> 53
   <212> PRT
   <213> Homo sapiens
<400> 149
   Asp Gln Trp Ser Thr Gln Asp Leu Tyr Asp Asn Pro Val Thr Ala Val
   1               5                   10                  15
   Phe Asn Tyr Gln Gly Leu Trp Arg Ser Cys Val Arg Glu Ser Ser Gly
               20                  25                  30
   Phe Thr Glu Cys Arg Gly Tyr Phe Thr Leu Leu Gly Leu Pro Ala Met
           35                  40                  45
   Leu Gln Ala Val Arg
       50
<210> 150
   <211> 14
   <212> **PRT**
   <213> Homo sapiens
<400> 150
   Ser Thr Gln Asp Leu Tyr Asp Asn Pro Val Thr Ala Val Phe
   1               5                   10

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen. An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC-(Major Histocompatibility complex = Histokompatibilitätsantigene) Klassen II bzw. I präsentiert werden, während B-Lymphozyten zirkulierende Antikörpermoleküle produzieren, die direkt an unprozessierte Antigene binden. Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumorspezifischem Lysemuster oder zirkulierende Autoantikörper, wurden genutzt, um die respektiven Antigene zu klonieren.

Durch diese Ansätze sind in den letzten Jahren eine Vielzahl von Antigenen in verschiedenen Neoplasien definiert worden. Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumoren z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Offenbarungsgemäß wurde eine Strategie für eine Identifizierung und Bereitstellung Tumor-assoziiert exprimierter Antigene und der dafür kodierenden Nukleinsäuren verfolgt. Diese Strategie beruht auf der Tatsache, dass bestimmte Gene, die Organ-spezifisch, z.B. ausschließlich im Kolon-, Lungen- oder Nieren-Gewebe, exprimiert werden, in den entsprechenden Organen auch von Tumorzellen und darüber hinaus in anderen Geweben in Tumorzellen ektop und unerlaubt reaktiviert werden. Durch Datamining wird zunächst eine möglichst komplette Liste aller bekannten Organ-spezifischen Gene aufgestellt und diese sodann durch Expressionsanalysen mittels spezifischer RT-PCR auf ihre aberrante Aktivierung in unterschiedlichen Tumoren evaluiert. Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumorspezifisch sind (Schmitt et al., Nucleic Acids Res. 27:4251-60, 1999; Vasmatzis et al., Proc. Natl. Acad. Sci. U S A. 95:300-4, 1998; Scheurle et al., Cancer Res. 60:4037-43, 2000).

Das offenbarnngsgemäße Konzept, das sich als viel erfolgreicher erwiesen hat, beruht jedoch darauf, Datamining zur elektronischen Extraktion aller Organ-spezifischer Gene zu nutzen und diese sodann auf Expression in Tumoren zu evaluieren.

Somit betrifft die vorliegende Lehre in einem Aspekt eine Strategie zur Identifizierung von Gewebe-spezifischen und differentiell in Tumoren exprimierten Genen. Diese Strategie kombiniert Datamining von öffentlichen Sequenzbanken ("*in silico*") mit darauf folgenden evaluierenden labor-experimentellen ("wet bench") Untersuchungen.

Eine kombinierte Strategie basierend auf zwei unterschiedlichen bioinformatischen Skripten ermöglichte erfindungsgemäß die Identifizierung neuer Tumor-Gene. Diese sind bisher als rein Organ-spezifisch eingestuft worden. Die Erkenntnis, dass diese Gene aberrant in Tumorzellen aktiviert werden, erlaubt, ihnen eine substantiell neue Qualität mit funktionellen Implikationen zuzuordnen. Die Identifizierung und Bereitstellung dieser Tumor-assoziierten Gene und der dadurch kodierten Genprodukte erfolgte unabhängig von einer immunogenen Wirkung.

Die identifizierten Tumor-assoziierten Antigene weisen eine Aminosäuresequenz auf, die von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: 1-8, 41-44, 51-59, 84, 117, 119 und 138, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist und (d) einer Nukleinsäure, die zu der Nukleinsäure unter (a), (b) oder (c) komplementär ist. In einer bevorzugten Ausführungsform weist ein identifiziertes Tumor-assoziiertes Antigen eine Aminosäuresequenz auf, die von einer Nukleinsäure kodiert wird, die aus der Gruppe bestehend aus SEQ ID NO: 1-8, 41-44, 51-59, 84, 117, 119 und 138 ausgewählt ist. Offenbarungsgemäß umfasst ein identifiziertes Tumor-assoziiertes Antigen eine Aminosäuresequenz, die aus der Gruppe bestehend aus SEQ ID NO: 9-19, 45-48, 60-66, 85, 90-97, 100-102, 105, 106, 111-116, 118, 120, 123, 124, 135-137, 139 und 142-150, einem Teil oder Derivat davon ausgewählt ist.

Die vorliegende Offenbarung betrifft allgemein die Verwendung von offenbarungsgemäß identifizierten Tumor-assoziierten Antigenen oder von Teilen oder Derivaten davon, von dafür kodierenden Nukleinsäuren oder von Nukleinsäuren, die gegen die kodierenden Nukleinsäuren gerichtet sind, oder von Antikörpern, die gegen die identifizierten Tumor-assoziierten Antigene oder Teile oder Derivate davon gerichtet sind, für die Therapie und Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Bevorzugte Erkrankungen für eine Therapie und/oder Diagnose sind solche, bei denen eine selektive Expression oder abnormale Expression von einem oder mehreren der offenbarungsgemäß identifizierten Tumor-assoziierten Antigene vorliegt.

Die Offenbarung betrifft auch Nukleinsäuren und Genprodukte, die tumorzellassoziiert exprimiert werden.

Desweiteren betrifft die vorligende technische Lehre Genprodukte, d.h. Nukleinsäuren und Proteine bzw. Peptide, die durch verändertes Spleißen (Spleißvarianten) bekannter Gene bzw. durch veränderte Translation unter Nutzung alternativer offener Leserahmen entstehen. In diesem Aspekt werden Nukleinsäuren offenbart, die eine Nukleinsäuresequenz ausgewählt aus der Gruppe bestehend aus den Sequenzen gemäß SEQ ID NO: 3-5 des Sequenzprotokolls umfassen. Außerdem betrifft die offenbarte Lehre in diesem Aspekt Proteine bzw. Peptide, die eine Aminosäuresequenz ausgewählt aus der Gruppe bestehend aus den Sequenzen gemäß SEQ ID NO: 10 und 12-14 des Sequenzprotokolls umfassen. Die offenbarten Spleißvarianten sind als Targets für die Diagnostik und Therapie von Tumorerkrankungen verwendbar.

Insbesondere betrifft die vorliegende Lehre die Aminosäuresequenz gemäß SEQ ID NO: 10 des Sequenzprotokolls, die durch einen erfindungsgemäß identifizierten alternativen offenen Leseraster kodiert wird und sich von der vorbeschriebenen Protein-Sequenz (SEQ ID NO: 9) durch 85 zusätzliche Aminosäuren am N-Terminus des Proteins unterscheidet.

Für die Entstehung von Spleißvarianten können verschiedenste Mechanismen ursächlich sein, beispielsweise
- die Nutzung variabler Transkriptionsinitiationsstellen,
- die Nutzung zusätzlicher Exons,
- das vollständige oder unvollständige Ausspleißen von einzelnen oder mehreren Exons,
- durch Mutation veränderte Spleißregulatorsequenzen (Deletion bzw. Schaffung neuer Donor/Acceptorsequenzen),
- die unvollständige Elimination von Intronsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei Tumor-assoziierten Spleißvarianten können Tumor-assoziierte Transkripte und Tumor-assoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion von Nukleinsäuren durch PCR-Amplifikation mit Spleißvarianten-spezifischen Oligonukleotiden erfolgen. Als Oligonukleotide eignen sich insbesondere Paare von Primern, von denen mindestens einer unter stringenten Bedingungen an die Region der Spleißvariante bindet, die Tumor-assoziiert ist. Offenbarungsgemäß geeignet sind die in den Beispielen für diesen Zweck beschriebenen Oligonukleotide, insbesondere Oligonukleotide, die eine Sequenz ausgewählt aus SEQ ID NO: 34-36, 39, 40 und 107-110 des Sequenzprotokolls aufweisen bzw. umfassen. Zum Nachweis eignen sich alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. In diesem Aspekt betrifft die offenbarte Lehre insbesondere Peptide, die eine Sequenz ausgewählt aus SEQ ID NO: 17-19, 111-115, 120 und 137 des Sequenzprotokolls aufweisen bzw. umfassen und dagegen gerichtete spezifische Antikörper. Die Detektion von Tumorzellen kann auch durch Antikörper erfolgen, die tumorspezifisch veränderte Glykosylierungsvarianten erkennen. Für die Generierung von derartigen Antikörpern können Peptidregionen genutzt werden, die sich in Tumorzellen und gesunden Zellen glykosylierungsbedingt unterscheiden. In diesem Aspekt betrifft die Offenbarung insbesondere Peptide, die eine Sequenz ausgewählt aus SEQ ID NO: 17-19, 111-115, 120, 137 und 142-145, des Sequenzprotokolls aufweisen bzw. umfassen und dagegen gerichtete spezifische Antikörper. Durch endogene Deglykosylierung von N-gekoppelten Zuckerresten wird Asparagin in Asparaginsäure transformiert. Offenbarungsgemäß können daher die hier beschriebenen Proteine tumorspezifisch sequenzverändert sein und damit andere biochemische und Antikörper-Bindungseigenschaften aufweisen. In diesem Aspekt betrifft die offenbarte Lehre insbesondere Peptide, die eine Sequenz ausgewählt aus SEQ ID NO: 146-150 des Sequenzprotokolls aufweisen bzw. umfassen und dagegen gerichtete spezifische Antikörper. Für die Immunisierung eignen sich besonders die Aminosäuren, die deutliche Epitopunterschiede zu der (den) Variante(n) des Genprodukts aufweisen, welche(s) bevorzugt in gesunden Zellen gebildet wird (werden). Der Nachweis der Tumorzellen mit Antikörpern kann dabei an einer vom Patienten isolierten Probe oder als Imaging mit intravenös applizierten Antikörpern erfolgen. Neben der diagnostischen Nutzbarkeit stellen Spleißvarianten, die neue oder veränderte Epitope aufweisen, attraktive Targets für die Immuntherapie dar. Die Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferriert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben (vgl. z.B. Kessler JH, et al. 2001, Sahin et al., 1997) und basieren ebenfalls auf der Nutzung von Oligo- oder Polypeptiden, die die Spleißvarianten-spezifischen Epitope beinhalten oder Nukleinsäuren, die für diese kodieren. Oligo- oder Polypeptiden, die die Spleißvarianten-spezifischen Epitope beinhalten, oder Nukleinsäuren, die für diese Polypeptide kodieren, sind auch als pharmazeutisch wirksame Substanzen bei der aktiven Immuntherapie (Vakzinierung, Vakzintherapie) verwendbar.

Vorliegend werden auch Proteine beschrieben, die sich durch Art und Menge ihrer sekundären Modifikationen in Normal- und Tumorgewebe unterscheiden (z.B. Durand & Seta, 2000; Clin. Chem. 46: 795-805; Hakomori, 1996; Cancer Res. 56: 5309-18).

Die Analyse von Proteinmodifikationen kann im Western-Blot erfolgen. Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western-Blot. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Von besonderem Interesse sind Proteinbereiche, die in Tumorzellen und gesunden Zellen differenziell glykosyliert sind. Derartige Glykosylierungsunterschiede sind jedoch bisher für wenige Zelloberflächenproteine (z.B. Muc1) beschrieben.

Offenbarungsgemäß konnte für Claudin-18 eine differentielle Glykosylierung in Tumoren nachgewiesen werden. Gastrointestinale Karzinome, Pankreaskarzinome, Ösophagustumoren, Prostatatumoren als auch Lungentumoren weisen eine weniger glykosylierte Form von Claudin-18 auf. Die Glykosylierung in gesunden Geweben maskiert Proteinepitope von Claudin-18, die auf Tumorzellen aufgrund fehlender Glykosylierung freigelegt sind. Entsprechend lassen sich offenbarungsgemäß Liganden und Antikörper selektieren, die an diese Domänen binden. Derartige Liganden und Antikörper binden erfindungsgemäß nicht an das Claudin-18 auf gesunden Zellen, da hier die Epitope durch die Glykosylierung verdeckt sind.

Ähnlich wie oben für von Tumor-assoziierten Spleißvarianten abgeleitete Proteinepitope beschrieben kann somit die differenzielle Glycosylierung zur Unterscheidung von Normal- und Tumorzellen mit diagnostischer wie auch therapeutischer Intention genutzt werden.

In einem Aspekt betrifft die Offenbarung eine pharmazeutische Zusammensetzung umfassend ein Mittel, das das offenbarungsgemäß identifizierte Tumor-assoziierte Antigen erkennt und vorzugsweise selektiv für Zellen ist, die eine Expression oder abnormale Expression eines offenbarungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf. In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter oder Toxin-konjugierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein offenbarungsgemäß identifiziertes Tumor-assoziiertes Antigen ist. Die Erkennung muss nicht direkt mit einer Hemmung von Aktivität oder Expression des Antigens einhergehen. In diesem Aspekt der offenbarten technischen Lehre dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zellen lysiert. In einer weiteren Ausführungsform ist das Mittel ein Antikörper, der selektiv an das Tumor-assoziierte Antigen bindet und somit natürliche oder artifizielle Effektormechanismen zu dieser Zelle rekrutiert. In einer weiteren Ausführungsform ist das Mittel ein T-Helfer-Lymphozyt, der Effektorfunktionen von anderen Zellen, die spezifisch dieses Antigen erkennen, stärkt.

In einem Aspekt betrifft die offenbarte Lehre eine pharmazeutische Zusammensetzung umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausführungsform umfasst das Mittel mehrere Mittel, die jeweils selektiv die Expression oder Aktivität verschiedener Tumor-assoziierter Antigene hemmen, wobei mindestens eines der Tumor-assoziierten Antigene ein offenbarungsgemäß identifiziertes Tumor-assoziiertes Antigen ist.

Des weiteren betrifft die offenbarte Lehre eine pharmazeutische Zusammensetzung, die ein Mittel umfasst, das bei einer Verabreichung selektiv die Menge an Komplexen zwischen einem HLA-Molekül und einem Peptidepitop aus dem offenbarungsgemäß identifizierten Tumor-assoziierten Antigen erhöht. Das Mittel umfasst in einer Ausführungsform einen oder mehrere Bestandteile, die aus der Gruppe ausgewählt sind, bestehend aus (i) dem Tumor-assoziierten Antigen oder einem Teil davon, (ii) einer Nukleinsäure, die für das Tumor-assoziierte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführungsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein offenbarungsgemäß identifiziertes Tumor-assoziiertes Antigen ist.

Des weiteren betrifft die offenbarte Lehre eine pharmazeutische Zusammensetzung, die einen oder mehrer Bestandteile umfasst, die aus der Gruppe ausgewählt sind, bestehend aus (i) einem offenbarungsgemäß identifizierten Tumor-assoziierten Antigen oder einem Teil davon, (ii) einer Nukleinsäure, die für ein offenbarungsgemäß identifiziertes Tumor-assoziiertes Antigen oder einen Teil davon kodiert, (iii) einem Antikörper, der an ein offenbarungsgemäß identifiziertes Tumor-assoziiertes Antigen oder einen Teil davon bindet, (iv) einer Antisense-Nukleinsäure, die spezifisch mit einer Nukleinsäure, die für ein offenbarungsgemäß identifiziertes Tumor-assoziiertes Antigen kodiert, hybridisiert, (v) einer Wirtszelle, die ein offenbarungsgemäß identifiziertes Tumor-assoziiertes Antigen oder einen Teil davon exprimiert, und (vi) isolierten Komplexen zwischen einem offenbarungsgemäß identifizierten Tumor-assoziierten Antigen oder einem Teil davon und einem HLA-Molekül.

Eine Nukleinsäure, die für ein offenbarungsgemäß identifiziertes Tumor-assoziiertes Antigen oder einen Teil davon kodiert, kann in der pharmazeutische Zusammensetzung in einem Expressionsvektor vorliegen und funktionell mit einem Promotor verbunden sein.

Eine in einer pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers, oder ein synthetischer Antikörper, die alle durch kombinatorische Techniken hergestellt werden können. Der Antikörper kann mit einem therapeutisch oder diagnostisch nützlichen Mittel oder Stoff gekoppelt sein.

Eine in einer pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das offenbarungsgemäß identifizierte Tumor-assoziierte Antigen kodiert, umfassen.

In weiteren Ausführungsformen bindet ein durch eine offenbarungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Cytokinausschüttung induziert.

Eine pharmazeutische Zusammensetzung kann einen pharmazeutisch verträglichen Träger und/oder ein Adjuvans umfassen. Das Adjuvans kann aus Saponin, GM-CSF, CpG-Nukleotiden, RNA, einem Cytokin oder einem Chemokin ausgewählt sein. Eine pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des weiteren betrifft die offenbarte Lehre Verfahren zur Behandlung, Diagnose und/oder Überwachung einer Erkrankung, die sich durch die Expression oder abnormale Expression eines oder mehrerer Tumor-assoziierter Antigene auszeichnet. In einer Ausführungsform umfasst die Behandlung die Verabreichung einer pharmazeutischen Zusammensetzung. Vorzugsweise ist die Erkrankung Krebs, wobei der Begriff "Krebs" in nicht-begrenzender Weise Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

In einem Aspekt betrifft die Offenbarung ein Verfahren zur Diagnose einer Erkrankung, die sich durch die Expression oder abnormale Expression eines offenbarungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

In einem weiteren Aspekt betrifft die offenbarte Lehre ein Verfahren zur Bestimmung der Regression, des Verlaufs oder des Ausbruchs einer Erkrankung, die sich durch die Expression oder abnormale Expression eines offenbarungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind, bestehend aus (i) der Menge der Nukleinsäure, die für das Tumor-assoziierte Antigen kodiert, oder eines Teil davon, (ii) der Menge des Tumor-assoziierten Antigens oder eines Teils davon, (iii) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben der Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In bestimmten Ausführungsformen liegt das nachzuweisende Tumor-assoziierte Antigen oder der Teil davon intrazellulär oder auf der Zelloberfläche vor. Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In weiteren Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, wodurch spezifische T-Lymphozyten identifiziert werden können.

In einem weiteren Aspekt betrifft die offenbarte Lehre ein Verfahren zur Behandlung, Diagnose oder Überwachung einer Erkrankung, die sich durch die Expression oder abnormale Expression eines offenbarungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel oder Stoff gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

In manchen Ausführungsformen erfolgen die offenbarten Verfahren zur Diagnose oder Überwachung einer Erkrankung, die sich durch die Expression oder abnormale Expression eines offenbarungsgemäß identifizierten Tumor-assoziierten Antigens auszeichnet, mit Hilfe von oder durch Nachweis von disseminierenden Tumorzellen oder von Tumormetastasen. Ein Nachweis disseminierender Tumorzellen kann beispielsweise im Blut, Serum, Knochenmark, Sputum, Bronchialaspirat und/oder Bronchiallavage erfolgen.

Die offenbarte Lehre betrifft auch ein Verfahren zur Behandlung eines Patienten mit einer Erkrankung, die sich durch die Expression oder abnormale Expression eines offenbarungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (iii) das Einbringen der cytolytischen T-Zellen in den Patienten in einer Menge, die geeignet ist, Zellen zu lysieren, die das Tumor-assoziierte Antigen oder einen Teil davon exprimieren. Die vorliegende Lehre betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die vorliegende Offenbarung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat), und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die offenbarte Lehre betrifft auch ein Verfahren zur Behandlung einer Erkrankung, die sich durch die Expression oder abnormale Expression eines offenbarungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die offenbarungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Offenbarung eine Nukleinsäure, die aus der Gruppe ausgewählt ist, bestehend aus (a) einer Nukleinsäure, die eine Nukleinsäuresequenz umfasst, die aus der Gruppe bestehend aus SEQ ID NO: 3-5, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. Des weiteren betrifft die Offenbarung eine Nukleinsäure, die für ein Protein oder Polypeptid kodiert, das eine Aminosäuresequenz umfasst, ausgewählt aus der Gruppe bestehend aus SEQ ID NO: 10, 12-14 und 146-150, einem Teil oder Derivat davon.

In einem weiteren Aspekt betrifft die offenbarte Lehre Promotorsequenzen von offenbarungsgemäßen Nukleinsäuren. Diese können funktionell mit einem anderen Gen vorzugsweise in einem Expressionsvektor verbunden werden und somit die selektive Expression dieses Gens in entsprechenden Zellen gewährleisten.

In einem weiteren Aspekt betrifft die Erfindung ein rekombinantes Nukleinsäuremolekül, insbesondere DNA- oder RNA-Molekül, das eine offenbarungsgemäße Nukleinsäure umfasst.

Die vorliegende Lehre betrifft auch Wirtszellen, die eine offenbarungsgemäße Nukleinsäure oder ein rekombinantes Nukleinsäuremolekül, das eine offenbarungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die offenbarungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die offenbarte Lehre Oligonukleotide, die mit einer offenbarungsgemäß identifizierten Nukleinsäure hybridisieren und als genetische Sonden oder als "Antisense"-Moleküle verwendet werden können. Nukleinsäuremoleküle in der Form von Oligonukleotid-Primem oder kompetenten Proben, die mit einer offenbarungsgemäß identifizierten Nukleinsäure oder Teilen davon hybridisieren, können zum Auffinden von Nukleinsäuren verwendet werden, die zu der offenbarungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Offenbarung ein Protein, Polypeptid oder Peptid, das von einer Nukleinsäure kodiert wird, die aus der Gruppe ausgewählt ist, bestehend aus (a) einer Nukleinsäure, die eine Nukleinsäuresequenz umfasst, die aus der Gruppe bestehend aus SEQ ID NO: 3-5, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform betrifft die Offenbarung ein Protein oder Polypeptid oder Peptid, das eine Aminosäuresequenz umfasst, ausgewählt aus der Gruppe bestehend aus SEQ ID NO: 10, 12-14 und 146-150, einem Teil oder Derivat davon.

In einem weiteren Aspekt betrifft die offenbarte Lehre ein immunogenes Fragment eines offenbarungsgemäß identifizierten Tumor-assoziierten Antigens. Das Fragment bindet vorzugsweise an einen menschlichen HLA-Rezeptor oder menschlichen Antikörper. Vorzugsweise umfasst ein offenbarungsgemäßes Fragment eine Sequenz von mindestens 6, insbesondere mindestens 8, mindestens 10, mindestens 12, mindestens 15, mindestens 20, mindestens 30 oder mindestens 50 Aminosäuren.

In diesem Aspekt betrifft die Offenbarung insbesondere ein Peptid, das eine Sequenz, ausgewählt aus der Gruppe bestehend aus SEQ ID NO: 17-19, 90-97, 100-102, 105, 106, 111-116, 120, 123, 124, 135-137, 139 und 142-150, einem Teil oder Derivat davon aufweist oder umfasst.

In einem weiteren Aspekt betrifft die offenbarte Lehre ein Mittel, das an ein offenbarungsgemäß identifiziertes Tumor-assoziiertes Antigen oder an einen Teil davon bindet. In einer bevorzugten Ausführungsform ist das Mittel ein Antikörper. In weiteren Ausführungsformen ist der Antikörper ein chimärer, ein humanisierter oder mit kombinatorischen Techniken hergestellter Antikörper oder ein Fragment eines Antikörpers. Des weiteren betrifft die Offenbarung einen Antikörper, der selektiv an einen Komplex aus (i) einem offenbarungsgemäß identifizierten Tumor-assoziierten Antigen oder einem Teil davon und (ii) einem MHC-Molekül bindet, an das das offenbarungsgemäß identifizierte Tumor-assoziierte Antigen oder der Teil davon bindet, wobei der Antiköper nicht alleine an (i) oder (ii) bindet. Ein Antikörper kann ein monoklonaler Antikörper sein. In weiteren Ausführungsformen ist der Antikörper ein chimärer oder humanisierter Antikörper oder ein Fragment eines natürlichen Antikörpers.

Insbesondere betrifft die vorliegende Offenbarung ein solches Mittel, insbesondere einen Antikörper, das/der spezifisch an ein Peptid bindet, das eine Sequenz ausgewählt aus der Gruppe bestehend aus SEQ ID NO: 17-19, 90-97, 100-102, 105, 106, 111-116, 120, 123, 124, 135-137, 139 und 142-150, einem Teil oder Derivat davon aufweist oder umfasst.

Hinsichtlich Claudin-18 betrifft die Erfindung auch Mittel, insbesondere Antikörper, die spezifisch an eine Variante von Claudin-18 binden. In einer Ausführungsform bindet das Mittel, insbesondere ein Antikörper, spezifisch an die Variante Claudin-18A1 (SEQ ID NO: 118). In einer anderen Ausführungsform bindet das Mittel, insbesondere ein Antikörper, spezifisch an die Variante Claudin-18A2 (SEQ ID NO: 16). Solche spezifischen Antikörper können beispielweise durch Immunisieren mit den in Beispiel 4 beschriebenen Peptiden erhalten werden.

Des weiteren betrifft die Offenbarung hinsichtlich Claudin-18 Mittel, insbesondere Antikörper, die spezifisch an eine Form von Claudin-18A2 binden, die ein bestimmtes Glykosylierungmuster aufweist. In einer Ausführungsform bindet das Mittel, insbesondere ein Antikörper, spezifisch an eine Form von Claudin-18A2, die an einer oder mehreren möglichen Glykosylierungsstellen nicht-glykosyliert ist. In einer anderen Ausführungsform bindet das Mittel, insbesondere ein Antikörper, spezifisch an eine Form von Claudin-18A2, die an einer oder mehreren möglichen Glykosylierungsstellen glykosyliert ist. Vorzugsweise betrifft eine derartige mögliche Glykosylierungsstelle eine oder mehrere Positionen, die aus der Gruppe ausgewählt sind, bestehend aus den Aminosäurepositionen 37, 38, 45, 116, 141, 146 und 205 von Claudin-18A2. Des weiteren betrifft eine derartige mögliche Glykosylierung vorzugsweise eine N-Glykosylierung.

Ein für eine Variante bzw. Form von Claudin-18 spezifisches Mittel, insbesondere ein für eine Variante bzw. Form von Claudin-18 spezifischer Antikörper, meint in diesem Zusammenhang, dass das Mittel bzw. der Antikörper stärker an die Variante bzw. Form für die es/er spezifisch ist, bindet als an eine andere Variante bzw. Form. Ein Mittel, insbesondere Antikörper, bindet stärker an eine erste Variante bzw. Form oder an ein erstes Epitop im Vergleich zu einer zweiten Variante bzw. Form oder einem zweiten Epitop, wenn es/er an die erste Variante bzw. Form oder an das erste Epitop mit einer Dissoziationskonstante (K_{D}) bindet, die geringer ist als die Dissoziationskonstante für die zweite Variante bzw. Form oder das zweite Epitop. Vorzugsweise ist die Dissoziationskonstante (K_{D}) für die Variante bzw. Form oder das Epitop, an die/das das Mittel, insbesondere ein Antikörper, spezifisch bindet mehr als 10-fach, vorzugsweise mehr als 20-fach, mehr bevorzugt mehr als 50-fach, noch mehr bevorzugt mehr als 100-fach und insbesondere mehr als 200-fach, 500-fach oder 1000-fach geringer als die Dissoziationskonstante (K_{D}) für die Variante bzw. Form oder das Epitop, an die/das das Mittel, insbesondere ein Antikörper, nicht spezifisch bindet. Vorzugsweise bindet ein Mittel, insbesondere ein Antikörper, nicht oder im wesentlichen nicht an die Variante bzw. Form oder das Epitop für die/das das Mittel, insbesondere der Antikörper, nicht spezifisch ist.

Die vorstehend beschriebenen Mittel, insbesondere Antikörper und Derivate davon wie hierin beschrieben, die an eine Variante bzw. Form von Claudin-18 spezifisch binden, sind auch für eine Verwendung in den offenbarungsgemäßen Zusammensetzungen und Verfahren vorgesehen.

Des weiteren betrifft die Offenbarung ein Konjugat zwischen einem offenbarungsgemäßen Mittel, das an ein offenbarungsgemäß identifiziertes Tumor-assoziiertes Antigen oder an einen Teil davon bindet, oder einem offenbarungsgemäßen Antikörper und einem therapeutischen oder diagnostischen Mittel oder Stoff. In einer Ausführungsform ist das therapeutische oder diagnostische Mittel ein Toxin.

In einem weiteren Aspekt betrifft die offenbarte Lehre einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

### Detaillierte Beschreibung der Erfindung

Der Schutzbereich der Erfindung wird durch den Umfang der angehängten Ansprüche abgedeckt. Vorliegend werden Gene beschrieben, die in Tumorzellen selektiv exprimiert oder aberrant exprimiert werden und Tumor-assoziierte Antigene darstellen.

Offenbarungsgemäß sind diese Gene und/oder deren Genprodukte und/oder ihre Derivate und/oder Teile bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.
Mit "Derivat" einer Nukleinsäure ist offenbarungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Nukleotidbase, am Zucker oder am Phosphat. Der Begriff "Derivat" umfasst auch Nukleinsäuren, die nicht in der Natur vorkommende Nukleotide und Nukleotidanaloga enthalten.

Eine Nukleinsäure ist offenbarungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann offenbarungsgemäß als einzelsträngiges oder doppelsträngiges und lineares oder kovalent kreisförmig geschlossenes Molekül vorliegen.

Die offenbarungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen offenbarungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können offenbarungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure offenbarungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist ein rekombinantes DNA-Molekül ein Vektor, gegebenenfalls mit einem Promotor, der die Expression einer Nukleinsäure, z.B. einer Nukleinsäure, die für ein offenbarungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide oder Virusgenome.

Die Nukleinsäuren, die für ein offenbarungsgemäß identifiziertes Tumor-assoziiertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft offenbarungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege, Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und nicht-humane embryonale Stammzellen. In weiteren Ausführungsformen ist die Wirtszelle eine Antigen-präsentierende Zelle, insbesondere eine dendritische Zelle, ein Monozyt oder ein Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird offenbarungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht) und die Enhancer-Promotor-Sequenzen von Cytomegalovirus (CMV).

In den Fällen der Offenbarung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Offenbarungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nichtüberlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst offenbarungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-Splicing-Stelle.

In einer Ausführungsform besteht ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die offenbarungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im Wesentlichen aufgereinigt" bedeutet, dass das Protein oder Polypeptid im Wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Offenbarungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Offenbarung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen, Polypeptiden oder Peptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine, Polypeptide oder Peptide. Ferner erstreckt sich der Begriff "Derivat" auch auf alle funktionellen chemischen Äquivalente der Proteine, Polypeptide oder Peptide.

Ein Teil oder Fragment eines Tumor-assoziierten Antigens weist eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer T-Zellen beruhen. Vorzugsweise umfasst ein offenbarungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen.

Ein Teil oder ein Fragment einer Nukleinsäure, die für ein Tumor-assoziiertes Antigen kodiert, betrifft offenbarungsgemäß den Teil der Nukleinsäure, der zumindest für das Tumor-assoziierte Antigen kodiert und/oder für einen Teil oder ein Fragment des Tumor-assoziierten Antigens wie vorstehend definiert kodiert.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Lehre ermöglicht auch die Isolierung von Proteinen, die an hier beschriebene Tumor-assoziierte Antigene binden, einschließlich Antikörper und zelluläre Bindepartner der Tumor-assoziierten Antigene.

Vorliegend werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Die offenbarte Lehre umfasst auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Offenbarungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Solche Antikörper können Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Dies nutzt die sogenannte "SLAM"-Technologie. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

Als anderes Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefahigkeit werden oft als "chimäre" Antikörper bezeichnet.

Offenbarungsgemäß bereitgestellt werden auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden. Offenbarungsgemäß umfasst sind auch sogenannte einzelkettige Antikörper. Offenbarungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung offenbarungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Offenbarungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluorin und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-*Exotoxin sein. Toxinreste können auch Hochenergie-emittierende Radionuklide wie Kobalt-60 sein.

Der Begriff "Patient" bedeutet offenbarungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft offenbarungsgemäß jeden pathologischen Zustand, bei dem Tumor-assoziierte Antigene exprimiert oder abnormal exprimiert werden. "Abnormale Expression" bedeutet erfindun offenbarungsgemäß gsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, wobei der Begriff "Krebs" erfindungsgemäß Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, und Lungenkrebs und deren Metastasen umfasst.

Eine biologische Probe kann offenbarungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Sputum, Urin, Fäces oder anderen Körperflüssigkeiten.

Der Begriff "immunreaktive Zelle" bedeutet offenbarungsgemäß eine Zelle, die in eine Immunzelle (wie B-Zelle, T-Helferzelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-9633265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen) mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen) mit cytotoxischen T-Lymphozyten von gesunden Personen oder von einer anderen Spezies (z.B. Maus) kombiniert. Dies führt zu einer Vermehrung hochaffiner spezifischer cytotoxischer T-Lymphozyten, wenn die T-Lymphozyten aus einem Spenderorganismus kommen, der mit dem spezifischen Komplex bisher keinen Kontakt hatte. Der hochaffine T-Zell-Rezeptor aus diesen vermehrten spezifischen T-Lymphozyten wird kloniert. Wurden die hochaffinen T-Zellrezeptoren aus einer anderen Spezies kloniert, können diese in unterschiedlichem Ausmaß humanisiert werden. Durch Gentransfer z.B. mit retroviralen Vektoren werden solche T-Zell-Rezeptoren dann beliebig in T-Zellen von Patienten transduziert. Adoptiver Transfer erfolgt dann mit diesen genetisch veränderten T-Lymphozyten (Stanislawski et al., Nat Immunol. 2:962-70, 2001 ; Kessels et al., Nat Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden, Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das antigene Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigenes in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer T-Zell-Antworten entstehen können. Eine effektive Immunantwort benötigt diese (Ossendorp et al., Immunol Lett. 74:75-9, 2000; Ossendorp et al., J. Exp. Med. 187:693-702, 1998). Im Allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc Natl Acad Sci USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J Immunol. 167:787-96, 2001; Appella et al., Biomed Pept Proteins Nucleic Acids 1:177-84, 1995; Wentworth et al., Mol Immunol. 32:603-12, 1995).

Die offenbarungsgemäß beschriebenen pharmazeutischen Zusammensetzungen können auch als Vakzinen für die Immunisierung eingesetzt werden. Die Begriffe "Immunisierung" oder "Vakzinierung" bedeuten offenbarungsgemäß eine Erhöhung oder Aktivierung einer Immunreaktion gegenüber einem Antigen. Tiermodelle können zum Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vakzinierung aufgrund ihrer regulatorischen Eigenschaften auf Lymphozyten verwendbar. Solche Cytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Cytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren.

Offenbarungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex vivo*-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Offenbarungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virusähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um offenbarungsgemäß Nukleinsäuren in Zellen *in vitro* oder *in vivo* einzubringen. Solche Verfahren umfassen die Transfektion von Nukleinsäure-CaPO₄-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die offenbarungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien, CpG und Cytokinen und gegebenenfalls andere therapeutische Wirkstoffe enthalten.

Die therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Krankheit oder eines Zustands kann auch die Verzögerung des Ausbruchs oder eine Verhinderung des Ausbruchs der Krankheit oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nicht-toxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine offenbarungsgemäße pharmazeutische Zusammensetzung kann einen pharmazeutisch verträglichen Träger umfassen. Der Begriff "pharmazeutisch verträglicher Träger" betrifft offenbarungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der pharmazeutischen Zusammensetzung sind gewöhnlich derart, dass keine Interaktion auftritt, die die gewünschte pharmazeutische Wirksamkeit wesentlich beeinträchtigt.

Die pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Offenbarungsgemäße pharmazeutische Zusammensetzungen können beispielsweise in Form von Kapseln, Tabletten, Lutschpastillen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich.

### Abbildungen:

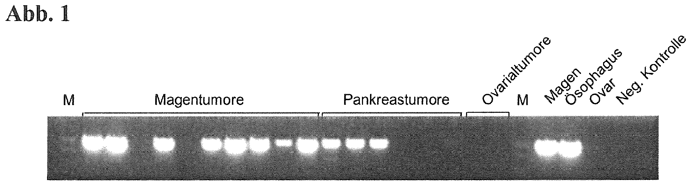

**Abb. 1****. Claudin-18A2.1-Expression in Magen und Ösophagus sowie Magen- und Pankreastumoren**
   RT-PCR-Analyse mit Claudin-18A2.1-spezifischen Primern (SEQ ID NO: 39, 40) zeigte erfindungsgemäß in 8/10 Magentumor-Biopsien sowie in 3/6 Pankreastumor-Biopsien eine ausgeprägte Claudin-18A2.1-Expression. In Magen- und Ösophagus-Normalgeweben wurde ebenfalls eine deutliche Expression nachgewiesen. Im Gegensatz dazu wurde im Ovar und in Ovarialtumoren keine Expression detektiert.

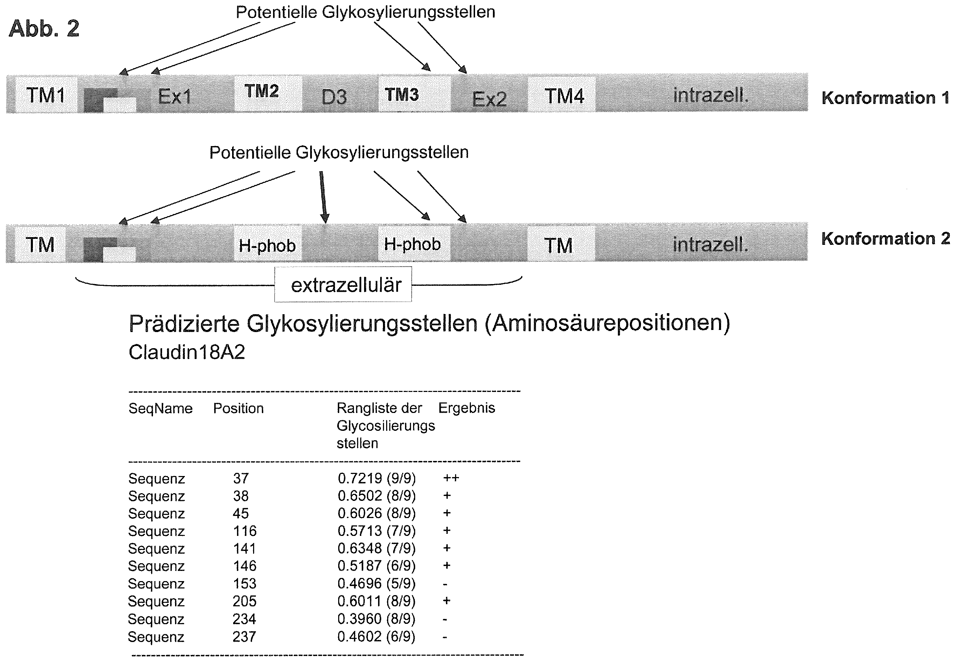

**Abb. 2****. Schematische Darstellung von Claudin-18-Konformationen**
   Offenbarungsgemäß kann das Claudin-18A2-Polypeptid in zwei Konformationen auf der Zelle vorliegen. In der Konformation 1 liegt das Protein als Membranmolekül mit 4 Transmembrandomänen (TM) vor und weist zwei separate, extrazellulär gelegene Domänen auf. In der Konformation 2 üben die beiden mittleren hyrophoben Bereiche (h-phob) keine Transmembrandomänen-Funktion aus. Dadurch sind in dieser Konformation im Vergleich zu der Konformation 1 zusätzliche Peptidregionen extrazellulär gelegen. Außerdem resultiert aus dieser Konformation eine zusätzliche N-Glykosylierungsstelle an Position 116 (dickerer Pfeil). Alle prädizierten Glykosylierungsdomänen sind im unteren Teil der Abbildung aufgeführt. Ex1: extrazelluläre Domäne 1, Ex2: extrazelluläre Domäne 2, TM: Transmembrandomäne, H-phob: extrazelluläre hydrophobe Region.
**Abb. 3****. Quantitative Expression von Claudin-18, Variante A1**
   Claudin-18A1 ist in keinem Normalgewebe außer Lungen- und Magengewebe detektierbar. Claudin-18A1 ist in einer Vielzahl von Tumorgeweben stark exprimiert. Eine besonders starke Expression findet sich in Magentumoren, Lungentumoren, Pankreastumoren und Ösophagustumoren.
**Abb. 4****. Quantitative Expression von Claudin-18, Variante A2**
   Claudin-18A2 ist in keinem Normalgewebe ausser Magengewebe detektierbar. Claudin-18A2 ist in einer Vielzahl von Tumorgeweben insbesondere Magentumoren, Lungentumoren, Pankreastumore und Ösophagustumoren exprimiert.
**Abb. 5****. Verwendung Claudin-18A2-spezffischer Antikörper (extrazelluläre Domäne)**
   **A:** Färbung von Claudin-18A2-positiven Magentumorzellen (SNU-16, Methanol-fixiert) mit einem Antikörper, der durch Immunisierung mit einem Peptid (SEQ ID NO: 17) hergestellt wurde. Eine Membranfärbung tritt besonders stark in den Zell/Zell-Interaktionsbereichen auf. Das Protein aggregiert in fokalen Membranbereichen.
   **B**, **C, D:** Nachweis der Spezifität des Antikörpes durch Kolokalisationsanalyse in Claudin-18A2-GFP-transfizierten 293T-Zellen. B: GFP-Fluoreszenz; C: anti-Claudin-18A2; D: Überlagerung.

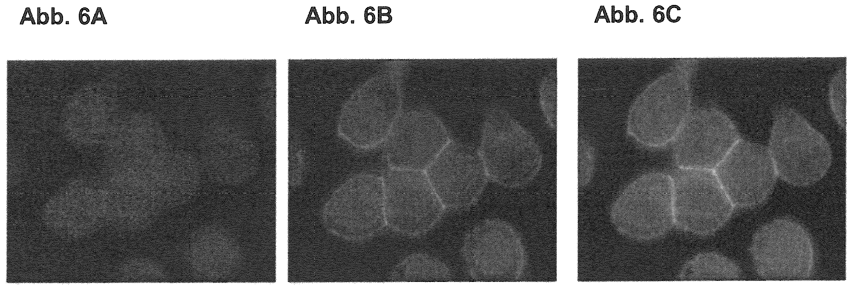

**Abb. 6****. Verwendung Claudin-18A2-spezifischer Antikörper (extrazelluläre Domäne)**
   Membranfärbung von Claudin-18A2-positiven Magentumorzellen (SNU-16) mit einem Antikörper, der durch Immunisierung mit einem Peptid (SEQ ID NO: 113, N-terminal gelegene extrazelluläre Domäne) hergestellt wurde. Zur Gegenfärbung wurde ein monoklonaler Antikörper verwendet, der gegen E-Cadherin gerichtet ist. A: Claudin-18A2-Antikörper; B: anti E-Cadherin-Gegenfärbung; C: Überlagerung.

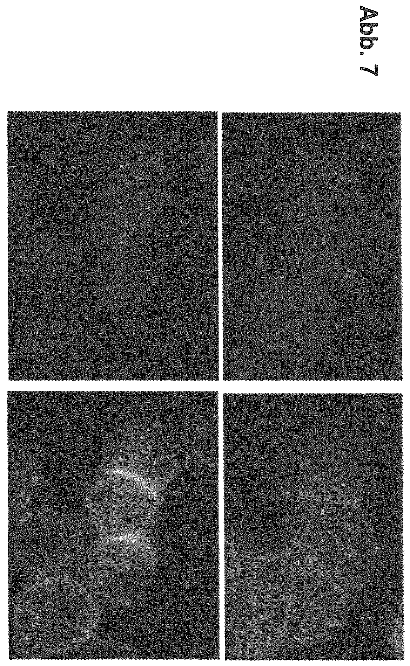

**Abb. 7****. Verwendung von Antikörpern gegen die C-terminale extrazelluläre Domäne von Claudin-18**
   Linke Abbildungen: Membranfärbung von Claudin-18A2-positiven Magentumorzellen (SNU-16) mit einem Antikörper, der durch Immunisierung mit einem Peptid (SEQ ID NO: 116, C-terminal gelegene extrazelluläre Domäne) hergestellt wurde. Zur Gegenfärbung wurde ein monoklonaler Antikörper verwendet, der gegen E-Cadherin gerichtet ist (rechte Abbildungen).

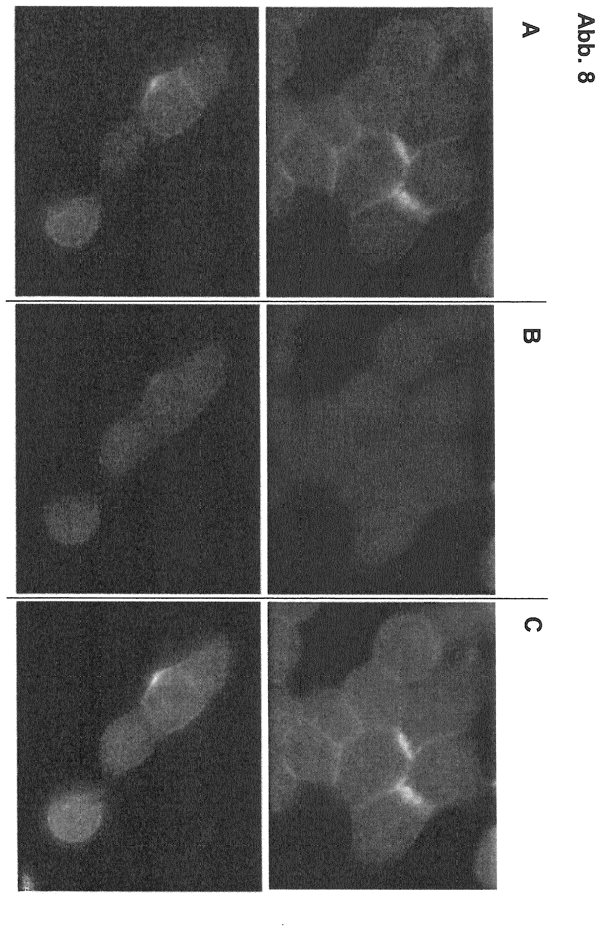

**Abb. 8****. Verwendung Claudin-18A1-spezifischer Antikörper**
   Oben: schwache bis fehlende Färbung von Magentumorzellen (SNU-16; Claudin18A2-positiv) mit einem Antikörper, der durch Immunisierung mit einem Claudin-18A1-spezifischen Peptid (SEQ ID NO: 115) hergestellt wurde. A: anti-E-Cadherin; B: anti-Claudin-18A1; C: Überlagerung.
   Unten: Nachweis der Spezifität des Antikörpes durch Kolokalisationsanalyse in Claudin-18A1-GFP-transfizierten 293T-Zellen. A: GFP-Fluoreszenz; B: anti-Claudin-18A1; C: Überlagerung.

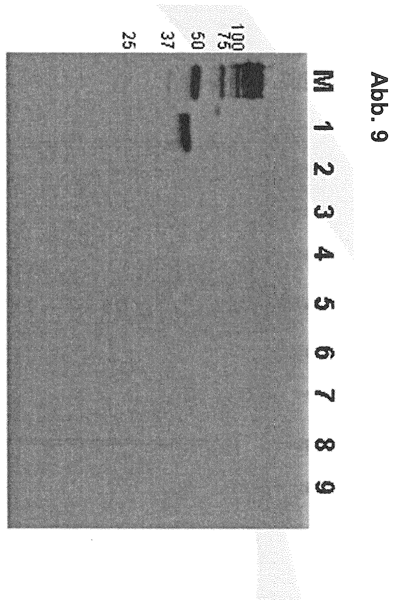

**Abb. 9****. Nachweis von Claudin-18A2 im Western-Blot.**
   Western-Blot mit Lysaten aus verschiedenen gesunden Geweben mit einem Claudin-18A2-spezifischen Antikörper, gerichtet gegen das Epitop mit SEQ ID NO: 17. 1: Magen-; 2: Testis-; 3: Haut-; 4: Brust-; 5: Leber-; 6: Kolon-; 7: Lunge-; 8: Niere-; 9: Lymphknoten-Normalgewebe.

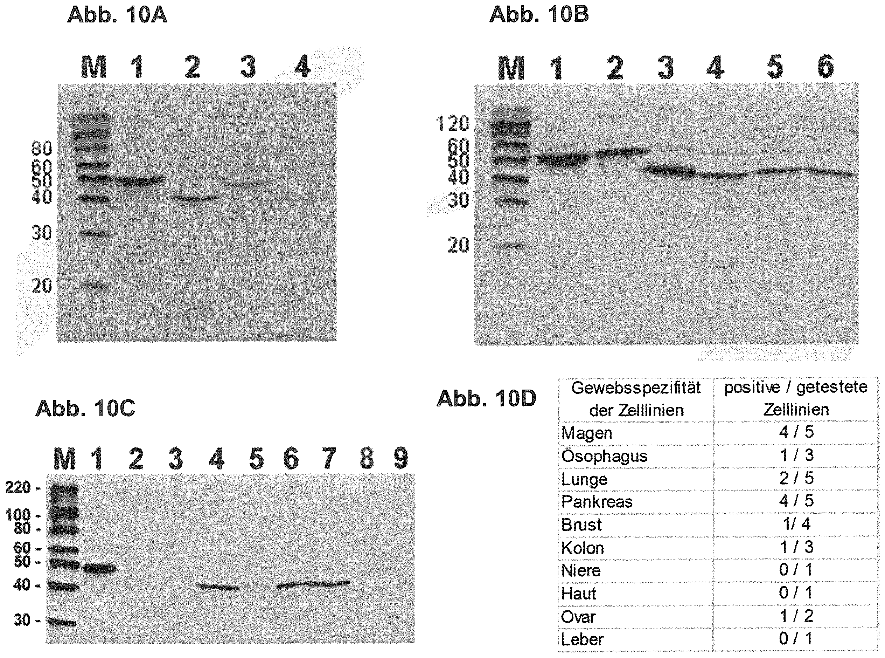

**Abb. 10****. Western-Blot von Claudin-18A2 mit Proben aus Magen und Magentumoren sowie verschiedenen Tumorzelllinien.**
   Lysate aus Magen und Magentumoren (A, B) und Tumorzelllinien (C, D) wurden geblottet und mit einem Claudin-18A2-spezifischen Antikörper gegen das Epitop mit SEQ ID NO: 17 getestet. Magentumore weisen eine geringer glykosylierte Form von Claudin-18A2 auf. Eine PNGase F-Behandlung von Magenlysaten führt zur Bildung der niedrigglykosylierten Form.
   **A:** 1: Magen-Normalgewebe #A; 2: Magentumor #A; 3: Magen-Normalgewebe #B; 4: Magentumor #B
   **B:** 1: Magen-Normalgewebe #A; 2: Magen-Normalgewebe #B; 3: Magen-Normalgewebe #B + PNGase F; 4: Magentumor #C; 5: Magentumor #D; 6: Magentumor #D + PNGase F
   **C:** 1: Magen-Normalgewebe; 2: MDA-MB-231; 3: SK-MEL-37; 4: AGS; 5: SNU-1; 6: SNU-16; 7: EFO27; 8: TOV-112D; 9: OVCAR. Zu beachten ist, dass die Tumorzelllinien die deglykosylierte Variante von Claudin-18A2 exprimieren.
   **D:** Tabellarische Zusammenfassung der Western-Blot-Daten für eine Auswahl von Zelllinien, die mit dem Claudin-18A2-spezifischen Antikörper getestet wurden.

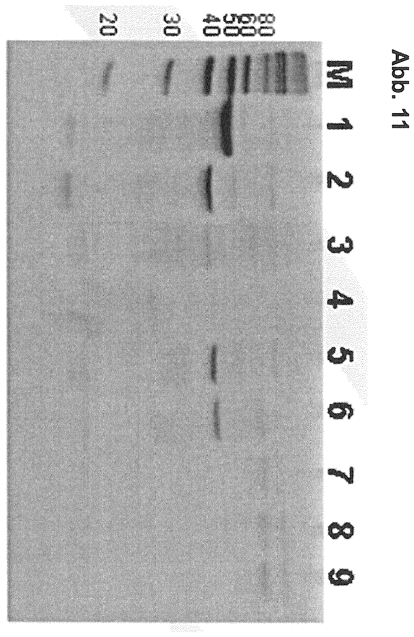

**Abb. 11****. Expression von Claudin-18 in Lungentumoren**
   Entsprechend zu Abb. 30 erfolgte ein Nachweis von niedrigglykosylierten Claudin-18A2-Varianten in Lungentumoren. 1: Magen-Normalgewebe; 2: Magentumor; 3-9: Lungentumore.

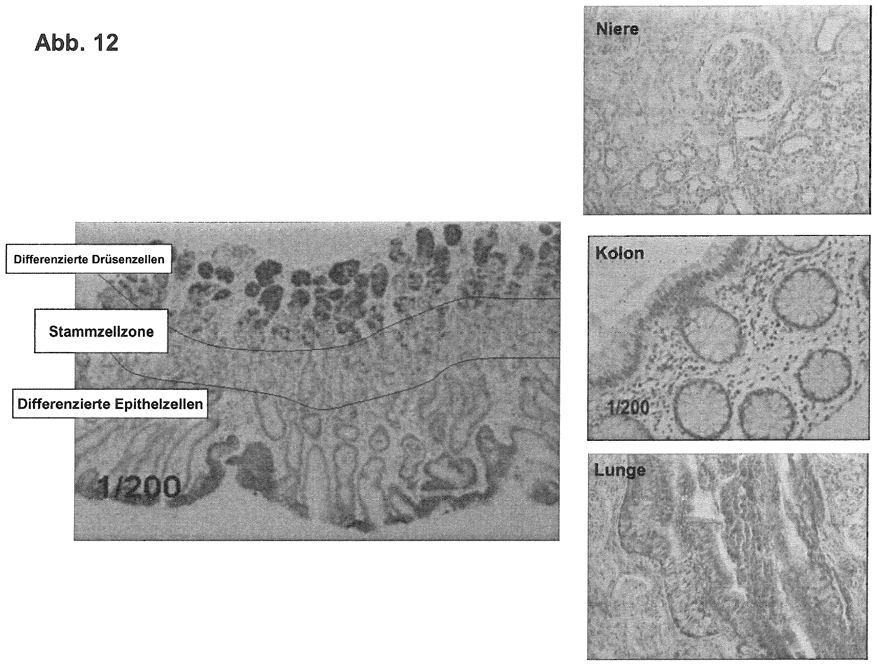

**Abb. 12****. Immunhistochemische Analyse von Claudin-18 mit Claudin-18A2-spezifischen Antikörpern in Normalgeweben**
   In der Magenschleimhaut werden lediglich differenzierte epitheliale Zellen an der Öffnung wie auch am Boden der Drüsen gefärbt. Claudin-18A2 ist in Stammzellen des Magens nicht nachweisbar. Alle anderen von uns untersuchten Normalgewebe exprimieren dieses Gen ebenfalls nicht, wie beispielhaft für Niere, Lunge und Kolon dargestellt.

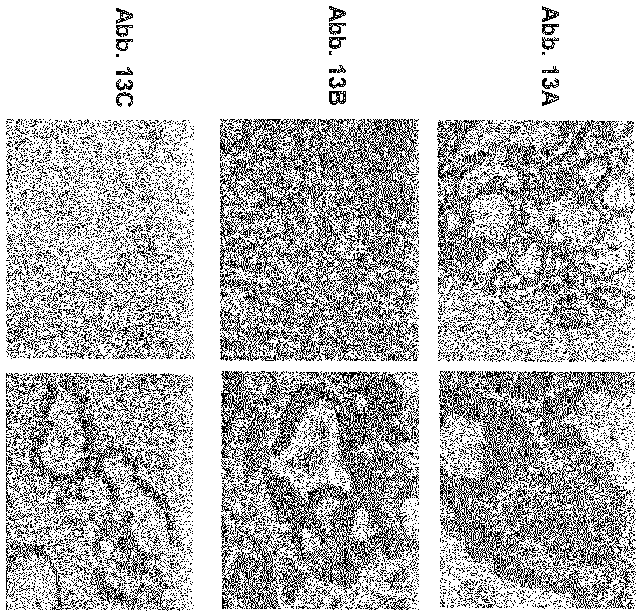

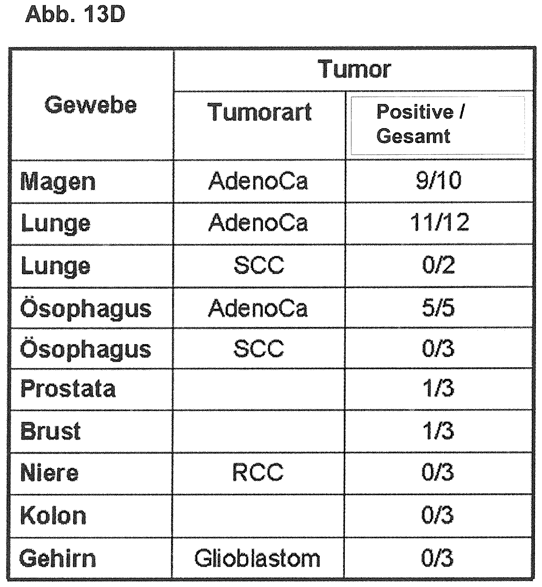

**Abb. 13****. Resultate der Immunhistologie mit Claudin-18A2-spezifischem polyklonalen Antiserum**
   **A:** Beispiele für spezifische Färbungen von Lungentumor-Geweben. Zu beachten ist, dass das normale Lungengewebe, welches die Variante Claudin-18A1 exprimiert, durch das Claudin-18A2-spezifische Antiserum nicht erkannt wird.
   **B:** Beispiele für spezifische Tumorfärbung von Ösophagustumoren. Zu beachten ist, dass gesunde Zellen in der Umgebung nicht gefärbt werden.
   **C:** Beispiele für spezifische Tumorfärbung von Magentumorepithelien. Auch hier werden gesunde Zellen in der Umgebung nicht gefärbt.
   **D:** Beispeilhafte tabellarische Zusammenfassung immunhistochemischer Färbedaten mit Claudin-18A2-spezifischen Antikörpern. AdenoCa: Adenokarzinom; SCC: Plattenepithelkarzinom; RCC: Nierenzellkarzinom.

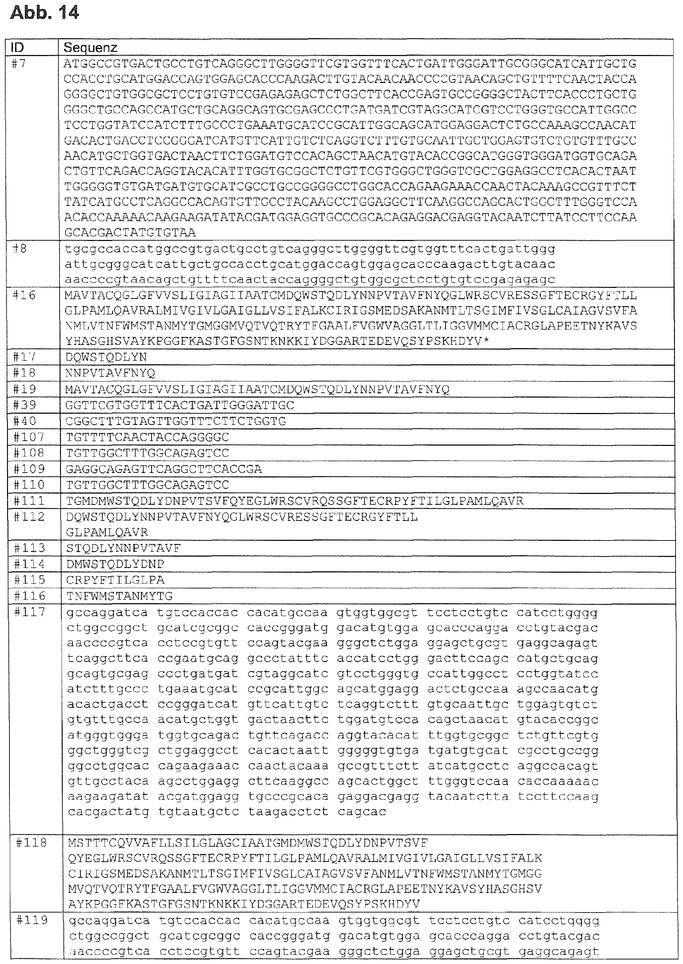

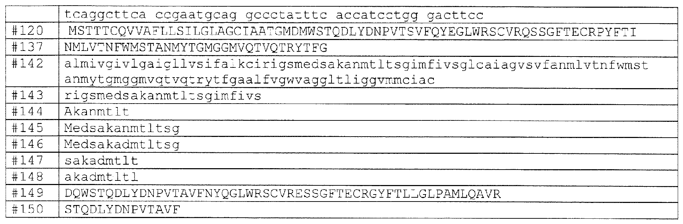

**Abb. 14****. Sequenzen**
   Gezeigt sind die Sequenzen, auf die hierin verwiesen wird.

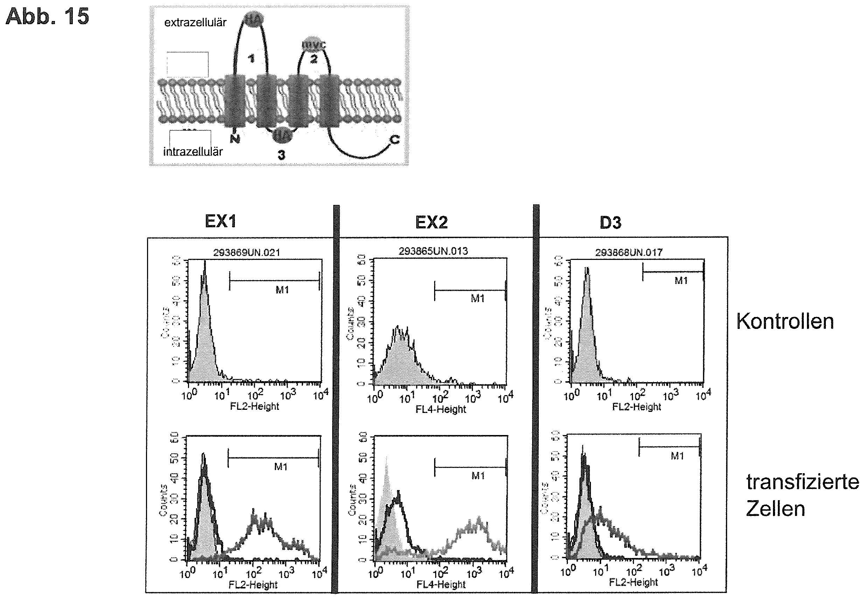

**Abb. 15****. Ermittlung extrazellulärer Bereiche von Claudin-18A2**
   Drei Konstrukte wurden hergestellt, die jeweils eine Marker-Sequenz (myc- oder HA-Tag) in einer der Domänen EX1 (= extrazelluläre Domäne 1), EX2 (= extrazelluläre Domäne 2), oder D3 (= Domäne 3) trugen (oben). Diese wurden in Zelllinien transfiziert und daraufhin getestet, ob ein gegen diese Markersequenzen gerichteter Antikörper an nichtpermeabilisierte Zellen bindet. Dies erfordert, dass der entsprechende Bereich des Proteins topologisch extrazellulär ist. In der Durchflusszytometrie erwies sich, dass alle drei Bereiche des Moleküls für die Antikörper zugänglich sind (unten).

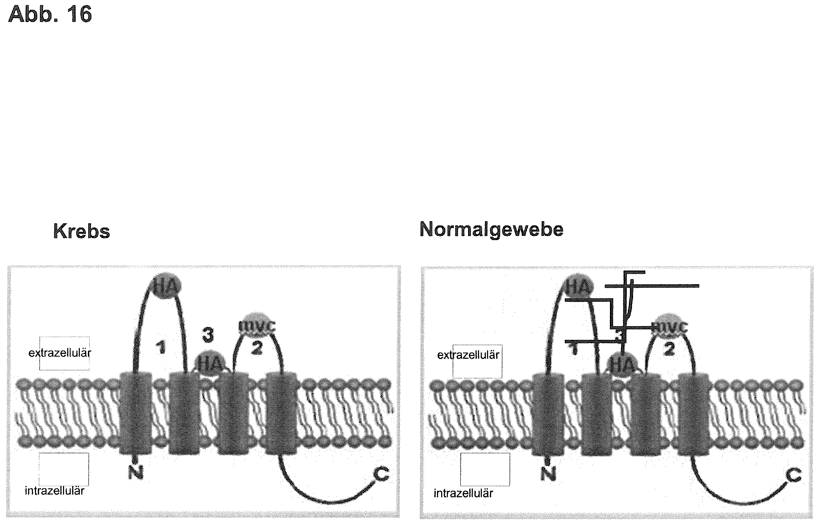

**Abb. 16****. Claudin-18A2-Membrantopologie**
   Entsprechend unserer Daten kann Claudin-18A2 in der Konformation 2 vorliegen, wobei die inneren beiden hydrophoben Domänen nicht integral durch die Zellmembran hindurchführen. Dadurch sind größere Bereiche dieses Moleküls extrazellulär. Hier befinden sich auch Glykosylierungsdomänen, die nach unseren Daten in Magennormalgewebe glykosyliert sind, in Tumoren aber nicht. Es entstehen somit rein tumorgewebsspezifische Epitope.

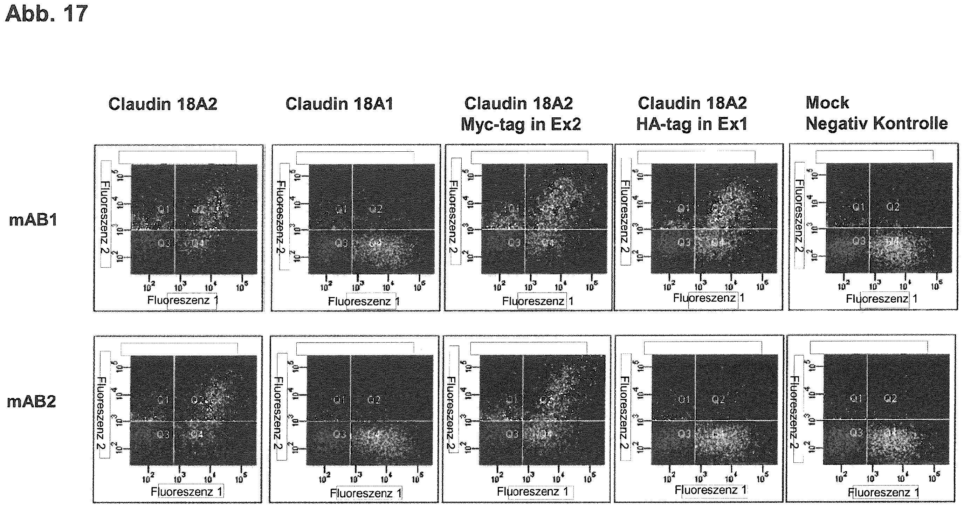

**Abb. 17****. FACS-Analyse zur Bestimmung der extrazellulären Lokalisation von Claudin 18**
   Die Abbildung zeigt durchflusszytometrische Analysen an nichtpermeabilisierten Zellen, die mit Voll-Längen Claudin-18A1, Claudin-18A2 und Mock sowie Abschnitten aus Claudin-18A2 transfiziert wurden. Erkennbar ist, dass die Antikörper mAB1 und mAB2 spezifisch Claudin-18A2 (linke Spalte) und die extrazelluläre Domäne 2 (Ex2, 3. Spalte) an der Zelloberfläche erkennen, während Claudin-18A1 (2. Spalte) und die Negativ-Kontrolle (letzte Spalte) negativ sind. Der Antikörper mAB1 bindet, im Gegensatz zu mAB2, auch spezifisch die extrazelluläre Domäne 1 (Exl, 4. Spalte).

### Beispiele:

### Material und Methoden

Die Begriffe *"in silico",* "elektronisch" und "virtuell klonieren" beziehen sich rein auf die Nutzung von auf Datenbanken beruhenden Verfahren, mit denen auch Labor-experimentelle Vorgänge simuliert werden können.

Alle anderen Begriffe und Termini werden, falls nicht explizit anders definiert, so verwendet, wie sie der Fachmann versteht. Die genannten Techniken und Methoden erfolgen in an sich bekannter Weise und sind z.B. in Sambrook et al., Molecular Cloning: A Laboratory Manual, 2. Auflage (1989) Cold Spring Harbor Laboratory Press, Cold Spring Harbor, N. Y beschrieben. Alle Verfahren, die die Verwendung von Kits und Reagenzien einschließen, sind entsprechend den Angaben der Hersteller durchgeführt.

### Datamining-basierte Strategie zur Ermittlung von neuen Tumor-assoziierten Genen

Zwei *in silico* Strategien nämlich GenBank-Schlagwort-Suche und der cDNAxProfiler wurden kombiniert. Es wurde unter Nutzung des ENTREZ Search and Retrieval Systems des NCBI (http://www.ncbi.nlm.nih.gov/Entrez) eine Suche nach Kandidaten-Genen in der GenBank durchgeführt, die annotiert sind als spezifisch exprimiert in bestimmten Geweben (Wheeler et al., Nucleic Acids Research 28:10-14, 2000).
Durch Suchabfragen mit Schlagworten wie beispielsweise "colon-specific gene", " stomachspecific gene", oder "kidney-specific gene" wurden Kandidatengene (GOI, genes of interest) aus den Datenbanken herausextrahiert. Die Suche wurde auf einen Teil der Gesamtinformation dieser Datenbanken eingeschränkt, indem als Limits "homo sapiens" für den Organismus und "mRNA" für die Molekülart eingesetzt wurden.
Die Liste der gefundenen GOI wurde kuratiert, indem unterschiedliche Bezeichnungen für dieselbe Sequenz ermittelt und solche Redundanzen behoben wurden.
Alle Kandidatengene, die sich durch die Schlagwort-Suche ergaben, wurden wiederum durch das Verfahren des "electronic Northern" (eNorthern) bezüglich ihrer Gewebeverteilung untersucht. Der eNorthern basiert darauf, dass die Sequenz eines GOI gegenüber einer EST-(expressed sequence tag) Datenbank (Adams et al., Science 252:1651, 1991) abgeglichen wird (http://www.ncbi.nlm.nih.gov/BLAST). Zu jedem EST, das sich als homolog zum eingegebenen GOI ergibt, lässt sich die Gewebeherkunft ermitteln und durch die Summe aller ESTs auf diese Weise eine vorläufige Einschätzung der Gewebeverteilung des GOI erreichen. Nur diejenigen GOI wurden weiteren Untersuchungen zugeführt, die keine Homologien zu EST aus nicht Organ-spezifischen Normalgeweben hatten. Für dies Beurteilung wurde auch berücksichtigt, dass es falsch-annotierte cDNA-Banken in der öffentlichen Domäne gibt (Scheurle et al., Cancer Res. 60: 4037-4043, 2000) (www.fau.edu/cmbb/publications/cancergenes6.htm). Als zweites Datamining-Verfahren wurde der cDNA xProfiler des Cancer Genome Anatomy Projekts des NCBI (http://cgap.nci.nih.gov/Tissues/xProfiler) genutzt (Hillier et al., Genome Research 6:807-828, 1996; Pennisi, Science 276:1023-1024, 1997). Dieser erlaubt, Pools von in Datenbanken abgelegten Transkriptomen durch logische Operatoren in Beziehung zueinander zu setzen. Wir haben einen Pool A definiert, dem beispielsweise alle aus Colon hergestellten Expressionsbibliotheken, unter Ausschluss von gemischten Bibliotheken zugeordnet wurden. Dem Pool B wurden alle cDNA-Bibliotheken zugeordnet, die von Normalgeweben mit Ausnahme von Colon hergestellt waren. Generell wurden alle cDNA-Banken unabhängig vom zugrundeliegenden Herstellungsverfahren genutzt, allerdings lediglich solche mit einer Mächtigkeit > 1000 zugelassen. Mittels des BUT NOT Operators wurde Pool B digital von Pool A subtrahiert. Auch das Set der auf diese Weise gefundenen GOI wurde eNorthern-Studien unterzogen, sowie durch eine Literaturrecherche abgesichert.
Dieses kombinierte Datamining schließt alle etwa 13 000 Volllänge-Gene in der öffentlichen Domäne ein und prädiziert aus diesen Gene mit potentieller Organ-spezifischer Expression.

Alle anderen Gene wurden zunächst durch spezifische RT-PCR in Normalgeweben evaluiert. Alle GOI, die sich als in nicht Organ-spezifischen Normalgeweben exprimiert erwiesen, hatten als Falsch-Positive zu gelten und wurden aus weiteren Untersuchungen ausgeschlossen. Die verbliebenen wurden in einem großen Panel an verschiedensten Tumorgeweben untersucht. Die unten dargestellten Antigene erwiesen sich dabei als in Tumorzellen aktiviert.

### RNA-Extraktion, Herstellung von poly-d(T) geprimter cDNA und konventionelle RT-PCR Analyse

Gesamt-RNA aus nativem Gewebematerial wurde unter Verwendung von Guanidiumisothiocyanat als chaotrophem Agens extrahiert (Chomczynski & Sacchi, Anal. Biochem. 162:156-9, 1987). Nach Extraktion mit saurem Phenol und Fällung mit Isopropanol wurde die RNA in DEPC-behandeltem Wasser gelöst.
Aus 2-4 µg Gesamt-RNA wurde in einem 20µl Reaktionsansatz mittels Superscript II (Invitrogen) entsprechend den Angaben des Herstellers eine Erststrang-cDNA-Synthese durchgeführt. Als Primer wurde ein dT(18) Oligonukleotid verwendet. Integrität und Qualität der cDNA wurden durch Amplifikation von p53 in einer 30 Zyklen-PCR (sense CGTGAGCGCTTCGAGATGTTCCG, antisense CCTAACCAGCTGCCCAACTGTAG, Hybridisierungstemperatur 67°C) überprüft.
Es wurde ein Archiv aus Erststrang-cDNAs aus einer Reihe von Normalgeweben und Tumorentitäten hergestellt. Für Expressionsstudien wurden 0,5 µl dieser cDNAs in einem 30µl Reaktionsansatz mit GOI-spezifischen Primern (siehe unten) und 1 U HotStarTaq DNA Polymerase (Qiagen) amplifiziert. Der Reaktionsansatz enthielt jeweils 0,3 mM dNTPs, je 0,3 µM jeden Primers und 3 µl 10 x Reaktionspuffer.
Die Primer wurden so ausgewählt, dass sie in 2 verschiedenen Exons liegen, und die Beseitigung der Interferenz durch kontaminierende genomische DNA als Grund für falsch positive Resultate wurde durch Testen von nicht revers transkribierter DNA als Matrize bestätigt. Nach 15 Minuten bei 95°C zur Aktivierung der HotStarTaq DNA Polymerase wurden 35 Zyklen PCR durchgeführt (1 min 94°C, 1 min jeweilige Hybridisierungstemperatur, 2 min 72°C und abschließende Elongation bei 72°C für 6 min). 20µl dieser Reaktion wurden auf einem mit Ethidiumbromid gefärbten Agarosegel aufgetrennt und analysiert.

Folgende Primer wurden für die Expressionsanalyse der entsprechenden Antigene bei der angegebenen Hybridisierungstemperatur verwendet.
Claudin-18A1 (64°C)

| | |
|---|---|
| Sense: 5'-GAGGCAGAGTTCAGGCTTCACCGA-3' | (SEQ ID NO: 109) |
| Antisense: 5'-TGTTGGCTTTGGCAGAGTCC-3' | (SEQ ID NO: 110) |

Claudin-18A2 (68°C)

| | |
|---|---|
| Sense1: 5'-GGTTCGTGGTTTCACTGATTGGGATTGC-3' | (SEQ ID NO: 39) |
| Antisense1: 5'-CGGCTTTGTAGTTGGTTTCTTCTGGTG-3' | (SEQ ID NO: 40) |
| Sense2: 5'-TGTTTTCAACTACCAGGGGC-3' | (SEQ ID NO: 107) |
| Antisense2: 5'-TGTTGGCTTTGGCAGAGTCC-3' | (SEQ ID NO: 108) |

### Herstellung von Random-Hexamer-geprimter cDNA und quantitative Real-Time-PCR

Die Expression mehrere Gene wurde mittels Real-Time-PCR quantifiziert. Dabei wurden die PCR-Produkte mit SYBR-Green als interkalierendem Reporterfarbstoff detektiert. Die Reporterfluoreszenz von SYBR-Green ist in Lösung supprimiert und erst nach Bindung an dopplesträngige DNA-Fragmente ist der Farbstoff aktiv. Das Ansteigen der SYBR-Green-Fluoreszenz als Folge der spezifischen Amplifikation mittels GOI-spezifischen Primern nach jedem PCR-Zyklus wird zur Quantifizierung genutzt. Die Expressionsquantifizierung des Zielgens erfolgt absolut oder relativ zur Expression eines Kontrollgens mit konstanter Expression in den zu untersuchenden Geweben. Die Expression wurde nach Normalisierung der Proben gegen 18s RNA als sog. Housekeeping-Gen mittels der ΔΔ-Cₜ Methode (PE Biosystems, USA) ermittelt. Die Reaktionen wurden in Duplex-Ansätzen durchgeführt und in Triplikaten bestimmt. Verwendet wurde der QuantiTect SYBR-Green PCR Kit (Qiagen, Hilden) nach Angaben des Herstellers. Die Synthese der cDNA erfolgte mit dem High Capacity cDNA Archive Kit (PE Biosystems, USA) unter Verwendung von Hexamer-Primern nach Angaben des Herstellers. Jeweils 5 µl der verdünnten cDNA wurden in 25 µl Gesamtvolumen für die PCR eingesetzt: sense-Primer 300nM, antisense-Primer 300nM; initiale Denaturierung 95°C 15 min; 95°C 30 sec; Annealing 30 sec; 72°C 30 sec; 40 Zyklen. Die Sequenzen der verwendeten Primer sind in den jeweiligen Beispielen aufgeführt.

### Klonierung und Sequenzanalyse

Klonierung von Vollängen bzw. Genfragmenten erfolgte nach gängigen Methoden. Zur Ermittlung der Sequenz wurden entsprechende Antigene mittels der Proofreading-Polymerase pfu (Stratagene) amplifiziert. Nach Beendigung der PCR wurde Adenosin mittels HotStarTaq DNA Polymerase an die Enden des Amplikons ligiert, um die Fragmente entsprechend den Angaben des Herstellers in den TOPO-TA-Vektor zu klonieren. Die Sequenzierung wurde durch einen kommerziellen Service durchgeführt. Die Sequenzen wurden mittels gängiger Prädiktionsprogramme und Algorithmen analysiert.

### Western-Blot

Zellen aus Zellkultur (endogene Expression des Zielgens oder Synthese des Zielproteins nach Transfektion eines Expressionsvektors, der das Zielprotein kodiert) oder Gewebeproben, die das Zielprotein enthalten könnten, werden in einer 1%igen SDS Lösung lysiert. Das SDS denaturiert dabei die im Lysat enthaltenen Proteine. Die Lysate eines experimentellen Ansatzes werden abhängig von der erwarteten Proteingröße auf 8-15 %igen denaturierenden Polyacrylamidgelen (enthalten 1% SDS) der Größe nach elektrophoretisch aufgetrennt (SDS-Polyacrylamid-Gelelektrophorese, SDS-PAGE). Anschließend werden die Proteine durch das semi-dry Elektroblot Verfahren (Biorad) auf Nitrozellulose-Membran (Schleicher & Schüll) transferiert, auf der das gewünschte Protein nachgewiesen werden kann. Dazu wird die Membran zunächst blockiert (z.B. mit Milchpulver) und anschließend mit dem spezifischen Antikörper in einer Verdünnung von 1:20-1:200 (je nach Spezifität des Antikörpers) für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. Nach einem weiteren Waschschritt wird anschließend das Zielprotein in einer Farb- oder Chemilumineszenz-Reaktion auf der Membran mittels einer Enzymreaktion sichtbar gemacht (z.B. ECL, Amersham Bioscience). Das Ergebnis wird durch Aufnahme mit einer geeigneten Kamera dokumentiert.

Die Analyse von Proteinmodifikationen erfolgt in der Regel im Western-Blot. Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate aus Geweben oder Zellen vor der Denaturierung durch SDS mit O- oder N-Glykosidasen inkubiert (nach Angaben des jeweiligen Herstellers, z.B. PNgase, Endoglykosidase F, Endoglykosidase H, Roche Diagnostics). Anschließend erfolgt ein Western-Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden. Mit Algorithmen und Prädiktionsprogrammen kann die genaue Position der glykosylierten Aminosäure prädiziert werden.

### Immunfluoreszenz

Es werden Zellen etablierter Zelllinien benutzt, die entweder das Zielprotein endogen synthetisieren (Nachweis der RNA in der RT-PCR oder des Proteins im Western-Blot) oder aber vor der IF mit Plasmid-DNA transfiziert worden sind. Zur Transfektion von Zelllinien mit DNA sind die verschiedensten Methoden (z.B. Elektroporation, Liposomen-basierte Transfektion, Calciumphosphatpräzipitation) gut etabliert (z.B. Lemoine et al. Methods Mol. Biol. 1997; 75: 441-7). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen und kurze Peptidsequenzen von 6-12 Aminosäuren, für die hoch affine und spezifische Antikörper zur Verfügung stehen. Zellen, die das Zielprotein synthetisieren, werden mit Paraformaldehyd, Saponin oder Methanol fixiert. Anschließend können die Zellen bei Bedarf durch Inkubation mit Detergenzien (z.B. 0,2% Triton X-100) permeabilisiert werden. Nach der Fixierung/Permeabilisierung werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfarbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, dass direkt angeregt werden kann und selbst fluoresziert, so dass zum Nachweis keine Antikörper benötigt werden.

### Immunhistochemie

Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und/oder (3) den Zelltyp in einem Gewebe (Tumor, gesunde Zellen) zu definieren, in dem das Zielprotein nachweisbar ist.

Je nach dem individuellen Antikörper müssen unterschiedliche Protokolle verwendet werden (z.B. "Diagnostic Immunohistochemistry by David J., MD Dabbs ISBN: 0443065667" oder in "Microscopy, Immunohistochemistry, and Antigen Retrieval Methods: For Light and Electron Microscopy ISBN: 0306467704").

Die Immunhistochemie (IHC) an spezifischen Gewebeproben dient dem Proteinnachweis im entsprechenden Gewebe. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR-Analysen bekannt ist. Eine Hintergrundkontrolle ist immer mitzuführen.

Fixierte Gewebe (z.B. Fixation mit aldehydhaltigen Substanzen, Formaldehyd, Paraformaldehyd oder in alkoholischen Lösungen) oder schockgefrorene Gewebestücke mit einer Dicke von 1-10µm werden auf einem Glasträger aufgebracht. Paraffineingebettete Proben werden z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der von Farbsubstraten, die von dem gebundenen Enyzmen umgesetzt werden (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Immunisierung

(Siehe auch Monoclonal Antibodies: A Practical Approach by Philip Shepherd, Christopher Dean ISBN: 0-19-963722-9; Antibodies: A Laboratory Manual by Ed Harlow, David Lane ISBN: 0879693142; Using Antibodies: A Laboratory Manual: Portable Protocol NO. by Edward Harlow, David Lane, Ed Harlow ISBN: 0879695447).
Im Folgenden wird der Herstellungsprozess von Antikörpern kurz beschrieben, Details sind den zitierten Publikationen zu entnehmen. Zunächst werden Tiere (z.B. Kaninchen) durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend ca. alle 2 Wochen mit insgesamt bis zu 5 Entnahmen) wird den Tieren Blut entnommen und daraus ein Immunserum gewonnen.
Die Immunisierung der Tiere erfolgt in der Regel über eines von vier gut etablierten Verfahren, wobei auch andere Verfahren verfügbar sind. Immunisiert werden kann dabei mit Peptiden, die für das Zielprotein spezifisch sind, dem gesamten Protein oder mit extrazellulären Teilsequenzen eines Proteins, das experimentell oder über Prediktionsprogramme identifiziert werden kann.
(1) Im ersten Fall werden an KLH (keyhole limpet hemocyanin) konjugierte-Peptide (Länge: 8-12 Aminosäuren) über ein standardisiertes in vitro-Verfahren synthetisiert und diese Peptide zur Immunisierung verwendet. In der Regel erfolgen 3 Immunisierungen mit einer Konzentration von 5-1000 µg/Immunisierung. Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei in vitro, in Bakterien (z.B. E. coli), in Hefe (z.B. S. pombe), in Insektenzellen oder in mammalen Zellen synthetisiert. Nach Synthese in einem der Systeme wird das Zielprotein aufgereinigt, wobei die Aufreinigung dabei in der Regel über standardisierte chromatografische Methoden erfolgt. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; Gst-Fusionsproteine). Eine Vielzahl von Protokollen finden sich z.B. in den "Current Protocols in Molecular Biology", John Wiley & Sons Ltd., Wiley InterScience.
(3) Falls eine Zelllinie zur Verfügung steht, die das gewünschte Protein endogen synthetisiert, kann auch diese Zelllinie zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend werden 5-100 µg DNA als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielgen (Jung et al., Mol Cells 12: 41-49, 2001; Kasinrerk et al., Hybrid Hybridomics 21: 287-293, 2002).

### Qualitätskontrolle des polyklonalen Serums bzw. Antikörpers

Zum Spezifitätsnachweis eignen sich am besten auf Zellkultur-basierende Tests mit anschließendem Westem-Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter der Kontrolle eines starken eukaryontischen Promotors steht (z.B. Cytomegalovirus-Promotor). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgenspezifische RT-PCR). Zur Kontrolle werden im Experiment im Idealfall homologe Gene mit transfiziert, um im folgenden Westem-Blot die Spezifität des analysierten Antikörpers nachweisen zu können.

Im anschließenden Westem-Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid-Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

Zur Bestätigung der im in silico-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Westem-Blot) oder aber mit Plasmid-DNA transfiziert worden sind. Zur Transfektion von Zelllinien mit DNA sind die verschiedensten Vefahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln.

Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht.

Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz den Nachweis, ob ein immunogenes Epitop innerhalb oder außerhalb der Zelle lokalisiert ist. Die Prädiktion der ausgewählten Proteine kann so experimentell untermauert werden.

Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zur Immunfluoreszenz kann durch Verwendung von z.B. Propidiumiodid oder Trypanblau zwischen toten und lebenden Zellen unterschieden werden und damit falsch positive Ergebnisse vermieden werden.

### Affinitätsreinigung

Die Reinigung der polyklonalen Seren erfolgte im Fall der Peptidantikörper gänzlich oder im Fall der Antikörper gegen rekombinante Proteine teilweise als Service durch die beauftragten Firmen. Hierzu wurde in beiden Fällen das entsprechende Peptid bzw. rekombinante Protein kovalent an eine Matrix gebunden, diese nach der Kopplung mit einem nativen Puffer (PBS: phosphate buffered saline) äquilibriert und im Anschluß mit dem Rohserum inkubiert. Nach einem weiteren Waschschritt mit PBS wurde der Antikörper mit 100 mM Glycin, pH 2,7 eluiert und das Eluat sogleich in 2 M TRIS, pH 8 neutralisiert. Die so gereinigten Antikörper konnten dann zur spezifischen Detektion der Zielproteine sowohl durch Westernblotting als auch durch Immunfluoreszenz eingesetzt werden.

### Herstellung von GFP-Transfektanten

Für die Immunfluoreszenz-Mikroskopie von heterolog exprimierten Tumor-assoziierten Antigenen wurde der komplette ORF der Antigene in pGFP-C1- und pGFP-N3-Vektoren (Clontech) kloniert. Auf Objekträgern kultivierte CHO- und NIH3T3-Zellen wurden mit den entsprechenden Plasmidkonstrukten unter Verwendung von Fugene-Transfektionsreagenz (Roche) nach Herstellerangaben transfiziert und nach 12-24h mittels Immunfluoreszenz-Mikroskopie analysiert.

### Durchflusszytometrie

Durchflusszytometrische Messungen wurden gemäß bekannter Verfahren durchgeführt (z. B. Robinson (Editor) Handbook of flow cytometry methods. Wiley-Liss, New York, 1993).

### Beispiel 1: Identifizierung von Claudin-18A1- und Claudin-18A2-Spleißvarianten als diagnostische und therapeutische Krebs-Targets

Das Claudin-18-Gen kodiert für ein Oberflächenmembranmolekül mit 4 hydrophoben Bereichen. Entsprechend der Prädiktionsprogramme (TMHMM, TMPred) und der für viele andere Mitglieder dieser Familie beschriebenen Topologie zufolge besitzt Claudin-18 vier Transmembran- und somit zwei extrazelluläre Domänen EX1 und EX2, deren extrazelluläre Lokalisation (Konformation 1) in Abb. 2 dargestellt ist. Die Domäne D3, die zwischen den beiden extrazellulären Epitopen lokalisiert ist, ist in der Literatur für Claudin-18 und andere Mitglieder dieser Familie als intrazellulär beschrieben und wird mit herkömmlichen Prädiktionsprogrammen auch so prädiziert. Der N- wie auch C-Terminus sind intrazellulär. Niimi und Kollegen (Mol. Cell. Biol. 21:7380-90, 2001) beschrieben zwei Spleißvarianten des Maus- und humanen Claudin-18, die als selektiv in Lungengewebe (Claudin-18A1) bzw. in Magengewebe (Claudin-18A2) exprimiert beschrieben wurden. Diese Varianten unterscheiden sich im N-Terminus.

Es wurde untersucht, inwieweit die Spleißvarianten Claudin-18A2 (SEQ ID NO: 7) und Claudin-18A1 (SEQ ID NO: 117) sowie ihre jeweiligen Translationsprodukte (SEQ ID NO: 16 und 118) als Marker bzw. therapeutische Zielstrukturen für Tumoren genutzt werden können. Es wurde eine quantitative PCR etabliert, die zwischen beiden Varianten unterscheiden kann, indem A1-spezifische (SEQ ID NO: 109, 110) bzw. A2-spezifische (SEQ ID NO: 107, 108) Primerpaare ausgewählt wurden. Die Spleißvariante A2 wurde zusätzlich mit einem zweiten Primerpaar in einer konventionellen PCR getestet (SEQ ID NO: 39, 40).

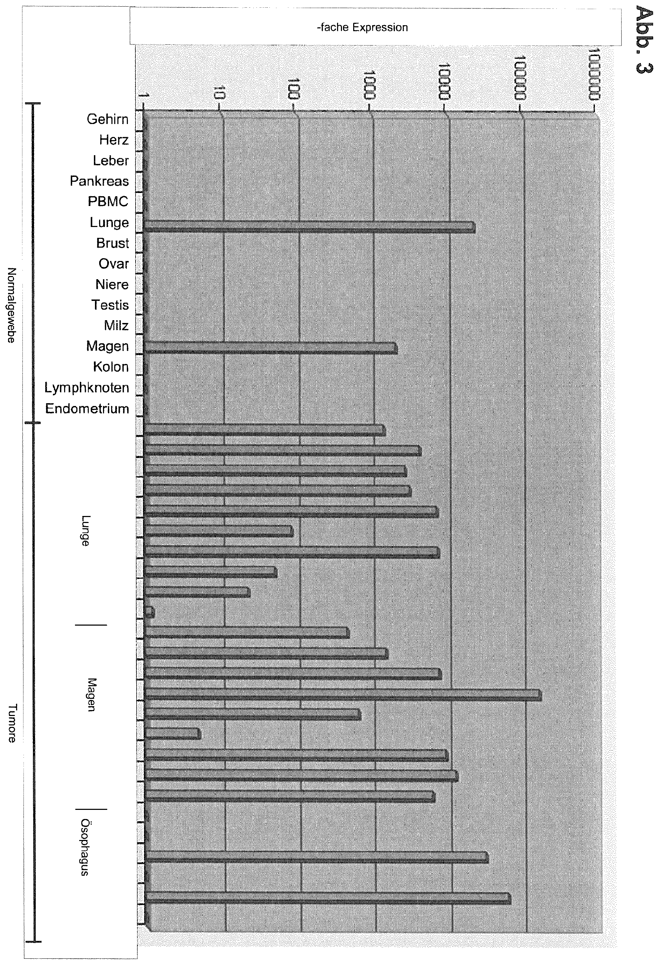

Für die Variante A1 ist beschrieben, dass sie nur in gesundem Lungengewebe aktiv ist. Jedoch stellten wir überraschenderweise fest, dass die Variante A1 auch in der Magenschleimhaut aktiv ist (Abb. 3). Magen und Lunge sind die einzigen Normalgewebe, die eine signifikante Aktivierung aufweisen. Alle anderen Normalgewebe sind negativ für Claudin-A1. Bei der Untersuchung von Tumoren wurde überraschend festgestellt, dass Claudin-A1 in einer Vielzahl von Tumorgeweben stark aktiviert ist. Ein besonders starke Expression findet sich in Magentumoren, Lungentumoren, Pankreastumoren, Ösophagustumoren (Abb. 3), HNO-Tumoren und Prostatatumoren. Die Expressionsspiegel von Claudin-A1 in HNO-, Prostata-, Pankreas- und Ösophagustumoren sind 100-10000 höher als die Spiegel in den korrespondierenden Normalgeweben.

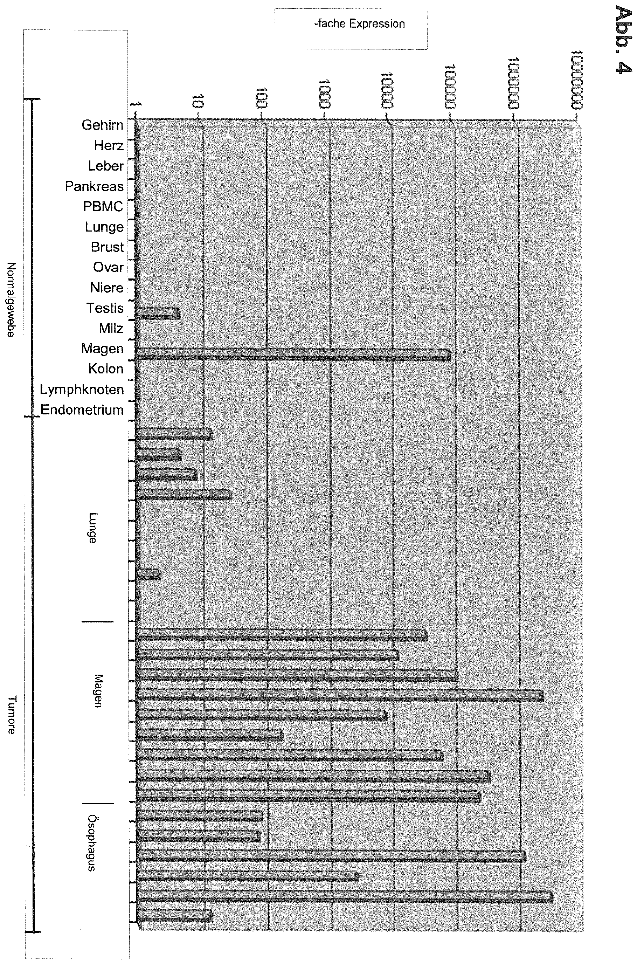

Für die Untersuchung der Claudin-A2-Spleißvariante wurden Oligonukleotide verwendet, die spezifisch die Amplifikation dieses Transkripts ermöglichen (SEQ ID NO: 39, 40 bzw. 107, 108). Die Untersuchung ergab, dass die Spleißvariante A2 in keinem der mehr als 20 untersuchten Normalgewebe außer in Magenschleimhaut und in geringem Ausmaß auch Testisgewebe exprimiert wird (Abb. 4). Wir stellten fest, dass wie die Variante A1 auch die Variante A2 in vielen Tumoren aktiviert ist (Abb. 4).

Hierzu zählen Magentumore, Pankreastumore, Ösophagustumore und Lebertumore. Obwohl sich in gesunder Lunge keine Aktivierung von Claudin-18A2 nachweisen lässt, wurde überraschend festgestellt, dass ein Teil der Lungentumoren die Spleißvariante A2 exprimieren.

**Tabelle 1A. Expression von Claudin-18A2 in Normal- und Tumor-Geweben**

| **Normalgewebe Expression** | **Tumortyp Expression** |
|---|---|
| Gehirn | - |
| Cerebellum | - |
| Myokard | - |
| Skelettmuskel | - |
| Endometrium | - |
| Magen | +++ |
| Kolon | - |
| Pankreas | - |
| Niere | - |
| Leber | - |
| Testis (Hoden) | + |
| Thymus | - |
| Brust | - |
| Ovar | - |
| Uterus | - |
| Haut | - |
| Lunge | - |
| Schilddrüse | - |
| Lymphknoten | - |
| Milz | - |
| PBMC | - |
| Ösophagus | - |
| | |

| **Tumortyp** | **Expression** |
|---|---|
| Kolon | - |
| Pankreas | ++ |
| Ösophagus | ++ |
| Magen | +++ |
| Lunge | ++ |
| Brust | - |
| Ovar | - |
| Endometrium | n.u. |
| HNO | ++ |
| Niere | - |
| Prostata | - |
| | |
| | |

**Tabelle 1B. Expression von Claudin-18A1 in Normal- und Tumor-Geweben**

| **Normalgewebe** | **Expression** |
|---|---|
| Gehirn | - |
| Cerebellum | - |
| Herzmuskel | - |
| Muskel | - |
| Endometrium | - |
| Magen | +++ |
| Kolon | - |
| Pankreas | - |
| Niere | - |
| Leber | - |
| Testis | + |
| Thymus | - |
| Brust | - |
| Ovar | - |
| Uterus | - |
| Haut | - |
| Lunge | +++ |
| Schilddrüse | - |
| Lymphknoten | - |
| Milz | - |
| PBMC | - |
| Ösophagus | - |
| | |

| **Tumortyp** | **Expression** |
|---|---|
| Kolon | - |
| Pankreas | ++ |
| Ösophagus | ++ |
| Magen | +++ |
| Lunge | ++ |
| Brust | + |
| Ovar | n.u |
| Endometrium | n.u. |
| HNO | ++ |
| Niere | - |
| Prostata | ++ |
| | |
| | |

Auch die konventionelle PCR als unabhängige Kontrolluntersuchung bestätigte die Resultate der quantitativen PCR. Hierzu wurden Oligonukleotide (SEQ ID NO: 39, 40) verwendet, die eine spezifische Amplifikation der Spleißvariante A2 erlauben. Es wurde gezeigt, dass die meisten Magentumore und die Hälfte der getesteten Pankreastumore eine starke Expression dieser Spleißvariante aufweisen (Abb. 1). Dagegen ist eine Expression mit konventioneller PCR in anderen Geweben nicht nachweisbar. Insbesondere findet sich in wichtigen Normalgeweben wie Lunge, Leber, Blut, Lymphknoten, Brust und Niere keine Expression (Tab. 1). Hiermit stellen die Spleißvarianten hochspezifische molekulare Marker für Tumoren des oberen Magendarmtraktes wie auch Lungentumoren, HNO-Tumoren, Prostatatumoren und ihre Metastasen dar. Diese molekulare Marker können erfindungsgemäß zum Nachweis von Tumorzellen genutzt werden. Die Detektion der Tumoren kann mit den genannten Oligonukleotiden (SEQ ID NO: 39, 40, 107-110) erfolgen. Als Oligonukleotide eignen sich insbesondere Primerpaare, von denen mindestens einer unter stringenten Bedingungen an einen 180 Basenpaare langen Abschnitt des Transkripts bindet, der spezifisch für die eine (SEQ ID NO: 8) oder andere Spleißvariante (SEQ ID NO: 119) ist.

Diese Genprodukte sind attraktive therapeutische Zielstukturen, da durch ihr Fehlen in den meisten toxizitätsrelevanten Organen an diesen keine Nebenwirkungen zu erwarten sind, während die starke Aktivierung in Zellen der genannten Krebsarten eine gute Bindung an diese und Vermittlung ensprechender zellschädigender Effekte erwarten lässt.

Um diese Daten auf Proteinebene zu bestätigen, wurden Claudin-spezifische Antikörper bzw. Immunseren durch Immunisierung von Tieren generiert.
Die N-terminale extrazelluläre Domäne EX1 unterscheidet sich in der Sequenz bei den beiden Spleißvarianten A1 und A2 (SEQ ID NO: 111 für A1 und SEQ ID NO: 112 für A2). Die C-terminale extrazelluläre Domäne EX2 ist für beide Varianten identisch (SEQ ID NO: 137). Bisher sind noch keine Antikörper beschrieben, die an die extrazelluläre Domänen von Claudin-18 binden. Auch wurden bisher keine Antikörper beschrieben, die spezifisch zwischen A1- und A2-Varianten unterscheiden können. Es wurden für die Immunisierung zur Generierung von Antikörpern extrazellulär gelegene Peptidepitope und Proteinfragemente ausgewählt, die spezifisch für die Variante A1 oder A2 sind bzw. in beiden Varianten vorkommen. Unter anderem wurden zur Immunisierung folgende Peptide für die Herstellung von Antikörpern ausgewählt:
SEQ ID NO: 17: DQWSTQDLYN (N-terminal-extrazelluläre Domäne, A2-spezifisch, Bindung unabhängig von Glykosylierung)
SEQ ID NO: 18: NNPVTAVFNYQ (N-terminal-extrazelluläre Domäne, A2-spezifisch, Bindung hauptsächlich an unglykosylierte Form, N37)
SEQ ID NO: 113: STQDLYNNPVTAVF (N-terminal-extrazelluläre Domäne, A2-spezifisch, Bindung nur an nicht glykosylierte Form, N37)
SEQ ID NO: 114: DMWSTQDLYDNP (N-terminal-extrazelluläre Domäne, A1-spezifisch)
SEQ ID NO: 115: CRPYFTILGLPA (N-terminal-extrazelluläre Domäne, hauptsächlich spezifisch für A1)
SEQ ID NO: 116: TNFWMSTANMYTG (C-terminal-extrazelluläre Domäne, erkennt sowohl A1 als auch A2).

Wir konnten unter anderem Antikörper generieren, die selektiv die N-terminale Domäne der Spleißvariante Claudin-18-A1 erkennen, aber nicht die A2-Variante (Abb. 8). Durch Nutzung von Epitopen für Immunisierungen, welche in der C-terminal-extrazellulären Domäne liegen, die identisch in beiden Spleißvarianten ist, konnten wir Antikörper herstellen, die beide Varianten erkennen (Abb. 7).

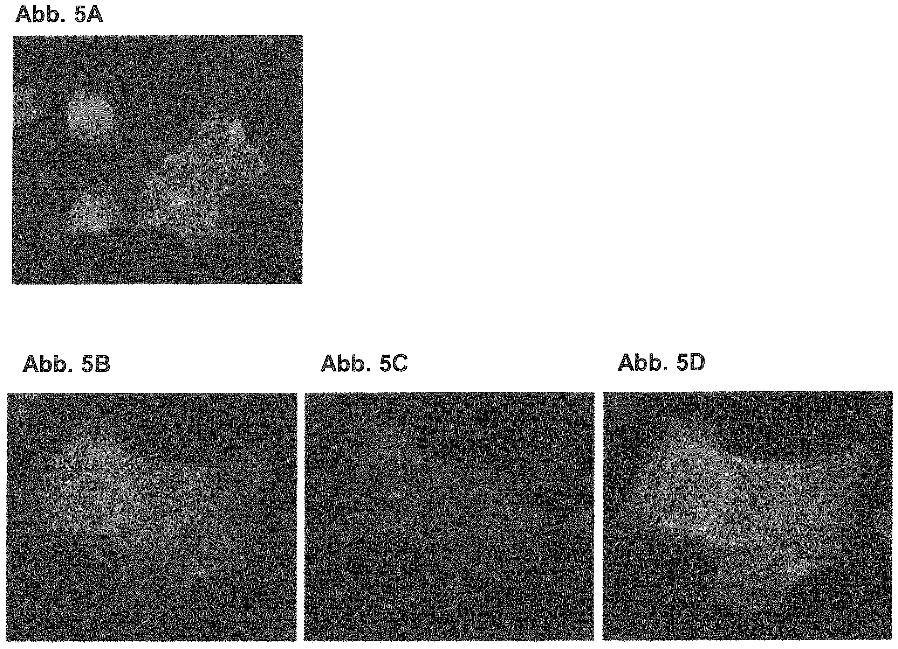

Beispielhaft werden die Daten für einen A2-spezifischen Antikörper, der durch Immunisierung mit SEQ ID NO: 17 hergestellt wurde, dargestellt. Der spezifische Antikörper lässt sich unter verschiedenen Fixierungsbedingungen für Immunfluoreszenz-Untersuchungen nutzen. Bei vergleichenden Färbungen von RT-PCR-positiven wie auch negativen-Zelllinien ist das entsprechende Protein in gut nachweisbarer Menge spezifisch u.a. in den als positiv typisierten Magentumor-, Ösophagustumor- und Pankreastumor-Zelllinien detektierbar (Abb. 5). Das endogene Protein ist membranlokalisiert und bildet größere fokale Aggregate an der Membran (Abb. 5). Mit diesem Antikörper wurden immunhistochemische Färbungen an menschlichen Geweben durchgeführt. Wir bestätigten die selektive Gewebeverteilung dieses Proteins. Ein umfangreiches Sortiment verschiedender Normalgewebe wurde untersucht, von denen in fast allen Claudin-18A2-Protein nicht nachweisbar ist, wie beispielhaft für Leber, Lunge, Niere und Kolon dargestellt wurde. Wir fanden lediglich eine Aktivierung dieses Proteins in normalem Magengewebe (Abb. 12). Überraschenderweise sahen wir, dass die A2-Variante von Claudin-18 zwar in den differenzierten Zellen der Magenschleimhaut nachweisbar ist, aber nicht in den Stammzellen. Differenzierte Magenschleimhautzellen sind einer ständigen Erneuerung unterworfen. Physiologischerweise wird das gesamte Magenepithel ausgehend von den Stammzellen des Magens kontinuierlich ersetzt. Dies unterstützt die Nutzbarkeit der A2-Variante als therapeutische Zielstuktur, da wir erfindungsgemäß zeigen, dass Stammzellen des Magens als die indispensible Zellpopulation der Magenschleimhaut genauso wie alle anderen gesunden Organe die A2-Variante nicht tragen und somit von einer spezifisch gegen die A2-Variante gerichteten Substanz nicht angegriffen werden können. Wir wiesen in einer Reihe humaner Tumoren mittels dieses Antikörpers die A2-Variante von Claudin-18 nach (Abb. 13), insbesondere in Tumoren von Magen, Ösophagus und Lunge, die uns bereits in RT-PCR-Untersuchungen aufgefallen waren. Erfindungsgemäß sind diese Tumoren therapeutisch zugänglich.

Der oben beschriebene Antikörper wurde des Weiteren für einen Proteinnachweis im Western-Blot eingesetzt. Erwartungsgemäß wird Protein lediglich in Magen und keinem anderen Normalgewebe, auch nicht Lunge, in der lediglich die A1-Variante aktiviert ist, detektiert (Abb. 9). Bei der vergleichenden Färbung von Magentumoren und adjazentem normalem Magengewebe aus Patienten fiel überraschend auf, dass in allen Magentumoren, in denen Claudin-18A2 detektiert wird, dieses Protein ein kleineres Massengewicht hat (Abb. 10, links). In einer Serie von Experimenten wurde festgestellt, dass eine Bande auf dieser Höhe sich dadurch ergibt, dass man Lysat normalen Magengewebes mit dem deglykosylierenden Agens PNGase F behandelt (Abb. 10, rechts). Während in allen normalen Magengeweben ausschließlich die glykosylierte Form der Variante A2 nachweisbar ist, ist A2 als solches in über 60% der untersuchten Magentumore, und zwar ausschließlich in der deglykosylierten Form, nachweisbar. Obwohl die A2-Variante von Claudin-18 in normaler Lunge auch auf Proteinebene nicht detektiert wird, ist sie wie auch schon in der quantitativen RT-PCR in Lungentumoren zu finden. Auch hier liegt lediglich die deglykosylierte Variante vor (Abb. 11).

Claudin-18 selbst ist ein hochselektives Differenzierungsantigen von Magen (Variante A2) bzw. von Lunge und Magen (Variante A1). Unsere Daten weisen daraufhin, dass es offensichtlich von Tumor-assoziierten Veränderungen der Glykosylierungmaschinerie betroffen ist und in Tumoren eine besondere Form der Variante A2 entsteht, die deglykosyliert ist. Die Resultate der PNGaseF-Behandlung zeigen, dass Claudin-18A2 sich in Tumor-und Normalgewebe hinsichtlich seiner N-Glykosylierung unterscheidet.

Die Glykosylierung eines Epitops kann die Bindung eines für dieses Epitop-spezifischen Antikörpers verhindern und im vorliegenden Fall dazu beitragen, dass ein solcher Antikörper nicht an Claudin-18A2 in Normalgeweben binden kann, sondern exklusiv an die nichtglykosylierte Form in Krebszellen. Um Antikörper herzustellen, die exklusiv an nichtglykosylierte Epitope binden, wurde dies bei der Auswahl der Immunogene berücksichtigt. Wir haben verschiedene Regionen von Claudin-18A2 identifiziert, die in Tumor- und Normalgewebe unterschiedlich glykosyliert vorliegen können. Als potenzielle Glykosylierungsstellen für Claudin-18A2 haben wir u.a. die Regionen, die die Aminosäuren 37, 38, 45, 116, 141, 146, 205 von Claudin-18A2 umfassen, identifiziert (Abb. 2 unten). Tumorzellen und Normalgewebe unterscheiden sich durch eine unterschiedliche Glykosylierung in einer oder mehrerer dieser Positionen. Die meisten dieser Bereiche stellen keine klassische Glykosylierungsstelle dar, aber enthalten Asparagin, Serin und Threonin, die in seltenen Fällen auch glykosyliert werden können (Prädiktion Abb. 2 unten). Beide Varianten von Claudin-18 haben ein einziges klassisches Glykosylierungsmotiv in der D3-Domäne, die in der Literatur und entsprechend gängigen Prädiktionsalgorithmen als intrazellulär gilt. Allerdings konnte bei einem strukturell dem Claudin-18 ähnlichen Tetraspanin, dem PMP 22, gezeigt werden, dass die hydrophoben Membrandomänen 2 und 3 von PMP 22 nicht vollständig durch die Zellmembran laufen, sondern nur partiell in die Plasmamembran interkalieren (Taylor et al., J Neurosc. Res. 62:15-27, 2000). Aus diesem Grund ist der gesamte Bereich zwischen den zwei äusseren Transmembrandomänen bei PMP22 extrazellulär lokalisiert.

Wir haben die Möglichkeit einer solchen Topologie für Claudin-18A2 hypothetisiert und überprüft. Hierzu haben wir 3 Konstrukte hergestellt, die jeweils eine Marker-Sequenz (His- oder HA-Tag) in einer der Domänen EX1, EX2, oder D3 trugen (Abb. 15 oben). Diese wurden in Zelllinien transfiziert und daraufhin getestet, ob ein gegen diese Markersequenzen gerichteter Antikörper an nichtpermeabilisierte Zellen bindet, was erfordert, dass der entsprechende Bereich des Proteins topologisch extrazellulär ist. Da alle drei Bereiche des Moleküls durchflusszytometrisch als extrazellulär gemessen wurden (Abb. 15 unten), konnten wir bestätigen, dass Claudin-18A2 in einer Konformation mit zwei Transmembrandomänen und einer großen extrazellulär gelegenen Domäne vorliegen kann (Abb. 2, Konformation 2). Diese Konformation ist biochemisch und therapeutisch relevant, da sie zusätzliche Bindungsstellen für therapeutische Antikörper (SEQ ID NO: 142, 143) beinhaltet.

Somit können Antikörper hergestellt werden, die zwischen glykosylierten und nicht glykosylierten Varianten von Claudin-18A2 unterscheiden. Diese haben eine besonders hohe Spezifität für Tumorzellen. Bei der Herstellung glykosilierungsspezifischer Antikörper haben wir neben den Glykosylierungsdomänen auch diese unterschiedlichen Konformationsoptionen berücksichtigt.

Vorzugsweise, aber nicht ausschließlich, eignen sich Proteinfragmente aus dem D3-Bereich von Claudin-18A2 zur Immunisierung von Tieren. Beispielhaft ist dies für zwei Antikörper mAB1 und mAB2 dargestellt (Abb. 17). Wir untersuchten die Bindeeigenschaften dieser Antikörper an Zelllinien, die entweder die A1- oder die A2-Variante des Claudin-18 exprimieren. Wir konnten zeigen, dass Claudin-18A2 für Antikörper auf der Zelloberfläche zugänglich ist. Solche Antikörper sind spezifisch für die A2-Variante und binden nicht an die A1-Variante (Abb. 17). Wir haben kurze Fremdsequenzen (myc-tag) jeweils in den Bereich der Extrazellulärdomänen Ex1 und Ex2 eingeführt (Abb. 43). Am Beispiel von mAB1 ist dargestellt, dass die Bindeeigenschaften des Antikörpers dadurch nicht beeinträchtigt werden und das eigentliche Epitop sich in der D3-Domäne befindet. Die generierten Antikörper können diagnostisch wie auch therapeutisch genutzt werden. Immunseren wie das hier beschriebene (gegen Peptid SEQ ID NO: 17) können z.B. im Western-Blot diagnostisch genutzt werden. Durch Immunisierung mit Peptiden, die mindestens eine diese Regionen beinhalten (z.B. Peptid SEQ ID NO: 113 (Abbildung 6), Peptide SEQ ID NO: 142-145), können Antikörper hergestellt werden, die an das glykosylierte Epitop gar nicht binden können. Derartige Antikörper binden spezifisch an die deglykosylierten Epitope auf Tumorzellen. Die im Vergleich zu Normalgeweben fehlende Glykosylierung an einer der genannten Positionen könnte auch durch eine sekundäre, endogene Deglykosylierung in Tumorzellen bedingt sein. Eine derartige Deglykosylierung ist mit einer Asn (N)→ Asp (D) Transformation der betreffenden Aminosäure assoziiert. Für die Herstellung von Antikörpern gegen derartig veränderte tumorasssoziierte Varianten können daher Claudin-18A2-derivierte Peptide genutzt werden, in denen die Aminosäure Asn (N) an mindestens einer der Positionen 37, 38, 45, 116, 141, 146, 205 des Claudin-18A2-Peptids durch ein Asp (D) ersetzt ist (z.B. SEQ ID NO: 146-150). Insbesondere können solche Antikörper therapeutisch eingesetzt werden, da sie hochselektiv für Tumorzellen sind. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigenspezifischen T-Lymphozyten stellen die Aminosäuren 1-47 (SEQ ID NO: 19 und 120) besonders gute Epitope dar.

## Patentansprüche

1. Pharmazeutische Zusammensetzung zur Verwendung bei der Behandlung eines Adenokarzinoms des Magens, das sich durch die Expression eines Tumor-assoziierten Antigens auszeichnet, wobei die pharmazeutische Zusammensetzung einen Antikörper umfasst, der spezifisch an das Tumor-assoziierte Antigen bindet, wobei das Tumor-assoziierte Antigen eine Aminosäuresequenz aufweist, die von einer Nukleinsäuresequenz gemäß SEQ ID NO: 7 oder 8 kodiert wird und wobei der Antikörper ein einzelkettiger Antikörper ist oder wobei der Antikörper mit einem therapeutischen Mittel gekoppelt ist, wobei das therapeutische Mittel aus der Gruppe ausgewählt ist, bestehend aus Toxin, cytostatische oder cytolytische Arzneistoffe, Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat, Pokeweed-antivirales Protein, Choleratoxin, Pertussistoxin, Ricin, Gelonin, Abrin, Diphtherie-Exotoxin, *Pseudomonas-*Exotoxin*,* und Kobalt-60.

2. Pharmazeutische Zusammensetzung zur Verwendung nach Anspruch 1, wobei der Antikörper ein monoklonaler, chimärer oder humanisierter Antikörper oder ein Antigen-bindendes Fragment eines Antikörpers ist.

3. Pharmazeutische Zusammensetzung zur Verwendung nach Anspruch 1 oder 2, wobei das Tumor-assoziierte Antigen eine Aminosäuresequenz umfasst, die aus der Gruppe bestehend aus SEQ ID NOs: 16-19, 112, 113, 116, 137 und 142-145 ausgewählt ist.

4. Pharmazeutische Zusammensetzung zur Verwendung nach einem der Ansprüche 1-3, wobei der Antikörper an ein Peptid bindet, das eine Aminosäuresequenz umfasst, die aus der Gruppe bestehend aus SEQ ID NOs: 16-19, 112 und 113 ausgewählt ist.

5. Verwendung eines Antikörpers, der spezifisch an ein Tumor-assoziiertes Antigen bindet und mit einem therapeutischen Mittel gekoppelt ist, zur Herstellung einer pharmazeutischen Zusammensetzung zur Behandlung eines Adenokarzinoms des Magens, das sich durch die Expression des Tumor-assoziierten Antigens auszeichnet, wobei das Tumor-assoziierte Antigen eine Aminosäuresequenz aufweist, die von einer Nukleinsäuresequenz gemäß SEQ ID NO: 7 oder 8 kodiert wird,
wobei das therapeutische Mittel aus der Gruppe ausgewählt ist, bestehend aus Toxin, cytostatische oder cytolytische Arzneistoffe, Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat, Pokeweed-antivirales Protein, Choleratoxin, Pertussistoxin, Ricin, Gelonin, Abrin, Diphtherie-Exotoxin, *Pseudomonas-*Exotoxin*,* und Kobalt-60.

6. Verwendung nach Anspruch 5, wobei der Antikörper ein monoklonaler, chimärer oder humanisierter Antikörper oder ein Antigen-bindendes Fragment eines Antikörpers ist.

7. Verwendung nach Anspruch 5 oder 6, wobei das Tumor-assoziierte Antigen eine Aminosäuresequenz umfasst, die aus der Gruppe bestehend aus SEQ ID NOs: 16-19, 112, 113, 116, 137 und 142-145 ausgewählt ist.

8. Verwendung nach einem der Ansprüche 5 bis 7, wobei der Antikörper an ein Peptid bindet, das eine Aminosäuresequenz umfasst, die aus der Gruppe bestehend aus SEQ ID NOs: 16-19, 112 und 113 ausgewählt ist.

## Claims

1. A pharmaceutical composition for use in treating an adenocarcinoma of the stomach **characterised by** the expression of a tumor-associated antigen, wherein the pharmaceutical composition comprises an antibody which specifically binds to the tumor-associated antigen, wherein the tumor-associated antigen has an amino acid sequence encoded by a nucleic acid sequence according to SEQ ID NO: 7 or 8, and wherein the antibody is a single-chain antibody, or wherein the antibody is coupled to a therapeutic agent, wherein the therapeutic agent is selected from the group consisting of toxin, cytostatic or cytolytic drugs, aminoglutethimide, azathioprine, bleomycin sulfate, busulfan, carmustine, chlorambucil, cisplatin, cyclophosphamide, cyclosporine, cytarabidine, dacarbazine, dactinomycin, daunorubin, doxorubicin, taxol, etoposide, fluorouracil, interferon-α, lomustine, mercaptopurine, methotrexate, mitotane, procarbazine HCl, thioguanine, vinblastine sulfate and vincristine sulfate, pokeweed antiviral protein, cholera toxin, pertussis toxin, ricin, gelonin, abrin, diphtheria exotoxin, *Pseudomonas* exotoxin, and cobalt-60.

2. The pharmaceutical composition for the use of claim 1, wherein the antibody is a monoclonal, chimeric or humanized antibody or an antigen-binding fragment of an antibody.

3. The pharmaceutical composition for the use of claim 1 or 2, wherein the tumor-associated antigen comprises an amino acid sequence selected from the group consisting of SEQ ID NOs: 16-19, 112, 113, 116, 137 and 142-145.

4. The pharmaceutical composition for the use of any one of claims 1-3, wherein the antibody binds to a peptide comprising an amino acid sequence selected from the group consisting of SEQ ID NOs: 16-19, 112 and 113.

5. Use of an antibody which specifically binds to a tumor-associated antigen and is coupled to a therapeutic agent for the preparation of a pharmaceutical composition for treating an adenocarcinoma of the stomach **characterised by** the expression of the tumor-associated antigen, wherein the tumor-associated antigen has an amino acid sequence encoded by a nucleic acid sequence according to SEQ ID NO: 7 or 8, wherein the therapeutic agent is selected from the group consisting of toxin, cytostatic or cytolytic drugs, aminoglutethimide, azathioprine, bleomycin sulfate, busulfan, carmustine, chlorambucil, cisplatin, cyclophosphamide, cyclosporine, cytarabidine, dacarbazine, dactinomycin, daunorubin, doxorubicin, taxol, etoposide, fluorouracil, interferon-α, lomustine, mercaptopurine, methotrexate, mitotane, procarbazine HCl, thioguanine, vinblastine sulfate and vincristine sulfate, pokeweed antiviral protein, cholera toxin, pertussis toxin, ricin, gelonin, abrin, diphtheria exotoxin, *Pseudomonas* exotoxin, and cobalt-60.

6. The use of claim 5, wherein the antibody is a monoclonal, chimeric or humanized antibody or an antigen-binding fragment of an antibody.

7. The use of claim 5 or 6, wherein the tumor-associated antigen comprises an amino acid sequence selected from the group consisting of SEQ ID NOs: 16-19, 112, 113, 116, 137 and 142-145.

8. The use of any one of claims 5 to 7, wherein the antibody binds to a peptide comprising an amino acid sequence selected from the group consisting of SEQ ID NOs: 16-19, 112 and 113.

## Revendications

1. Composition pharmaceutique pour une utilisation lors du traitement d'un adénocarcinome de l'estomac, qui se **caractérise par** l'expression d'un antigène associé à une tumeur, la composition pharmaceutique comprenant un anticorps, qui se lie spécifiquement à l'antigène associé à la tumeur, l'antigène associé à la tumeur présentant une séquence d'acides aminés qui est codée par une séquence d'acides nucléiques selon SEQ ID NO:7 ou 8, et l'anticorps étant un anticorps monocaténaire, ou l'anticorps étant couplé à un agent thérapeutique, l'agent thérapeutique étant choisi dans le groupe consistant en une toxine, les médicaments cytostatiques ou cytolytiques, l'aminoglutéthimide, l'azathioprine, le sulfate de bléomycine, le busulfan, la carmustine, le chlormabucil, le cisplatine, le cyclophosphamide, la cyclosporine, la cytarabidine, la dacarbazine, la dactinomycine, la daunorubine, la doxorubicine, le taxol, l'étoposide, le fluoruracile, l'interféron-a, la lomustine, la mercaptopurine, le méthotrexate, le mitotane, la procarbazine-HCl, la thioguanine, le sulfate de vinblastine et le sulfate de vincristine, la protéine antivirale de la phytolaque, une protéine, la toxine du choléra, la toxine de la coqueluche, la ricine, la gélonine, l'abrine, l'exotoxine diphtérique, l'exotoxine de *Pseudomonas,* et le cobalt-60.

2. Composition pharmaceutique pour l'utilisation selon la revendication 1, dans laquelle l'anticorps est un anticorps monoclonal, chimère ou humanisé, ou le fragment de liaison à l'antigène d'un anticorps.

3. Composition pharmaceutique pour l'utilisation selon la revendication 1 ou 2, dans laquelle l'antigène associé à une tumeur comprend une séquence d'acides aminés qui est choisie dans le groupe consistant en SEQ ID NO: 16-19, 112, 113, 116, 137 et 142-145.

4. Composition pharmaceutique pour l'utilisation selon l'une des revendications 1 à 3, dans laquelle l'anticorps se lie à un peptide qui comprend une séquence d'acides aminés qui est choisie dans le groupe consistant en SEQ ID NO: 16-19, 112 et 113.

5. Utilisation d'un anticorps qui se lie spécifiquement à un antigène associé à une tumeur et est couplé à un agent thérapeutique, pour la fabrication d'une composition pharmaceutique pour le traitement d'un adénocarcinome de l'estomac, qui se **caractérise par** l'expression d'un antigène associé à une tumeur, l'antigène associé à une tumeur présentant une séquence d'acides aminés qui est codée par une séquence d'acides nucléiques selon SEQ ID NO:7 ou 8, l'agent thérapeutique étant choisi dans le groupe consistant en une toxine, les médicaments cytostatiques ou cytolytiques, l'aminoglutéthimide, l'azathioprine, le sulfate de bléomycine, le busulfan, la carmustine, le chlormabucil, le cisplatine, le cyclophosphamide, la cyclosporine, la cytarabidine, la dacarbazine, la dactinomycine, la daunorubine, la doxorubicine, le taxol, l'étoposide, le fluoruracile, l'interféron-a, la lomustine, la mercaptopurine, le méthotrexate, le mitotane, la procarbazine-HCl, la thioguanine, le sulfate de vinblastine et le sulfate de vincristine, la protéine antivirale de la phytolaque, une protéine, la toxine du choléra, la toxine de la coqueluche, la ricine, la gélonine, l'abrine, l'exotoxine diphtérique, l'exotoxine de *Pseudomonas,* et le cobalt-60.

6. Utilisation selon la revendication 5, l'anticorps étant un anticorps monoclonal, chimère ou humanisé, ou un fragment de liaison à l'antigène d'un anticorps.

7. Utilisation selon la revendication 5 ou 6, l'antigène associé à une tumeur comprenant une séquence d'acides aminés qui est choisie dans le groupe consistant en SEQ ID NO: 16-19, 112, 113, 116, 137 et 142-145.

8. Utilisation selon l'une des revendications 5 à 7, l'anticorps se liant à un peptide qui comprend une séquence d'acides aminés qui est choisie dans le groupe consistant en SEQ ID NO: 16-19, 112 et 113.
